# EUROPÄISCHE PATENTSCHRIFT

(11) **EP 2 742 030 B1**
(45) Veröffentlichungstag und Bekanntmachung des Hinweises auf die Patenterteilung: **27.07.2016**
(21) Anmeldenummer: 12746088.9
(22) Anmeldetag: 10.08.2012
(51) Int. Cl.: C07D 249/08, C07D 403/04, C07D 403/12, C07D 405/04, C07D 405/12, C07D 413/06, C07D 487/04, C07D 487/10, C07D 491/04, C07D 491/10, C07D 493/10, A01N 43/653, A01N 43/76, A01N 43/90

(54) **1,2,4-TRIAZOLYL-SUBSTITUIERTE KETOENOLE ZUM EINSATZ IM PFLANZENSCHUTZ**
1,2,4-TRIAZOLYL-SUBSTITUTED KETOENOLS FOR USE IN PLANT PROTECTION
CETOENOLS 1,2,4-TRIAZOLYL-SUBSTITUES DESTINES A LA PROTECTION DES PLANTES

(30) Priorität: 11.08.2011 EP 11177285; 11.08.2011 US 201161522308 P
(43) Veröffentlichungstag der Anmeldung: 18.06.2014
(73) Patentinhaber: Bayer Intellectual Property GmbH, 40789 Monheim (DE); Bayer CropScience AG, 40789 Monheim am Rhein (DE)
(72) Erfinder: GIENCKE, Wolfgang, 65719 Hofheim (DE); LEHR, Stefan, 69006 Lyon (FR); FISCHER, Reiner, 40789 Monheim (DE); LINDELL, Stephen, David, 65779 Kelkheim (DE); HÄUSER-HAHN, Isolde, 51375 Leverkusen (DE); HEINEMANN, Ines, 65719 Hofheim (DE); GATZWEILER, Elmar, 61231 Bad Nauheim (DE); ROSINGER, Christopher Hugh, 65719 Hofheim (DE); BECKER, Angela, 40235 Düsseldorf (DE); VOERSTE, Arnd, 50674 Köln (DE); GÖRGENS, Ulrich, D., I., 40882 Ratingen (DE)
(74) Vertreter: BIP Patents
(86) Internationale Anmeldenummer: PCT/EP2012/065682
(87) Internationale Veröffentlichungsnummer: WO 2013/021044

(56) Entgegenhaltungen:
- WO-A1-03/035643
- WO-A1-2006/029799
- WO-A1-2009/000533
- US-A- 4 024 149
- PREP'YALOV ET AL.: "Reactions of Unsaturated 1,3-Oxazines", SYNTHESIS, Nr. 3, 1999, Seiten 483-486, XP002682989,

## Beschreibung

Die vorliegende Erfindung betrifft neue 1,2,4-Triazolyl-substituierte Ketoenole, mehrere Verfahren zu ihrer Herstellung und ihre Verwendung als Schädlingsbekämpfungsmittel und/oder Herbizide. Auch Gegenstand der Erfindung sind selektiv herbizide Mittel, die 1,2,4-Triazolyl-substituierte Ketoenole einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere 1,2,4-Triazolyl-substituierte Ketoenole, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschtem Pflanzenwuchs.

Es ist bekannt, dass Heteroaryl-substituierte cyclische Ketoenole herbizide und/oder insektizide Eigenschaften besitzen (US 4,678,501, EP-A-912547, EP-A-134985, WO 2001/96333, WO 2002/088098, WO 2003/035643, WO 2009/000533, WO 2009/015877, WO2011/012862).

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) gefunden, 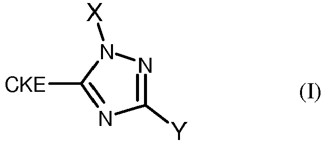 in welcher
- X: insbesondere bevorzugt für Methyl, Ethyl, n-Propyl, i-Propyl, n-Butyl, -CH₂CF₃ oder Cyclopropyl steht,
- Y: insbesondere bevorzugt für Phenyl, 4-Cl-Benzyl, 4-F-Phenyl, 4-Cl-Phenyl oder 2,4-Cl₂-Phenyl steht,

CKE insbesondere bevorzugt für eine der Gruppen 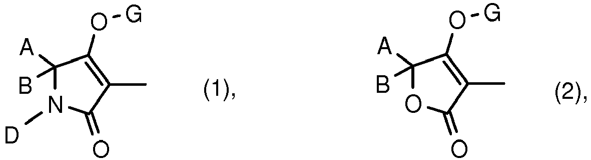 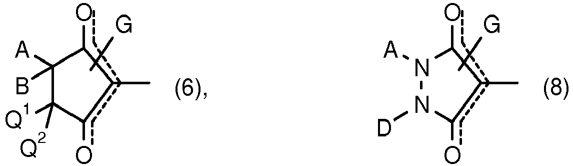 steht,
- A: insbesondere bevorzugt für Wasserstoff, Methyl, Ethyl oder Cyclopropyl steht,
- B: insbesondere bevorzugt für Wasserstoff, Methyl oder Cyclopropyl steht,
- A, B: und das Kohlenstoffatom an das sie gebunden sind, insbesondere bevorzugt für gesättigtes C₅-C₆-Cycloalkyl, in welchem gegebenenfalls ein Ringglied durch Sauerstoff oder Stickstoff ersetzt ist und welches gegebenenfalls einfach oder zweifach durch Methyl, Ethyl, Methoxymethyl, Methoxy, Ethoxy, Propoxy, Butoxy, Trifluorethoxy, Trifluormetyhl oder -O-CH₂CHCH₂ substituiert ist, wobei Methoxy oder Ethoxy auch als N-Substituenten in Frage kommen, oder
- A, B: und das Kohlenstoffatom, an das sie gebunden sind, stehen ganz besonders bevorzugt für C₆-Cycloalkyl stehen, welches gegebenenfalls durch eine mit zwei nicht direkt benachbarten Sauerstoffatomen enthaltende Alkylendioxyl-Gruppe substituiert ist, wobei ein weiterer 5- oder 6-Ring gebildet wird, der einfach oder zweifach durch Methyl substituiert sein kann,
- D: insbesondere bevorzugt für Wasserstoff oder cyclo-Propyl steht, oder
- A und: D gemeinsam insbesondere bevorzugt für C₃-C₅-Alkandiyl stehen, worin gegebenenfalls ein Kohlenstoffatom durch Sauerstoff ersetzt ist oder
- A und D: gemeinsam insbesondere bevorzugt für C₃-C₅-Alkandiyl stehen, welches gegebenenfalls durch eine mit zwei nicht direkt benachbarten Sauerstoffatomen enthaltende gegebenenfalls einfach bis zweifach durch Methyl substituierte Alkylendioxyl-Gruppe substituiert ist, wobei ein weiterer 5- Ring gebildet wird, (hervorgehoben stehen A und D gemeinsam für C₃-C₅-Alkandiyl, welches gegebenenfalls durch eine mit zwei nicht direkt benachbarten Sauerstoffatomen enthaltende Alkylendioxy-Gruppe substituiert ist, wobei ein weiterer 5- Ring gebildet wird), oder
- A und Q¹: gemeinsam insbesondere bevorzugt für C₃-C₄-Alkandiyl stehen,
- Q²: insbesondere bevorzugt für Wasserstoff steht,
- G: insbesondere bevorzugt für Wasserstoff (a) oder für eine der Gruppen 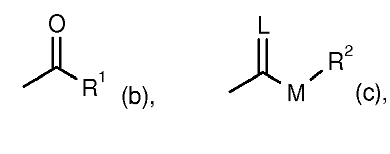
steht,
in welchen
- L: für Sauerstoff steht,
- M: für Sauerstoff steht,
- R¹: insbesondere bevorzugt für C₁-C₆-Alkyl steht,
- R²: insbesondere bevorzugt für C₁-C₆-Alkyl steht,

G auch insbesondere bevorzugt für die Gruppe (g) 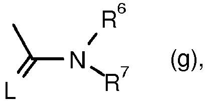 steht,
wobei L für Sauerstoff steht und
- R⁶ und R⁷: zusammen für einen C₅-Alkylenrest stehen, in welchem ein Kohlenstoffatom durch Sauerstoff ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische sind in erfindungsgemäßen Mitteln einsetzbar und lassen sich in ihrer Wirkung durch erfindungsgemäße Ammonium- oder Phosphoniumsalze steigern. Im folgenden wird der Einfachheit halber stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1), (2), (6) und (8) der Gruppe CKE ergeben sich folgende hauptsächliche Strukturen : 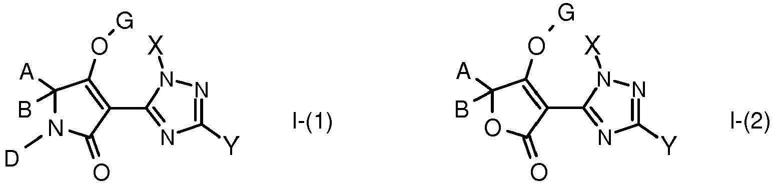 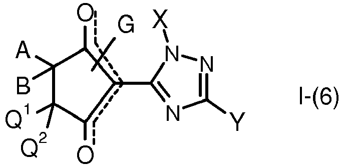 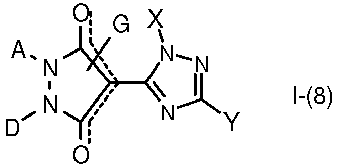 worin
A, B, D, G, Q¹, Q², X und Y die oben angegebene Bedeutung haben.

Unter Einbeziehung der verschiedenen Bedeutungen (a), (b), (c)und (g) der Gruppe G ergeben sich folgende hauptsächliche Strukturen (I-1-a) bis (I-1-g), wenn CKE für die Gruppe (1) steht, 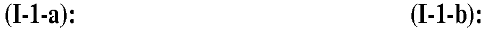 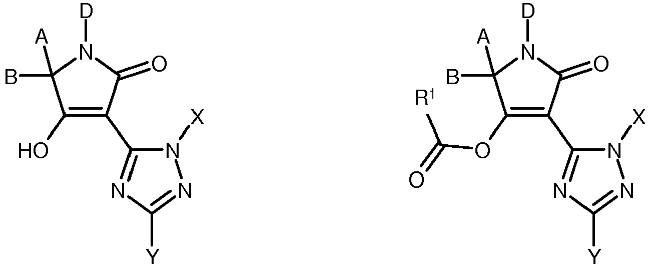 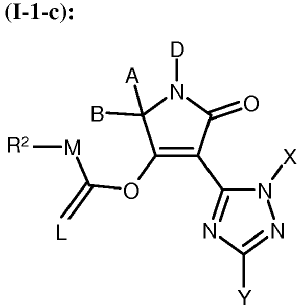 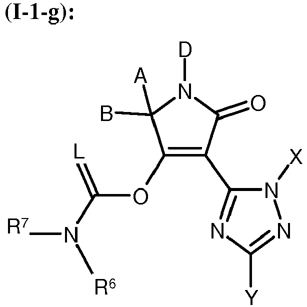 worin
A, B, D, L, M, X, Y, R¹, R², R⁶ und R⁷ die oben angegebenen Bedeutungen besitzen.

Es wurde gefunden, dass der Triazolteil der Verbindungen der Formel (I-1-a) im Kristall in nicht-aromatisierter Form vorliegt und sich folgende Struktur ergibt: 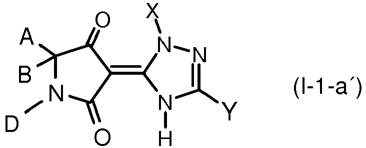 wobei X, Y, A, B und D die oben angegebenen Bedeutungen haben.

Die vorliegende Erfindung umfasst somit neben den Verbindungen der Formel (I-1-a) auch die Verbindungen der Formel (I-1-a').

Unter Einbeziehung der verschiedenen Bedeutungen (a), (b), (c)und (g) der Gruppe G ergeben sich folgende hauptsächliche Strukturen (I-2-a) bis (I-2-g), wenn CKE für die Gruppe (2) steht, 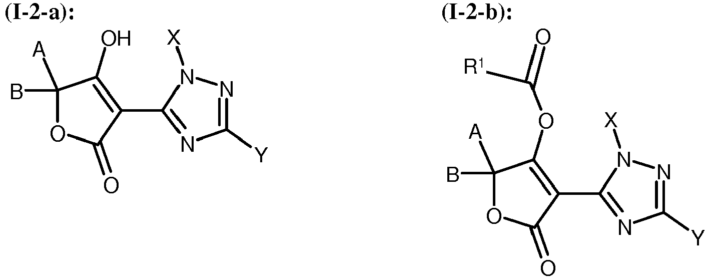 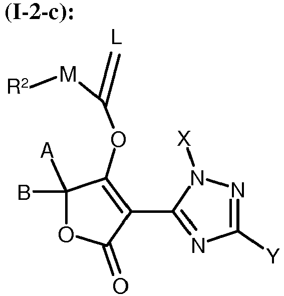 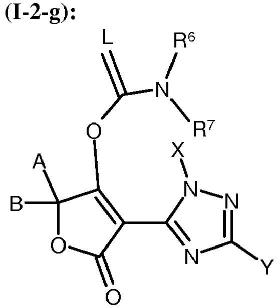 worin
A, B, D, L, M, X, Y, R¹, R², R⁶ und R⁷ die oben angegebene Bedeutung haben.

Die Verbindungen der Formel (1-6) können in Abhängigkeit von der Stellung des Substituenten G in den zwei isomeren Formen der Formeln (I-6-A) und (I-6-B) vorliegen, 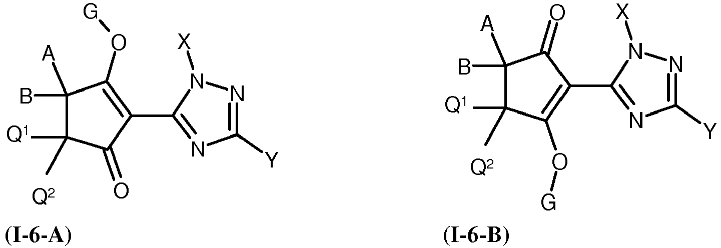 was durch die gestrichelte Linie in der Formel (1-6) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c) und (g) der Gruppe G ergeben sich folgende hauptsächlichen Strukturen (I-6-a) bis (I-6-g), wenn CKE für die Gruppe (6) steht, 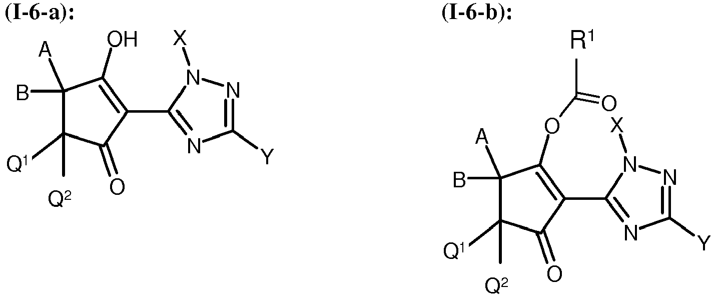 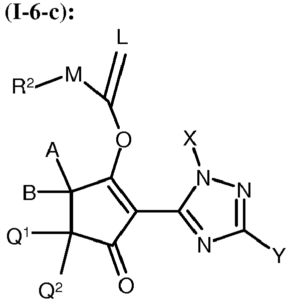 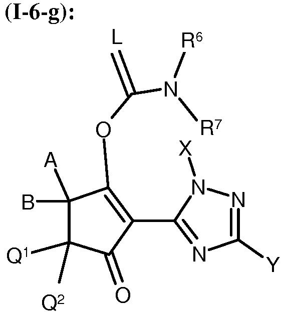 worin
A, B, D, L, M, X, Y, R¹, R², R⁶ und R⁷ die oben angegebenen Bedeutungen besitzen.

Die Verbindungen der Formel (1-8) können in Abhängigkeit von der Stellung des Substituenten G in den zwei isomeren Formeln (I-8-A) und (I-8-B) vorliegen, 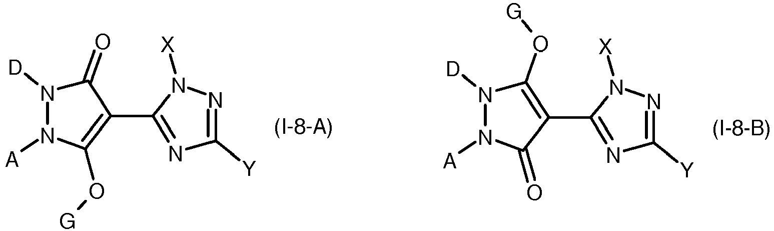 was durch die gestrichelte Linie in der Formel (1-8) zum Ausdruck gebracht werden soll.

Die Verbindungen der Formeln (I-8-A) und (I-8-B) können sowohl als Gemische als auch in Form ihrer reinen Isomeren vorliegen. Gemische der Verbindungen der Formel (I-8-A) und (I-8-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen

Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c)und (g) der Gruppe G ergeben sich folgende hauptsächliche Strukturen (I-8-a) (bis (I-8-g), wenn CKE für die Gruppe (8)
steht, 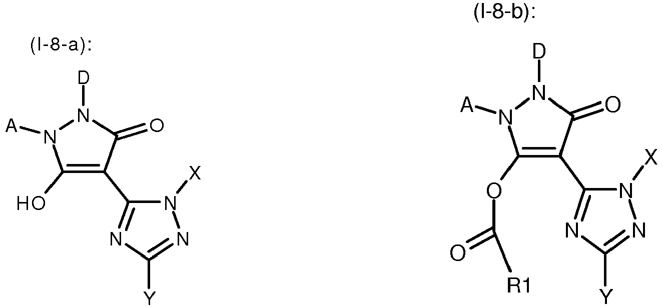 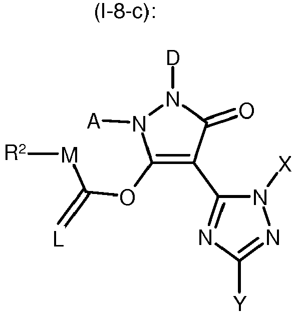 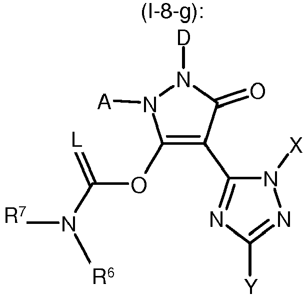 worin
A, B, D, L, M, X, Y, R¹, R², R⁶ und R⁷ die oben angegebenen Bedeutungen besitzen.

Es wurde gefunden, dass der Triazolteil der Verbindungen der Formeln (I-1-a), (I-2-a), (I-6-a), (I-8-a)im Kristall in nicht-aromatisierter Form vorliegt und sich folgende Strukturen ergeben: 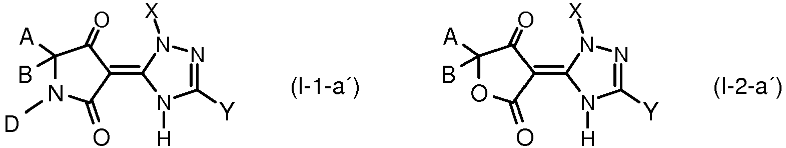 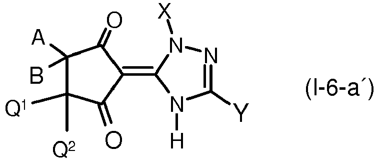 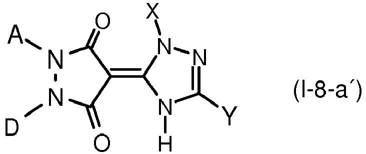 wobei X, Y, A, B, D, Q¹ und Q² die oben angegebenen Bedeutungen haben.

Die vorliegende Erfindung umfasst somit neben den Verbindungen der Formeln (I-1-a), (I-2-a), (I-6-a), (I-8-a) auch die Verbindungen der Formeln (I-1-a'), (I-2-a'), (I-6-a')(I-8-a')

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
"Alkoxy" bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Der Begriff "Aryl" bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3-oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2-oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1-oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5-oder 6- oder 7-yl; 1H-Azepin-1- oder -2- oder 3- oder 4- oder 5- oder 6- oder 7-yl; 2H-Azepin-2- oder 3-oder 4- oder 5- oder 6- oder 7-yl; 3H-Azepin-2- oder 3- oder 4- oder 5- oder 6- oder 7-yl; 4H-Azepin-2-oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2-oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4-oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5-oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thüranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4-oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4-oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5-oder 6-yl; 1,2,3,4-Tetrahydropyrazin-1- oder 2- oder 3- oder 4- oder 5- oder 6-yl; 1,2-Dihydropyrazin-1-oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2-oder 4-yl; 1,3-Dioxan-2- oder 4- oder 5-yl; 4H-1,3-Dioxin-2- oder 4- oder 5- oder 6-yl; 1,4-Dioxan-2-oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2-oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2-oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3-oder 4- oder 5- oder 6-yl; 6H-1,2-Oxazin-3- oder 4- oder 5- oder 6-yl; 4H-1,2-Oxazin-3- oder 4- oder 5-oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5-oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2-oder 3- oder 4- oder 5- oder 6- oder 7-yl; 2,5-Dihydro-1,2-oxazepin-2- oder 3- oder 4- oder 5- oder 6-oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5-oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5-oder 6- oder 7-yl; 1,3-Oxazepin-2- oder 4- oder 5- oder 6- oder 7-yl; 1,4-Oxazepan-2- oder 3- oder 5-oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2-oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5-oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5-oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5-oder 6- oder 7-yl; 5H-1,4,2-Dioxazepin-3- oder 5- oder 6- oder 7-yl; 7H-1,4,2-Dioxazepin-3- oder 5-oder 6- oder 7-yl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Erfindungsgemäß steht der Ausdruck "Hetaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod.

Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest.

"Halogenalkyl" bedeutet durch gleiche oder verschiedene Halogenatome, teilweise oder vollständig substituiertes Alkyl wie z. B. CH₂CH₂Cl, CH₂CH₂Br, CHClCH₃, CH₂Cl, CH₂F, Perhaloalkyl wie z. B. CCl₃, CClF₂, CFCl₂,CF₂CClF₂, CF₂CClFCF₃, Polyhaloalkyl wie z. B. CH₂CHFCl, CF₂CClFH, CF₂CBrFH, CH₂CF₃.

Weiterhin wurde gefunden, dass man die neuen Verbindungen der Formel (I) nach einem der im folgenden beschriebenen Verfahren erhält:
(A) Man erhält substituierte 3-(1-2-4-Triazolyl)-pyrrolidin-2,4-dione bzw. deren Enole der Formel (I-1-a) 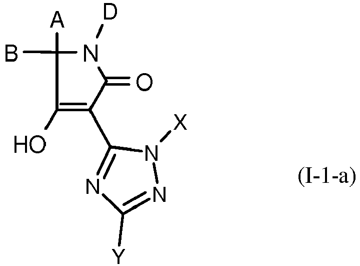 in welcher
   A, B, D, X und Y die oben angegebenen Bedeutungen haben,
   wenn man
   N-Acylaminosäureester der Formel (II) 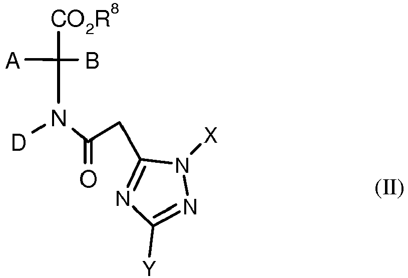 in welcher
   A, B, D, X und Y die oben angegebenen Bedeutungen haben,
      und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-(1-2-4-Triazolyl)-4-hydroxy-Δ³-dihydrofuranon-Derivate der Formel (I-2-a) 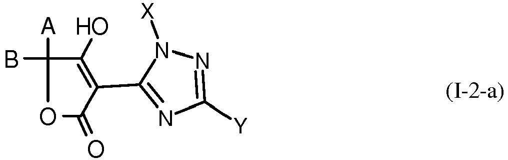 in welcher
   A, B, X und Y die oben angegebenen Bedeutungen haben,
   erhält, wenn man
   Carbonsäureester der Formel (III) 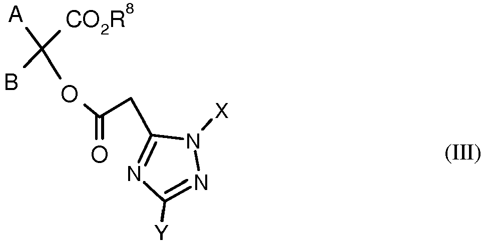 in welcher
   A, B, X, Y, und R⁸ die oben angegebenen Bedeutungen haben,
   in Gegenwart eines Verdünnungsmittels und in Gegenwart einer Base intramolekular kondensiert.

Weiterhin wurde gefunden,
(F) dass man Verbindungen der Formel (I-6-a) 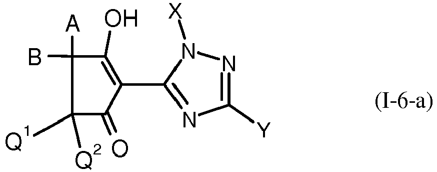 in welcher
   A, B, Q¹, Q², X und Y die oben angegebene Bedeutung haben,
   erhält, wenn man
   Ketocarbonsäureester der Formel (VIII) 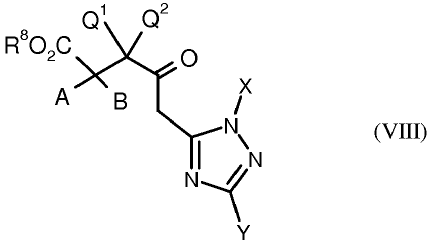 in welchen
   A, B, Q¹, Q², X und Y die oben angegebene Bedeutung haben, und
   R⁸ für Alkyl (insbesondere C₁-C₈-Alkyl) steht,
   gegebenenfalls in Gegenwart eines Verdünnungsmittels und in Gegenwart einer Base intramolekular cyclisiert.
(H) Weiterhin wurde gefunden, dass man die Verbindungen der Formel (I-8-a) 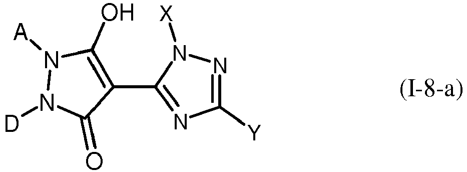 in welcher
   A, D, X und Y die oben angegebenen Bedeutungen haben,
   erhält, wenn man
   Verbindungen der Formel (X) 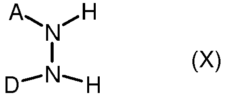 in welcher
   A und D die oben angegebene Bedeutung haben,
   α) mit Verbindungen der Formel (VI) 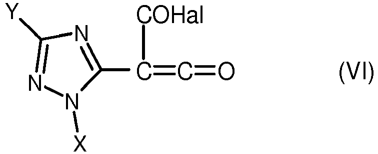 in welcher
      Hal, X und Y die oben angegebenen Bedeutungen haben,
      gegebenenfalls in Gegenwart eines Verdünnungsmittels und gegebenenfalls in Gegenwart eines Säureakzeptors umsetzt, oder
   ß) mit Verbindungen der Formel (XI) 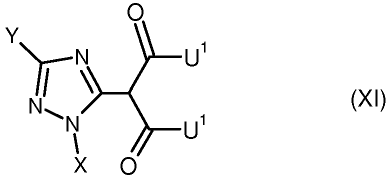 in welcher
      X und Y die oben angegebene Bedeutung haben,
      und U¹ für NH₂ oder O-R⁸ steht, wobei R⁸ die oben genannte Bedeutung hat,
      gegebenenfalls in Gegenwart eines Verdünnungsmittels und gegebenenfalls in Gegenwart einer Base umsetzt, oder
   γ) Verbindungen der Formel (XII) 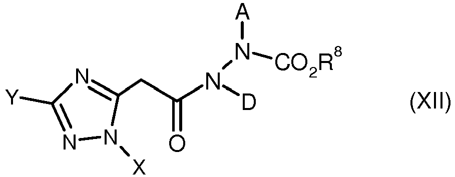 in welcher
      A, D, X, Y und R⁸ die oben angegebene Bedeutung haben,
      gegebenenfalls in Gegenwart eines Verdünnungsmittels und gegebenenfalls in Gegenwart einer Base umsetzt.

Außerdem wurde gefunden
(L) dass man die Verbindungen der oben gezeigten Formeln (I-1-b), (I-2-b), (I-6-b), (I-8-b), in welchen A, B, D, Q¹, Q², R¹, X und Y die oben angegebenen Bedeutungen haben, erhält, wenn man Verbindungen der oben gezeigten Formeln (I-1-a), (I-2-a), (I-6-a), (I-8-a), in welchen A, B, D, Q¹, Q², X und Y die oben angegebenen Bedeutungen haben, jeweils
   (α) mit Säurehalogeniden der Formel (XVI) 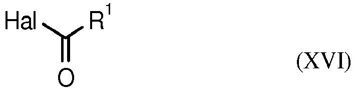 in welcher
      - R¹: die oben angegebene Bedeutung hat und
      - Hal: für Halogen (insbesondere Chlor oder Brom) steht
      oder
   (ß) mit Carbonsäureanhydriden der Formel (XVII)

      R¹-CO-O-CO-R¹ (XVII)

      in welcher
      - R¹: die oben angegebene Bedeutung hat, gegebenenfalls in Gegenwart eines Verdünnungsmittels und gegebenenfalls in Gegenwart eines Säurebindemittels umsetzt;
      - (M): dass man die Verbindungen der oben gezeigten Formeln (I-1-c), (I-2-c), (I-6-c), (I-8-c), in welchen A, B, D, Q¹ Q², R², M, X, und Y die oben angegebenen Bedeutungen haben und L für Sauerstoff steht, erhält, wenn man Verbindungen der oben gezeigten Formeln (I-1-a), (I-2-a), (I-6-a), (I-8-a), in welchen A, B, D, Q¹, Q², X und Y die oben angegebenen Bedeutungen haben, jeweils
      mit Chlorameisensäureestern der Formel (XVIII)

      R²-M-CO-Cl (XVIII)

      in welcher
      R² und M die oben angegebenen Bedeutungen haben,
      gegebenenfalls in Gegenwart eines Verdünnungsmittels und gegebenenfalls in Gegenwart eines Säurebindemittels umsetzt;
(R) dass man Verbindungen der oben gezeigten Formeln (I-1-g), (I-2-g), (I-6-g), (I-8-g), in welchen A, B, D, L, Q¹, Q²R⁶, R⁷, X und Y die oben angegebenen Bedeutungen haben, erhält, wenn man Verbindungen der oben gezeigten Formeln (I-1-a), (I-2-a), (I-6-a), (I-8-a), in welchen A, B, D, Q¹, Q², X und Y die oben angegebenen Bedeutungen haben, jeweils
   (α) mit Isocyanaten der Formel (XXIV)

      R⁶-N=C=L (XXIV)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   (ß) mit Carbamidsäurechloriden der Formel (XXV) 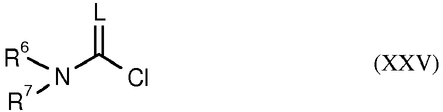 in welcher
      L, R⁶ und R⁷ die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte 1,2,4-Triazolyl-substituierte Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Raps, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher CKE, X und Y die oben angegebene Bedeutung haben
   und
(b') zumindest eine die Kulturpflanzen-Verträglichkeit verbessernde Verbindung (Safener).
   Die Safener sind vorzugsweise ausgewählt aus der Gruppe bestehend aus:
   S1) Verbindungen der Formel (S1), 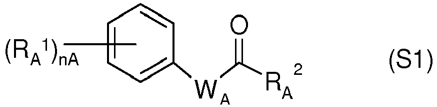 wobei die Symbole und Indizes folgende Bedeutungen haben:
      - n_{A}: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl;
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 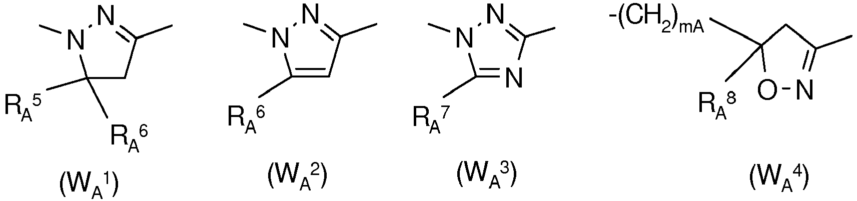
      - m_{A}: ist 0 oder 1;
      - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
      - R_{A}³: ist Wasserstoff oder ein unsubstituierter oder substituierter aliphatischer Kohlenwasserstoffrest, vorzugsweise mit insgesamt 1 bis 18 C-Atomen;
      - R_{A}⁴: ist Wasserstoff, (C₁-C₆)Alkyl, (C₁-C₆)Alkoxy oder substituiertes oder unsubstituiertes Phenyl;
      - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
      - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
      vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-ethoxycarbonyl)-5-methyl-2-pyrazolin-3-carbonsäure, 1-(2,4-Dichlorphenyl)-5-ethoxycarbonyl)-5-methyl-2-pyrazolin-3-carbon-säureethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}) vorzugsweise Verbindungen wie 1-(2,4-Dichlorphenyl)-5-methyl-pyrazol-3-carbonsäureethylester (S 1-2), 1-(2,4-Dichlorphenyl)-5-isopropyl-pyrazol-3-carbonsäureethylester (S 1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S 1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-richlormethyl-(1H)-1,2,4-triazol-3-carbonsäureethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
   S2) Chinolinderivate der Formel (S2), 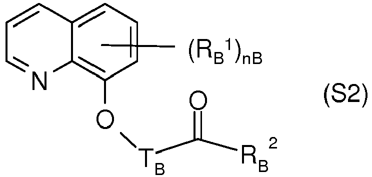 wobei die Symbole und Indizes folgende Bedeutungen haben:
      - R_{B}¹: ist Halogen, (C₁-C₄)Alkyl, (C₁-C₄)Alkoxy, Nitro oder (C₁-C₄)Haloalkyl;
      - n_{B}: ist eine natürliche Zahl von 0 bis 5, vorzugsweise 0 bis 3;
      - R_{B}²: ist OR_{B}³, SR_{B}³ oder NR_{B}³R_{B}⁴ oder ein gesättigter
      oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³_{;}
      - R_{B}³: ist Wasserstoff oder ein unsubstituierter oder substituierter aliphatischer Kohlenwasserstoffrest, vorzugsweise mit insgesamt 1 bis 18 C-Atomen;
      - R_{B}⁴: ist Wasserstoff, (C₁-C₆)Alkyl, (C₁-C₆)Alkoxy oder substituiertes oder unsubstituiertes Phenyl;
      - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
      vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-l-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
   S3) Verbindungen der Formel (S3) 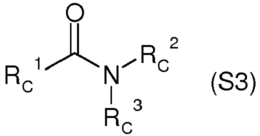 wobei die Symbole und Indizes folgende Bedeutungen haben:
      - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
      - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
      vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4),
      "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8),
      "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
      "Furilazol" oder "MON 13900" ((RS)-3-Dichloracetyl-5-(2-furyl)-2,2-dimethyloxazolidin) (S3-10); sowie dessen (R)-Isomer (S3-11).
   S4) N-Acylsulfonamide der Formel (S4) und ihre Salze, 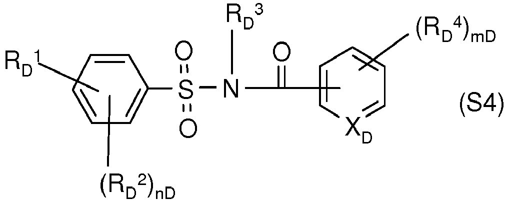 worin die Symbole und Indizes folgende Bedeutungen haben:
      - X_{D}: ist CH oder N;
      - R_{D}¹: ist CO-NR_{D}⁵R_{D}⁶ oder NHCO-R_{D}⁷;
      - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
      - R_{D}³: ist Wasserstoff, (C₁-C₄)Alkyl, (C₂-C₄)Alkenyl oder (C₂-C₄)Alkinyl;
      - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
      - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
      - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
      - R_{D}⁵ und R_{D}⁶: gemeinsam mit dem sie tragenden Stickstoffatom einen Pyrrolidinyl- oder Piperidinyl-Rest bilden;
      - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - n_{D}: ist 0, 1 oder 2;
      - m_{D}: ist 1 oder 2;
      - v_{D}: ist 0, 1, 2 oder 3;
      davon bevorzugt sind Verbindungen von Typ der N-Acylsulfonamide, z.B. der nachfolgenden Formel (S4^{a}), die z. B. bekannt sind aus WO-A-97/45016 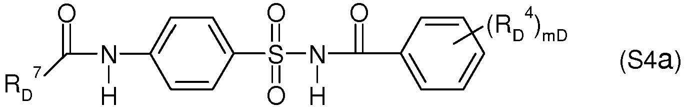 worin
      - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - R_{D}⁴: Halogen, (C₁-C₄)Alkyl, (C₁-C₄)Alkoxy, CF₃;
      - m_{D}: 1 oder 2;
      - v_{D}: ist 0, 1, 2 oder 3 bedeutet;
      sowie
      Acylsulfamoylbenzoesäureamide, z.B. der nachfolgenden Formel (S4^{b}), die z.B. bekannt sind aus WO-A-99/16744, 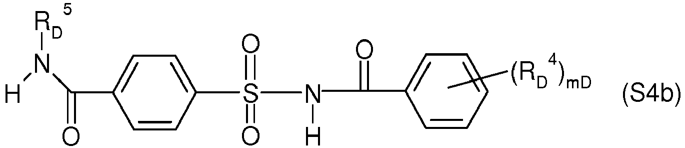
      z.B. solche worin
      R_{D}⁵ = Cyclopropyl und (R_{D}⁴) = 2-OMe ist ("Cyprosulfamide", S4-1),
      R_{D}⁵ = Cyclopropyl und (R_{D}⁴) = 5-Cl-2-OMe ist (S4-2),
      R_{D}⁵ = Ethyl und (R_{D}⁴) = 2-OMe ist (S4-3),
      R_{D}⁵ = Isopropyl und (R_{D}⁴) = 5-Cl-2-OMe ist (S4-4) und
      R_{D}⁵ = Isopropyl und (R_{D}⁴) = 2-OMe ist (S4-5)
      sowie
      Verbindungen vom Typ der N-Acylsulfamoylphenylharnstoffe der Formel (S4^{c}), die z.B. bekannt sind aus der EP-A-365484, 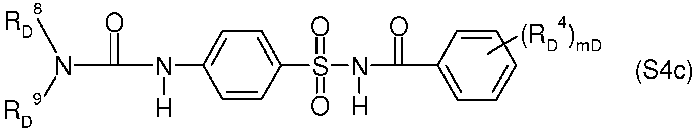 worin
      - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
      - R_{D}⁴: Halogen, (C₁-C₄)Alkyl, (C₁-C₄)Alkoxy, CF₃
      - m_{D}: 1 oder 2 bedeutet;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff.
   S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
   S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
   S7) Verbindungen der Formel (S7),wie sie in der WO-A-1998/38856 beschrieben sind 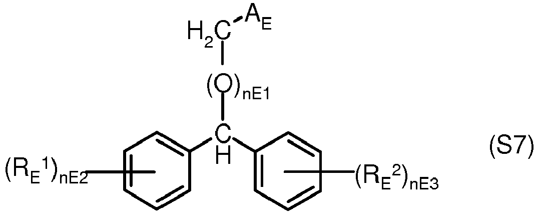 worin die Symbole und Indizes folgende Bedeutungen haben:
      - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
      - A_{E}: ist COOR_{E}³ oder COSR_{E} ⁴
      - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
      - n_{E}¹: ist 0 oder 1
      - n_{E}², n_{E}³: sind unabhängig voneinander 0, 1 oder 2,
      vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
   S8) Verbindungen der Formel (S8),wie sie in der WO-A-98/27049 beschrieben sind 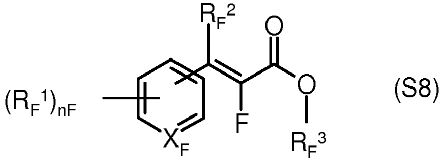 worin
      - X_{F}: CH oder N,
      - n_{F}: für den Fall, dass X_{F}=N ist, eine ganze Zahl von 0 bis 4 und für den Fall, dass X_{F}=CH ist, eine ganze Zahl von 0 bis 5 ,
      - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
      - R_{F}²: Wasserstoff oder (C₁-C₄)Alkyl
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
      vorzugsweise Verbindungen worin
      - X_{F}: CH,
      - n_{F}: eine ganze Zahl von 0 bis 2 ,
      - R_{F}¹: Halogen, (C₁-C₄)Alkyl, (C₁-C₄)Haloalkyl, (C₁-C₄)Alkoxy, (C₁-C₄)Haloalkoxy,
      - R_{F}²: Wasserstoff oder (C₁-C₄)Alkyl,
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
   S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
   S10) Verbindungen der Formeln (S10^{a}) oder (S10^{b})
      wie sie in der WO-A-2007/023719 und WO-A-2007/023764 beschrieben sind 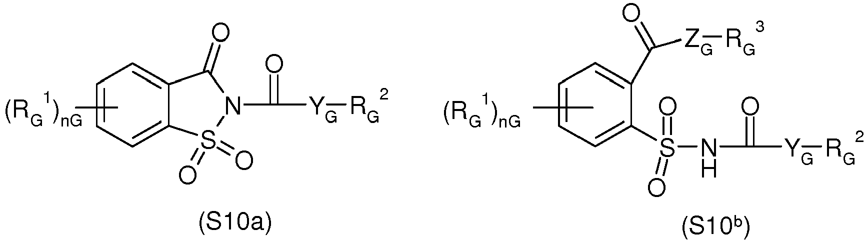 worin
      - R_{G}¹: Halogen, (C₁-C₄)Alkyl, Methoxy, Nitro, Cyano, CF₃, OCF₃
      - Y_{G}, Z_{G}: unabhängig voneinander O oder S,
      - n_{G}: eine ganze Zahl von 0 bis 4,
      - R_{G}²: (C₁-C₁₆)Alkyl, (C₂-C₆)Alkenyl, (C₃-C₆)Cycloalkyl, Aryl; Benzyl, Halogenbenzyl,
      - R_{G}³: Wasserstoff oder (C₁-C₆)Alkyl bedeutet.
   S11) Wirkstoffe vom Typ der Oxyimino-Verbindungen (S11), die als Saatbeizmittel bekannt sind, wie z. B. "Oxabetrinil" ((Z)-1,3-Dioxolan--ylmethoxyimino(phenyl)acetonitril) (S11-1), das als Saatbeiz-Safener für Hirse gegen Schäden von Metolachlor bekannt ist,
      "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
      "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
   S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
   S13) Eine oder mehrere Verbindungen aus Gruppe (S13):
      "Naphthalic anhydrid" (1,8-Naphthalindicarbonsäureanhydrid) (S13-1), das als Saatbeiz-Safener für Mais gegen Schäden von Thiocarbamatherbiziden bekannt ist,
      "Fenclorim" (4,6-Dichlor-2-phenylpyrimidin) (S13-2), das als Safener für Pretilachlor in gesätem Reis bekannt ist,
      "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
      "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
      "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
      "MG-838" (CAS-Reg.Nr. 133993-74-5) (2-propenyl 1-oxa-4-azaspiro[4.5]decane-4-carbodithioate) (S13-6) der Firma Nitrokemia,
      "Disulfoton" (O,O-Diethyl S-2-ethylthioethyl phosphordithioat) (S13-7),
      "Dietholate" (O,O-Diethyl-O-phenylphosphorotioat) (S13-8),
      "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (*S*-1-Methyl-1-phenylethyl-piperidin-1-carbothioat), das als Safener für Reis gegen Schäden des Herbizids Molinate bekannt ist,
      "Daimuron" oder "SK 23" (1-(1-Methyl-1-phenylethyl)-3-p-tolyl-harnstoff), das als Safener für Reis gegen Schäden des Herbizids Imazosulfuron bekannt ist,
      "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
      "Methoxyphenon" oder "NK 049" (3,3'-Dimethyl-4-methoxy-benzophenon), das als Safener für Reis gegen Schäden einiger Herbizide bekannt ist,
      "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
   S15) Wirkstoffe, die vorrangig als Herbizide eingesetzt werden, jedoch auch Safenerwirkung auf Kulturpflanzen aufweisen, z.B.
      (2,4-Dichlorphenoxy)essigsäure (2,4-D),
         (4-Chlorphenoxy)essigsäure,
         (R,S)-2-(4-Chlor-o-tolyloxy)propionsäure (Mecoprop),
         4-(2,4-Dichlorphenoxy)buttersäure (2,4-DB),
         (4-Chlor-o-tolyloxy)essigsäure (MCPA),
         4-(4-Chlor-o-tolyloxy)buttersäure,
         4-(4-Chlorphenoxy)buttersäure,
         3,6-Dichlor-2-methoxybenzoesäure (Dicamba),
         1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Mefenpyr-diethyl besonders hervorgehoben ist. Cyprosulfamide (S4-1) ist ebenfalls hervorgehoben.

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- X: steht hervorgehoben für Methyl oder Ethyl,
- Y: steht hervorgehoben für 4-F-Phenyl oder 4-Cl-Phenyl,

CKE steht hervorgehoben für eine der Gruppen 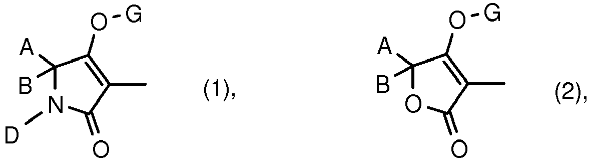
- A: steht hervorgehoben für Wasserstoff oder Methyl,
- B: steht hervorgehoben für Wasserstoff oder Methyl,
- A, B: und das Kohlenstoffatom an das sie gebunden sind, stehen hervorgehoben für gesättigtes C₆-Cycloalkyl, in welchem gegebenenfalls ein Ringglied durch Sauerstoff ersetzt ist und welches gegebenenfalls einfach durch Methyl oder Methoxy substituiert ist,
- D: steht hervorgehoben für Wasserstoff oder cyclo-Propyl, oder
- A und D: stehen gemeinsam hervorgehoben für C₃-Alkandiyl,
- G: steht hervorgehoben für Wasserstoff (a).
- X: steht auch hervorgehoben für Methyl, Ethyl, i-Propyl, n-Propyl, n-Butyl oder -CH₂-CF₃,
- Y: steht auch hervorgehoben für Phenyl, 4-Cl-Benzyl, 4-F-Phenyl, 4-Cl-Phenyl oder 2,4-Cl₂-Phenyl,

CKE steht auch hervorgehoben für eine der Gruppen 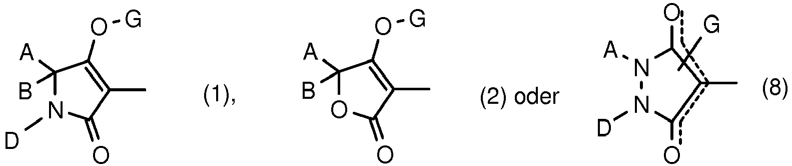
- A: steht auch hervorgehoben für Wasserstoff, Methyl oder cyclo-Propyl,
- B: steht auch hervorgehoben für Wasserstoff, Methyl oder cyclo-Propyl,
- A, B: und das Kohlenstoffatom an das sie gebunden sind, stehen auch hervorgehoben für gesättigtes C₆-Cycloalkyl, in welchem gegebenenfalls ein Ringglied durch Sauerstoff oder Stickstoff ersetzt ist und welches gegebenenfalls einfach oder zweifach durch Methyl, Ethyl, Methoxy, Ethoxy, n-Propoxy, Trifluormethyl oder -O-CH₂CHCH₂ substituiert ist, wobei Methoxy oder Ethoxy auch als N-Substituenten in Frage kommen,
- A, B: und das Kohlenstoffatom, an das sie gebunden sind, stehen auch hervorgehoben für C₆-Cycloalkyl, welches gegebenenfalls durch eine mit zwei nicht direkt benachbarten Sauerstoffatomen enthaltende Alkylendioxyl-Gruppe substituiert ist, wobei ein weiterer 6-Ring gebildet wird, der einfach durch Methyl substituiert sein kann,
- D: steht auch hervorgehoben für Wasserstoff oder cyclo-Propyl, oder
- A und D: stehen gemeinsam auch hervorgehoben für C₃-Alkandiyl,
- A und D: stehen im Fall von CKE = Gruppe (8) gemeinsam auch hervorgehoben für -(CH₂)₂-O-(CH₂)₂-,
- G: steht auch hervorgehoben für Wasserstoff (a) oder für die Gruppe (g) 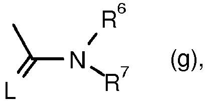
wobei L für Sauerstoff steht und
- R⁶ und R⁷: zusammen für einen C₅-Alkylenrest stehen, in welchem ein Kohlenstoffatom durch Sauerstoff ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Hervorgehoben sind ebenfalls Verbindungen der Formel (I) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im Einzelnen seien außer bei den Beispielen genannten Verbindungen die folgenden Verbindungen genannt:

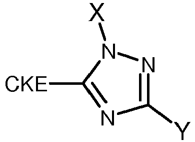

**Tabelle 1**

| **X** | **Y** |
|---|---|
| CH₃ | Ph |
| CH₃ | 4-Cl-C₆H₄ |
| CH₃ | 4-F-C₆H₄ |
| CH₃ | 4-MeO-C₆H₄ |
| CH₃ | 4-CF₃-C₆H₄ |
| CH₃ | 3-Cl-C₆H₄ |
| CH₃ | 3-F-C₆H₄ |
| CH₃ | 3-CF₃-C₆H₄ |
| CH₃ | 3-MeO-C₆H₄ |
| CH₃ | 2-Cl-C₆H₄ |
| CH₃ | 2-MeO-C₆H₄ |
| CH₃ | 2-F-C₆H₄ |
| CH₃ | 2,4-Cl₂-C₆H₃ |
| C₂H₅ | Ph |
| C₂H₅ | 4-Cl-C₆H₄ |
| C₂H | 4-F-C₆H₄ |
| C₂H₅ | 4-MeO-C₆H₄ |
| C₂H₅ | 4-CF₃-C₆H₄ |
| C₂H₅ | 3-Cl-C₆H₄ |
| C₂H₅ | 3-F-C₆H₄ |
| C₂H₅ | 3-CF₃-C₆H₄ |
| C₂H₅ | 3-MeO-C₆H₄ |
| C₂H₅ | 2-Cl-C₆H₄ |
| C₂H₅ | 2-MeO-C₆H₄ |
| C₂H₅ | 2-F-C₆H₄ |
| C₂H₅ | 2,4-Cl₂-C₆H₃ |
| CH₂-Ph | Ph |
| CH₂-Ph | 4-Cl-C₆H₄ |
| CH₂-Ph | 4-F-C₆H₄ |
| CH₂-Ph | 4-MeO-C₆H₄ |
| CH₂-Ph | 4-CF₃-C₆H₄ |
| CH₂-Ph | 3-Cl-C₆H₄ |
| CH₂-Ph | 3-F-C₆H₄ |
| CH₂-Ph | 3-CF₃-C₆H₄ |
| CH₂-Ph | 3-MeO-C₆H₄ |
| CH₂-Ph | 2-Cl-C₆H₄ |
| CH₂-Ph | 2-MeO-C₆H₄ |
| CH₂-Ph | 2-F-C₆H₄ |
| CH₂-Ph | 2,4-Cl₂-C₆H₃ |
| CH(CH₃)₂ | Ph |
| CH(CH₃)₂ | 4-Cl-C₆H₄ |
| CH(CH₃)₂ | 4-F-C₆H₄ |
| CH(CH₃)₂ | 4-MeO-C₆H₄ |
| CH(CH₃)₂ | 4-CF₃-C₆H₄ |
| CH(CH₃)₂ | 3-Cl-C₆H₄ |
| CH(CH₃)₂ | 3-F-C₆H₄ |
| CH(CH₃)₂ | 3-CF₃-C₆H₄ |
| CH(CH₃)₂ | 3-MeO-C₆H₄ |
| CH(CH₃)₂ | 2-Cl-C₆H₄ |
| CH(CH₃)₂ | 2-MeO-C₆H₄ |
| CH(CH₃)₂ | 2-F-C₆H₄ |
| CH(CH₃)₂ | 2,4-Cl₂-C₆H₃ |
| CH₂COOCH₃ | Ph |
| CH₂COOCH₃ | 4-Cl-C₆H₄ |
| CH₂COOCH₃ | 4-F-C₆H₄ |
| CH₂COOCH₃ | 4-MeO-C₆H₄ |
| CH₂COOCH₃ | 4-CF₃-C₆H₄ |
| CH₂COOCH₃ | 3-Cl-C₆H₄ |
| CH₂COOCH₃ | 3-F-C₆H₄ |
| CH₂COOCH₃ | 3-CF₃-C₆H:, |
| CH₂COOCH₃ | 3-MeO-C₆H₄ |
| CH₂COOCH₃ | 2-Cl-C₆H₄ |
| CH₂COOCH₃ | 2-MeO-C₆H₄ |
| CH₂COOCH₃ | 2-F-C₆H₄ |
| CH₂COOCH₃ | 2,4-Cl₂-C₆H₃ |
| CH₂-c-Pr | Ph |
| CH₂-c-Pr | 4-Cl-C₆H₄ |
| CH₂-c-Pr | 4-F-C₆H₄ |
| CH₂-c-Pr | 4-MeO-C₆H₄ |
| CH₂-c-Pr | 4-CF₃-C₆H₄ |
| CH₂-c-Pr | 3-Cl-C₆H₄ |
| CH₂-c-Pr | 3-F-C₆H₄ |
| CH₂-c-Pr | 3-CF₃-C₆H:, |
| CH₂-c-Pr | 3-MeO-C₆H₄ |
| CH₂-c-Pr | 2-Cl-C₆H₄ |
| CH₂-c-Pr | 2-MeO-C₆H₄ |
| CH₂-c-Pr | 2-F-C₆H₄ |
| CH₂-c-Pr | 2,4-Cl₂-C₆H₃ |
| c-Pr | Ph |
| c-Pr | 4-Cl-C₆H₄ |
| c-Pr | 4-F-C₆H₄ |
| c-Pr | 4-MeO-C₆H₄ |
| c-Pr | 4-CF₃-C₆H:, |
| c-Pr | 3-Cl-C₆H₄ |
| c-Pr | 3-F-C₆H₄ |
| c-Pr | 3-CF₃-C₆H₄ |
| c-Pr | 3-MeO-C₆H₄ |
| c-Pr | 2-Cl-C₆H₄ |
| c-Pr | 2-MeO-C₆H₄ |
| c-Pr | 2-F-C₆H₄ |
| c-Pr | 2,4-Cl₂-C₆H₃ |
| CH₂CH₂CH₃ | Ph |
| CH₂CH₂CH₃ | 4-Cl-C₆H₄ |
| CH₂CH₂CH₃ | 4-F-C₆H₄ |
| CH₂CH₂CH₃ | 4-MeO-C₆H₄ |
| CH₂CH₂CH₃ | 4-CF₃-C₆H₄ |
| CH₂CH₂CH₃ | 3-Cl-C₆H₄ |
| CH₂CH₂CH₃ | 3-F-C₆H₄ |
| CH₂CH₂CH₃ | 3-CF₃-C₆H₄ |
| CH₂CH₂CH₃ | 3-MeO-C₆H₄ |
| CH₂CH₂CH₃ | 2-Cl-C₆H₄ |
| CH₂CH₂CH₃ | 2-MeO-C₆H₄ |
| CH₂CH₂CH₃ | 2-F-C₆H₄ |
| CH₂CH₂CH₃ | 2,4-Cl₂-C₆H₃ |
| CH₂CF₃ | Ph |
| CH₂CF₃ | 4-Cl-C₆H₄ |
| CH₂CF₃ | 4-F-C₆H₄ |
| CH₂CF₃ | 4-MeO-C₆H₄ |
| CH₂CF₃ | 4-CF₃-C₆H₄ |
| CH₂CF₃ | 3-Cl-C₆H₄ |
| CH₂CF₃ | 3-F-C₆H₄ |
| CH₂CF₃ | 3-CF₃-C₆H₄ |
| CH₂CF₃ | 3-MeO-C₆H₄ |
| CH₂CF₃ | 2-Cl-C₆H₄ |
| CH₂CF₃ | 2-MeO-C₆H₄ |
| CH₂CF₃ | 2-F-C₆H₄ |
| CH₂CF₃ | 2,4-Cl₂-C₆H₃ |
| CH₂CH₂OCH₃ | Ph |
| CH₂CH₂OCH₃ | 4-Cl-C₆H₄ |
| CH₂CH₂OCH₃ | 4-F-C₆H₄ |
| CH₂CH₂OCH₃ | 4-MeO-C₆H₄ |
| CH₂CH₂OCH₃ | 4-CF₃-C₆H₄ |
| CH₂CH₂OCH₃ | 3-Cl-C₆H₄ |
| CH₂CH₂OCH₃ | 3-F-C₆H₄ |
| CH₂CH₂OCH₃ | 3-CF₃-C₆H₄ |
| CH₂CH₂OCH₃ | 3-MeO-C₆H₄ |
| CH₂CH₂OCH₃ | 2-Cl-C₆H₄ |
| CH₂CH₂OCH₃ | 2-MeO-C₆H₄ |
| CH₂CH₂OCH₃ | 2-F-C₆H₄ |
| CH₂CH₂OCH₃ | 2,4-Cl₂-C₆H₃ |
| CH₂CH₂CH₂CH₃ | Ph |
| CH₂CH₂CH₂CH₃ | 4-Cl-Ph |
| CH₂CH₂CH₂CH₃ | 4-F-Ph |
| CH₂CH₂CH₂CH₃ | 4-MeO-Ph |
| CH₂CH₂CH₂CH₃ | 4-CF₃-Ph |
| CH₂CH₂CH₂CH₃ | 3-Cl-Ph |
| CH₂CH₂CH₂CH₃ | 3-F-Ph |
| CH₂CH₂CH₂CH₃ | 3-CF₃-Ph |
| CH₂CH₂CH₂CH₃ | 3-MeO-Ph |
| CH₂CH₂CH₂CH₃ | 2-Cl-Ph |
| CH₂CH₂CH₂CH₃ | 2-MeO-Ph |
| CH₂CH₂CH₂CH₃ | 2-F-Ph |
| CH₂CH₂CH₂CH₃ | 2,4-Cl₂-Ph |
| Ph | Ph |
| Ph | 4-Cl-C₆H₄ |
| Ph | 4-F-C₆H₄ |
| Ph | 4-MeO-C₆H₄ |
| Ph | 4-CF₃-C₆H₄ |
| Ph | 3-Cl-C₆H₄ |
| Ph | 3-F-C₆H₄ |
| Ph | 3-CF₃-C₆H₄ |
| Ph | 3-MeO-C₆H₄ |
| Ph | 2-Cl-C₆H₄ |
| Ph | 2-MeO-C₆H₄ |
| Ph | 2-F-C₆H₄ |
| Ph | 2,4-Cl₂-C₆H₃ |
| 4-Cl-C₆H₄ | Ph |
| 4-Cl-C₆H₄ | 4-Cl-C₆H₄ |
| 4-Cl-C₆H₄ | 4-F-C₆H₄ |
| 4-Cl-C₆H₄ | 4-MeO-C₆H₄ |
| 4-Cl-C₆H₄ | 4-CF₃-C₆H₄ |
| 4-Cl-C₆H₄ | 3-Cl-C₆H₄ |
| 4-Cl-C₆H₄ | 3-F-C₆H₄ |
| 4-Cl-C₆H₄ | 3-CF₃-C₆H₄ |
| 4-Cl-C₆H₄ | 3-MeO-C₆H₄ |
| 4-Cl-C₆H₄ | 2-Cl-C₆H₄ |
| 4-Cl-C₆H₄ | 2-MeO-C₆H₄ |
| 4-Cl-C₆H₄ | 2-F-C₆H₄ |
| 4-Cl-C₆H₄ | 2,4-Cl₂-C₆H₃ |
| CH₃ | CH₃ |
| CH₃ | CH₂CH₃ |
| CH₃ | i-Pr |
| CH₃ | n-Pr |
| CH₃ | CF₃ |
| CH₃ | CH₂Ph |
| CH₃ | CH₂C₆H₄-4-Cl |
| CH₃ | CH₂C₆H₅ |
| CH₃ | CH₂C₆H₄-4-OMe |
| CH₃ | CH₂C₆H₄-4-CF₃ |
| CH₃ | CH₂C₆H₄-4-F |
| CH₃ | 2-Thienyl |
| CH₃ | 3-Thienyl |
| CH₃ | 2-Furyl |
| CH₃ | 3-Furyl |
| CH₃ | 2-Pyridyl |
| CH₃ | 3-Pyridyl |
| CH₃ | 4-Pyridyl |
| CH₃ | CH₂CH₂Ph |
| CH₂CH₃ | CH₃ |
| CH₂CH₃ | CH₂CH₃ |
| CH₂CH₃ | i-Pr |
| CH₂CH₃ | n-Pr |
| CH₂CH₃ | CF₃ |
| CH₂CH₃ | CH₂Ph |
| CH₂CH₃ | CH₂C₆H₄-4-Cl |
| CH₂CH₃ | CH₂C₆H₅ |
| CH₂CH₃ | CH₂C₆H₄-4-OMe |
| CH₂CH₃ | CH₂C₆H₄-4-CF₃ |
| CH₂CH₃ | CH₂C₆H₃-2,4-Cl₂ |
| CH₂CH₃ | 2-Thienyl |
| CH₂CH₃ | 3-Thienyl |
| CH₂CH₃ | 2-Furyl |
| CH₂CH₃ | 3-Furyl |
| CH₂CH₃ | 2-Pyridyl |
| CH₂CH₃ | 3-Pyridyl |
| CH₂CH₃ | 4-Pyridyl |
| CH₂CH₃ | CH₂CH₂Ph |
| n-Pr | CH₃ |
| n-Pr | CH₂CH₃ |
| n-Pr | i-Pr |
| n-Pr | n-Pr |
| n-Pr | CF₃ |
| n-Pr | CH₂Ph |
| n-Pr | CH₂C₆H₄-4-Cl |
| n-Pr | CH₂C₆H₅ |
| n-Pr | CH₂C₆H₄-4-OMe |
| n-Pr | CH₂C₆H₄-4-Br |
| n-Pr | CH₂C₆H₄-4-F |
| n-Pr | 2-Thienyl |
| n-Pr | 3-Thienyl |
| n-Pr | 2-Furyl |
| n-Pr | 3-Furyl |
| n-Pr | 2-Pyridyl |
| n-Pr | 3-Pyridyl |
| n-Pr | 4-Pyridyl |
| n-Pr | CH₂CH₂Ph |
| n-Bu | CH₃ |
| n-Bu | CH₂CH₃ |
| n-Bu | i-Pr |
| n-Bu | n-Pr |
| n-Bu | CF₃ |
| n-Bu | CH₂Ph |
| n-Bu | CH₂C₆H₄-4-Cl |
| n-Bu | CH₂C₆H₅ |
| n-Bu | CH₂C₆H₄-4-OMe |
| n-Bu | CH₂C₆H₄-4-CF₃ |
| n-Bu | CH₂C₆H₄-4-F |
| n-Bu | 2-Thienyl |
| n-Bu | 3-Thienyl |
| n-Bu | 2-Furyl |
| n-Bu | 3-Furyl |
| n-Bu | 2-Pyridyl |
| n-Bu | 3-Pyridyl |
| n-Bu | 4-Pyridyl |
| CH₂CF₃ | CH3 |
| CH₂CF₃ | CH₂CH₃ |
| CH₂CF₃ | i-Pr |
| CH₂CF₃ | n-Pr |
| CH₂CF₃ | CF₃ |
| CH₂CF₃ | CH₂Ph |
| CH₂CF₃ | CH₂C₆H₄-4-Cl |
| CH₂CF₃ | CH₂C₆H₄-4-Cl |
| CH₂CF₃ | CH₂C₆H₄-4-OMe |
| CH₂CF₃ | 2-Thienyl |
| CH₂CF₃ | 3-Thienyl |
| CH₂CF₃ | 2-Furyl |
| CH₂CF₃ | 3-Furyl |
| CH₂CF₃ | 2-Pyridyl |
| CH₂CF₃ | 3-Pyridyl |
| CH₂CF₃ | 4-Pyridyl |

| | |
|---|---|
| Me = Methyl, Ph = Phenyl | |

Als erfindungsgemäße Wirkstoffe kommen insbesondere bevorzugt Verbindungen aus den in Tabelle 1 genannten Restekombinationen für X und Y mit den in Tabellen 2a und 2b genannten Restekombinationen für A, B und D in Frage:

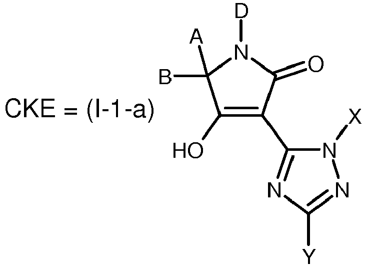

**Tabelle 2a**

| **A** | **B** | **D** |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 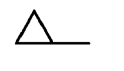 | CH₃ | H |
| 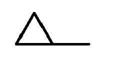 | 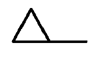 | H |
| 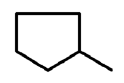 | CH₃ | H |
| 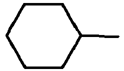 | CH₃ | H |
| H₃CO-CH₂- | CH₃ | H |
| H₅C₂O-CH₂- | CH₃ | H |
| H₃CO-(CH₂)₂- | CH₃ | H |
| H₅C₂O-(CH₂)₂- | CH₃ | H |
| 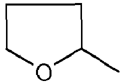 | CH₃ | H |
| 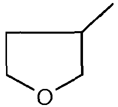 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| 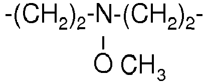 | | H |
| 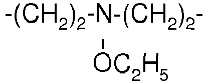 | | H |
| -(CH₂)₂-O-(H₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -CH₂-CHOCH₃-(CH₂)₂- | | H |
| -CH₂-CHOC₂H₅-(CH₂)₂- | | H |
| -CH₂-CHOC₃H₇-(CH₂)₂- | | H |
| -CH₂-CHOC₄H₉-(CH₂)₂- | | H |
| -CH₂-CHO(CH₂)₂OCH₃-(CH₂)₂- | | H |
| 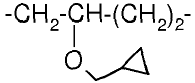 | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO(CH₂)₂OCH₃-(CH₂)3- | | H |
| 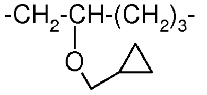 | | H |
| -(CH₂)₂-CHCH₃-(CH₂)₂- | | H |
| -(CH₂)₂-CHCF₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-CH₂CF₃-(CH₂)₂- | | H |
| -(CH₂)₂-C(CH₃)₂-(CH₂)₂- | | H |
| -CH₂-(CHCH₃)₂-(CH₂)₂- | | H |
| 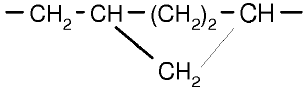 | | H |
| 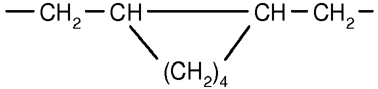 | | H |
| 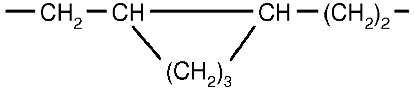 | | H |
| 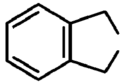 | | H |
| 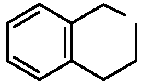 | | H |
| 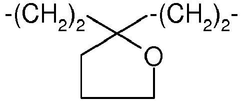 | | H |
| 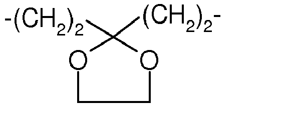 | | H |
| 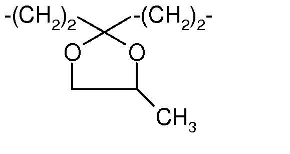 | | H |
| 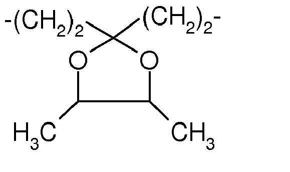 | | H |
| 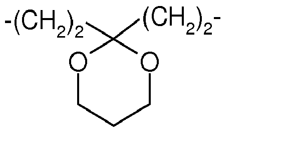 | | H |
| 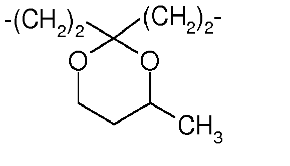 | | H |
| 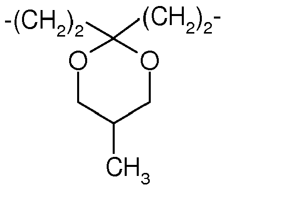 | | H |
| 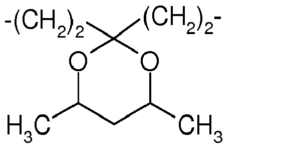 | | H |
| 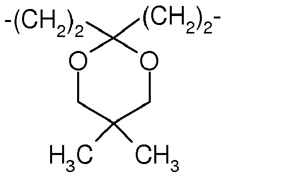 | | H |
| 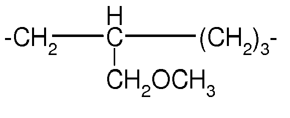 | | H |
| 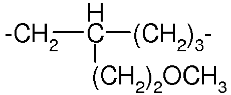 | | H |
| 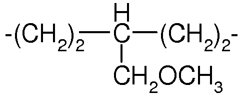 | | H |
| 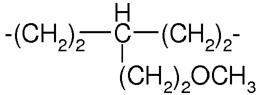 | | H |
| 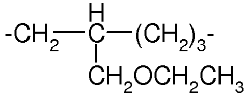 | | H |
| 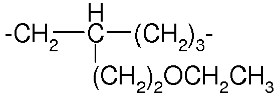 | | H |
| 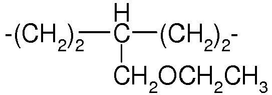 | | H |
| 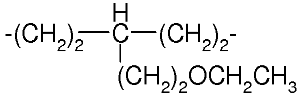 | | H |
| -(CH₂)₂-CO-(CH₂)₂- | | H |
| -(CH₂)₂-CNOMe-(CH₂)₂- | | H |
| -(CH₂)₂-CNOEt-(CH₂)₂- | | H |
| -(CH₂)₂-NOMe-(CH₂)₂- | | H |
| -(CH₂)₂-NOEt-(CH₂)₂- | | H |

**Tabelle 2b**

| **A** | **D** | **B** |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 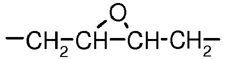 | | H |
| -CH₂-S-CH₂- | | H |
| -CH₂-S-(CH₂)₂- | | H |
| -(CH₂)₂-S-CH₂- | | H |
| 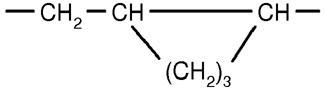 | | H |
| H | CH₃ | H |
| H | C₂H₅ | H |
| H | C₃H₇ | H |
| H | i-C₃H₇ | H |
| H | 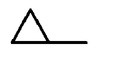 | H |
| H | 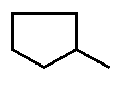 | H |
| H | 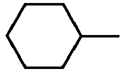 | H |
| CH₃ | CH₃ | H |
| CH₃ | C₂H₅ | H |
| CH₃ | C₃H₇ | H |
| CH₃ | i-C₃H₇ | H |
| CH₃ | 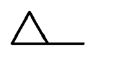 | H |
| CH₃ | 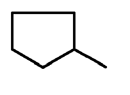 | H |
| CH₃ | 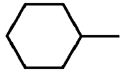 | H |
| C₂H₅ | CH₃ | H |
| C₂H₅ | C₂H₅ | H |
| H | H₃CO-(CH₂)₂- | H |
| H | H₅C₂O-(CH₂)₂- | H |
| H | H3CO-CH₂-CH(CH3)- | H |
| H | H₃CO-CHCH₃-CH₂- | H |
| CH₃ | H₃CO-(CH₂)₂- | H |
| CH₃ | H₅C₂O-(CH₂)₂- | H |
| CH₃ | H₃CO-CH₂-CH(CH₃)- | H |
| CH₃ | H₃CO-CHCH₃-CH₂- | H |

Als erfindungsgemäße Wirkstoffe kommen insbesondere bevorzugt Verbindungen aus den in Tabelle 1 genannten Restekombinationen für X und Y mit den in Tabelle 2c genannten Restekombinationen für A, B, D, R₆ und R₇ in Frage:

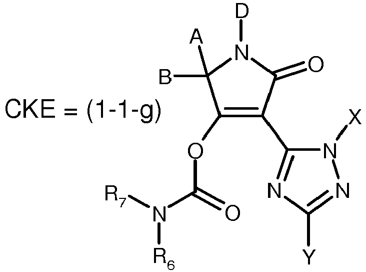

**Tabelle 2c**

| **A** | **B** | **D** | **R₆** | **R₇** |
|---|---|---|---|---|
| CH₃ | CH₃ | H | CH₃ | CH₃ |
| CH₃ | CH₃ | H | C₂H₅ | C₂H₅ |
| CH₃ | CH₃ | H | CH₂CH₂CH₂CH₂ | |
| CH₃ | CH₃ | H | CH₂CH₂CH₂CH₂CH₂ | |
| CH₃ | CH₃ | H | CH₂CH₂OCH₂CH₂ | |
| C₂H₅ | C₂H₅ | H | CH₃ | CH₃ |
| C₂H₅ | C₂H₅ | H | C₂H₅ | C₂H₅ |
| C₂H₅ | C₂H₅ | H | CH₂CH₂CH₂CH₂ | |
| C₂H₅ | C₂H₅ | H | CH₂CH₂CH₂CH₂CH₂ | |
| C₂H₅ | C₂H₅ | H | CH₂CH₂OCH₂CH₂ | |
| -(CH₂)₂-O-(CH₂)₂- | | H | CH₃ | CH₃ |
| -(CH₂)₂-O-(CH₂)₂- | | H | C₂H₅ | C₂H₅ |
| -(CH₂)₂-O-(CH₂)₂- | | H | CH₂CH₂CH₂CH₂ | |
| -(CH₂)₂-O-(CH₂)₂- | | H | CH₂CH₂CH₂CH₂CH₂ | |
| -(CH₂)₂-O-(CH₂)₂- | | H | CH₂CH₂OCH₂CH₂ | |
| -(CH₂)₂-CHOMe-(CH₂)₂- | | H | CH₂CH₂CH₂CH₂ | |
| -(CH₂)₂-CHOMe-(CH₂)₂- | | H | CH₂CH₂CH₂CH₂CH₂ | |
| -(CH₂)₂-CHOMe-(CH₂)₂- | | H | CH₂CH₂OCH₂CH₂ | |
| -(CH₂)₂-CHOMe-(CH₂)₂- | | H | CH₃ | CH₃ |
| -(CH₂)₂-CHOMe-(CH₂)₂- | | H | C₂H₅ | C₂H₅ |
| 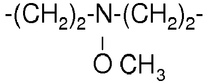 | | H | CH₃ | CH₃ |
| 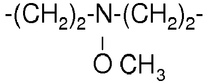 | | H | C₂H₅ | C₂H₅ |
| 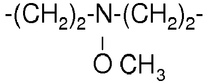 | | H | CH₂CH₂CH₂CH₂ | |
| 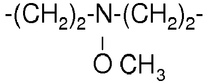 | | H | CH₂CH₂CH₂CH₂CH₂ | |
| 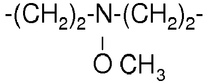 | | H | CH₂CH₂OCH₂CH₂ | |
| 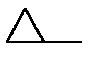 | 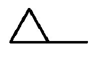 | H | CH₃ | CH₃ |
| 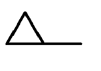 | 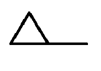 | H | C₂H₅ | C₂H₅ |
| 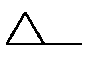 | 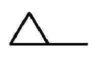 | H | CH₂CH₂CH₂CH₂ | |
| 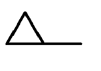 | 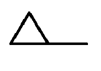 | H | CH₂CH₂CH₂CH₂CH₂ | |
| 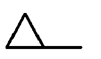 | 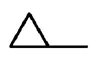 | H | CH₂-CH₂OCH₂CH₂ | |
| -(CH₂)₂-CHCH₃-(CH₂)₂- | | H | CH₃ | CH₃ |
| -(CH₂)₂-CHCH₃-(CH₂)₂- | | H | C₂H₅ | C₂H₅ |
| -(CH₂)₂-CHCH₃-(CH₂)₂- | | H | CH₂CH₂CH₂CH₂ | |
| -(CH₂)₂-CHCH₃-(CH₂)₂- | | H | CH₂CH₂CH₂CH₂CH₂ | |
| -(CH₂)₂-CHCH₃-(CH₂)₂- | | H | CH₂-CH₂OCH₂CH₂ | |

Als erfindungsgemäße Wirkstoffe kommen weiterhin insbesonders bevorzugt Verbindungen aus mit den in Tabelle 1 genannten Restekombinationen für X und Y mit den in Tabelle 3 genannten Restekombinationen für A und B in Frage:

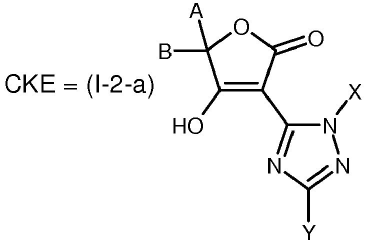

**Tabelle 3**

| **A** | **B** |
|---|---|
| CH₃ | H |
| C₂H₅ | H |
| C₃H₇ | H |
| i-C₃H₇ | H |
| C₄H₉ | H |
| i-C₄H₉ | H |
| S-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| S-C₄H₉ | CH₃ |
| t-C₄H₉ | CH₃ |
| C₂H₅ | C₂H₅ |
| C₃H₇ | C₃H₇ |
| 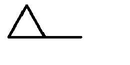 | CH₃ |
| 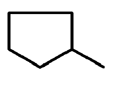 | CH₃ |
| 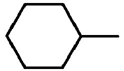 | CH₃ |
| H₃CO-CH₂- | CH₃ |
| HₛC₂O-CH₂- | CH₃ |
| H₃CO-(CH₂)₂- | CH₃ |
| H₅C₂O-(CH₂)₂- | CH₃ |
| 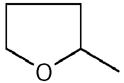 | CH₃ |
| 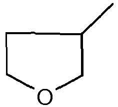 | CH₃ |
| -(CH₂)₃- | |
| -(CH₂)₄- | |
| -(CH₂)₅- | |
| -(CH₂)₆- | |
| -(CH₂)₇- | |
| 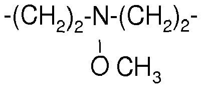 | |
| 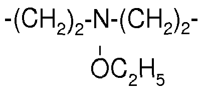 | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -CH₂-CHOCH₃-(CH₂)₃- | |
| -CH₂-CHOC₂H₅-(CH₂)₃- | |
| -CH₂-CHOC₃H₇-(CH₂)₃- | |
| -CH₂-CHOC₄H₉-(CH₂)₃- | |
| -CH₂-CHO(CH₂)₂OCH3-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOC₂H₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-CH₂CF₃-(CH₂)₂- | |
| -(CH₂)₂-C(CH₃)₂-(CH₂)₂- | |
| -CH₂-(CHCH₃)₂-(CH₂)₂- | |
| 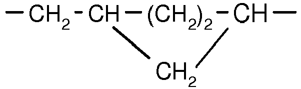 | |
| 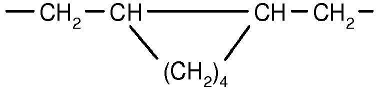 | |
| 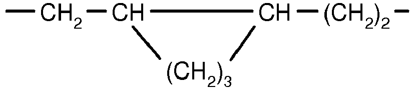 | |
| 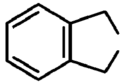 | |
| 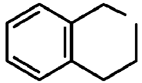 | |
| 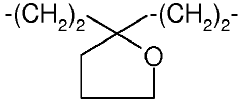 | |
| 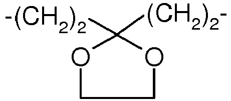 | |
| 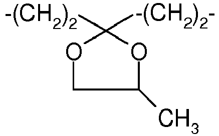 | |
| 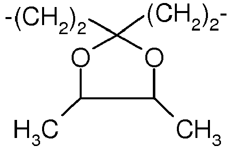 | |
| 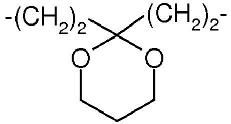 | |
| 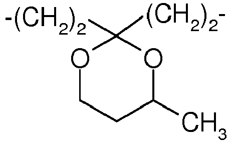 | |
| 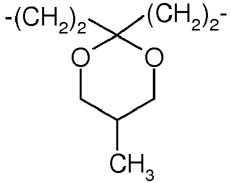 | |
| 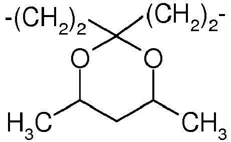 | |
| 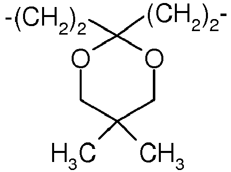 | |
| 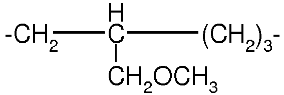 | |
| 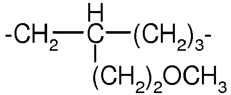 | |
| 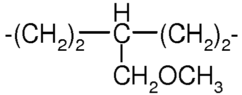 | |
| 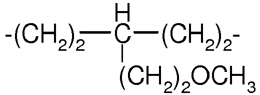 | |
| 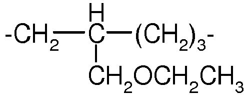 | |
| 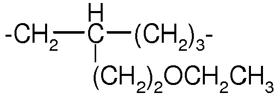 | |
| 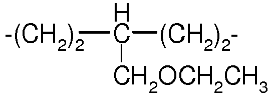 | |
| 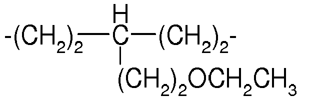 | |

Als erfindungsgemäße Wirkstoffe kommen insbesondere bevorzugt Verbindungen aus den in Tabelle 1 genannten Restekombinationen für X und Y mit den in Tabelle 4 genannten Restekombinationen für A und D in Frage:

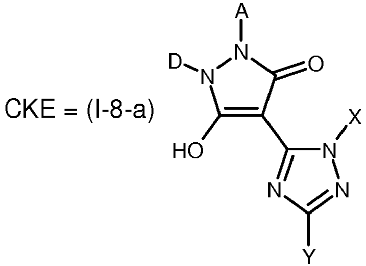

**Tabelle 4**

| **A** | **D** |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 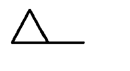 | CH₃ |
| 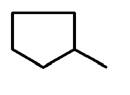 | CH₃ |
| 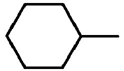 | CH₃ |
| H₃CO-CH₂- | CH₃ |
| H₅C₂O-CH₂- | CH₃ |
| H₃CO-(CH₂)₂- | CH₃ |
| H₅C₂O-(CH₂)₂- | CH₃ |
| 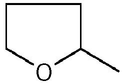 | CH₃ |
| 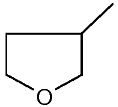 | CH₃ |
| -(CH₂)₃- | |
| -(CH₂)₄- | |
| -(CH₂)₅- | |
| -(CH₂)₆- | |
| -(CH₂)₇- | |
| 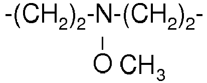 | |
| 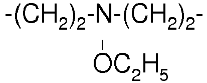 | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)₂-NMe-(CH₂)₂- | |
| -CH₂-CHOCH₃-(CH₂)₃- | |
| -CH₂-CHOC₂H₅-(CH₂)₃- | |
| -CH₂-CHOC₃H₇-(CH₂)₃- | |
| -CH₂-CHOC₄H₉-(CH₂)₃- | |
| -CH₂-CHO(CH₂)₂OCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-CH₂CF₃-(CH₂)₂- | |
| -(CH₂)₂-C(CH₃)₂-(CH₂)₂- | |
| -CH₂-(CHCH₃)₂-(CH₂)₂- | |
| 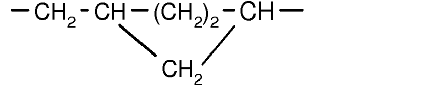 | |
| 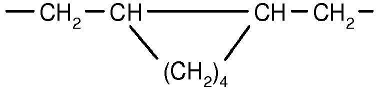 | |
| 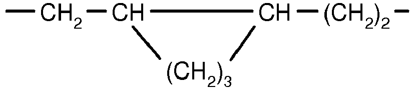 | |
| 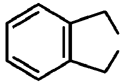 | |
| 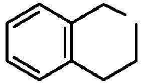 | |
| 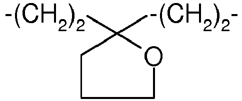 | |
| 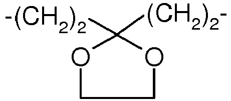 | |
| 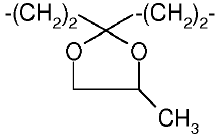 | |
| 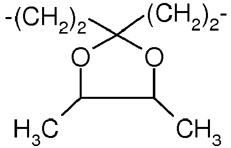 | |
| 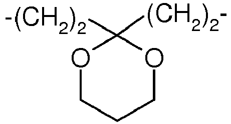 | |
| 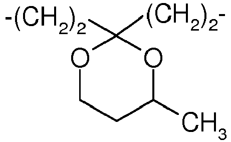 | |
| 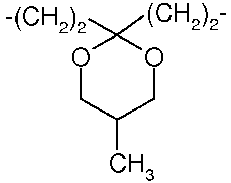 | |
| 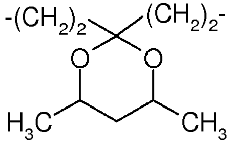 | |
| 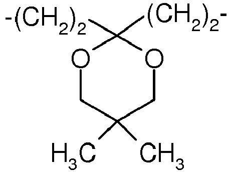 | |
| 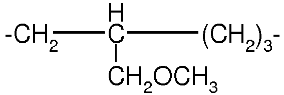 | |
| 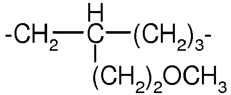 | |
| 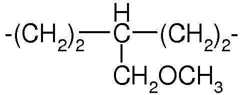 | |

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkyl-sulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Verwendet man beispielsweise gemäß Verfahren (A) Ethyl-1-({[3-(4-chlorphenyl)-1-methyl-1H-1,2,4-triazol-5-yl]acetyl}amino)cyclohexancarboxylat als Ausgangsstoff, so kann der Verlauf des erfindungsgemäßen Verfahrens durch folgendes Reaktionsschema wiedergegeben werden:

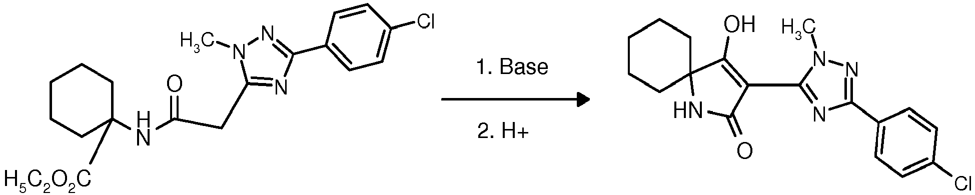

Verwendet man beispielsweise gemäß Verfahren (B) Ethyl-2-{2-[3-(4-chlorphenyl)-1-methyl-1H-1,2,4-triazol-5-yl]acetoxy}-2-methylpropanoat, so kann der Verlauf des erfindungsgemäßen Verfahrens durch folgendes Reaktionsschema wiedergegeben werden:

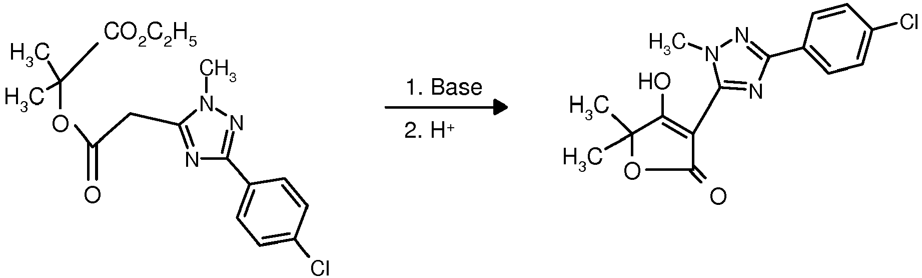

Verwendet man beispielsweise gemäß Verfahren (F) Ethyl-2-{[3-(4-chlorphenyl)-1-methyl-1H-1,2,4-triazol-5-yl]acetyl}cyclopentancarboxylat, so kann der Verlauf des erfindungsgemäßen Verfahrens durch folgendes Reaktionsschema wiedergegeben werden:

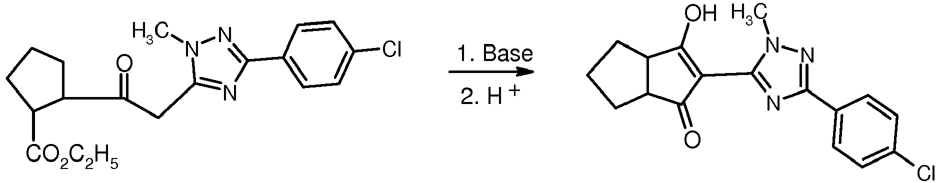

Verwendet man beispielsweise gemäß Verfahren (Hα) Hexahydropyridazin und 2-[3-(4-Chlorphenyl)-1-methyl-1H-1,2,4-triazol-5-yl]-3-oxoacryloylchlorid als Ausgangsverbindungen, so kann der Reaktionsverlauf des erfindungsgemäßen Verfahrens durch folgendes Reaktionsschema wiedergegeben werden:

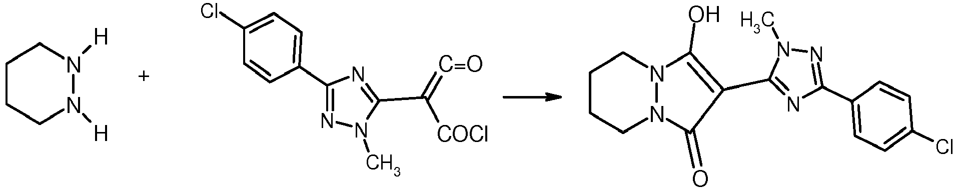

Verwendet man beispielsweise gemäß Verfahren (Hβ) Hexahydropyridazin und Dimethyl-[3-(4-chlorphenyl)-1-methyl-1H-1,2,4-triazol-5-yl]malonat als Ausgangsprodukte, so kann der Verlauf des erfindungsgemäßen Verfahrens durch folgendes Reaktionsschema wiedergegeben werden:

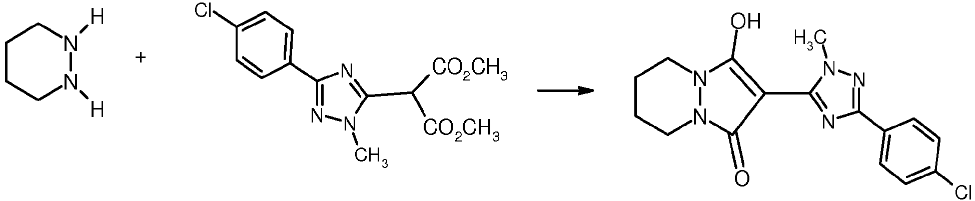

Verwendet man beispielsweise gemäß Verfahren (Hγ) Ethyl-2-{[3-(4-chlorphenyl)-1-methyl-1H-1,2,4-triazol-5-yl]acetyl}tetrahydropyridazin-1(2H)-carboxylat als Ausgangsprodukt, so kann der Reaktionsverlauf durch folgendes Schema wiedergegeben werden:

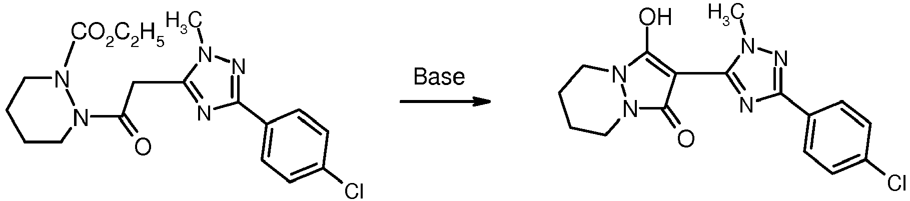

Verwendet man beispielsweise gemäß Verfahren (Lα) 3-[3-(4-Chlorphenyl)-1-methyl-1H-1,2,4-triazol-5-yl]-4-hydroxy-5,5-dimethyl-1,5-dihydro-2H-pyrrol-2-on und Pivaloylchlorid als Ausgangsstoffe, so kann der Verlauf des erfindungsgemäßen Verfahrens durch folgendes Reaktionsschema wiedergegeben werden:

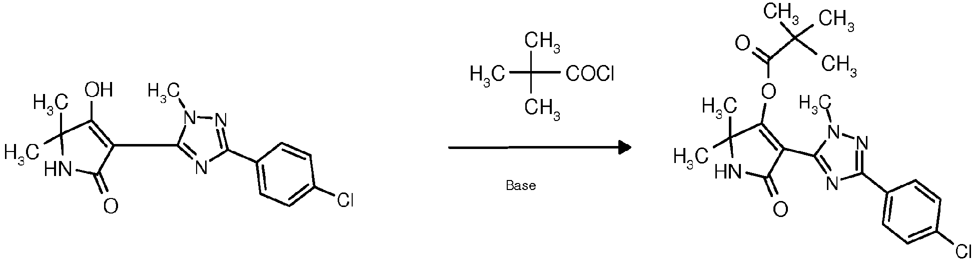

Verwendet man beispielsweise gemäß Verfahren (Lβ) α) 3-[3-(4-Chlorphenyl)-1-methyl-1H-1,2,4-triazol-5-yl]-4-hydroxy-5,5-dimethyl-1,5-dihydro-2H-pyrrol-2-on und Acetanhydrid als Ausgangsverbindungen, so kann der Verlauf des erfindungsgemäßen Verfahrens durch folgendes Reaktionsschema wiedergegeben werden:

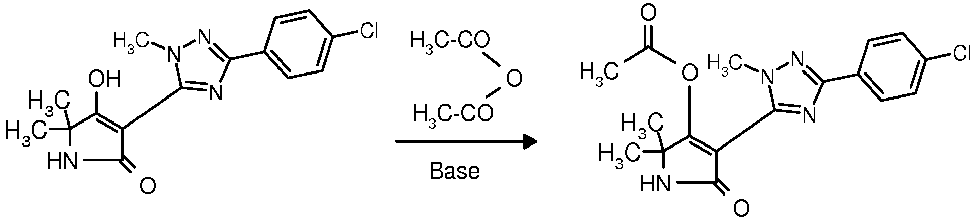

Verwendet man beispielsweise gemäß Verfahren (M) 2-[3-(4-Chlorphenyl)-1-methyl-1H-1,2,4-triazol-5-yl]-1-hydroxy-6,7,8,8a-tetrahydroindolizin-3(5H)-on und Chlorameisensäureethylester als Ausgangsverbindungen, so kann der Verlauf des erfindungsgemäßen Verfahrens durch folgendes Reaktionsschema wiedergegeben werden:

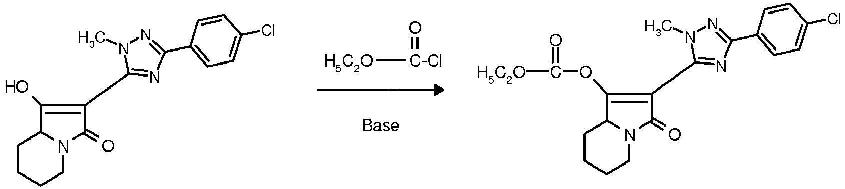

Verwendet man beispielsweise gemäß Verfahren (R) Variante α 3-[3-(4-Chlorphenyl)-1-methyl-1H-1,2,4-triazol-5-yl]-4-hydroxy-1-oxaspiro[4.4]non-3-en-2-on und Ethylisocyanat als Ausgangsprodukte, so kann der Reaktionsverlauf durch folgendes Reaktionsschema wiedergegeben werden:

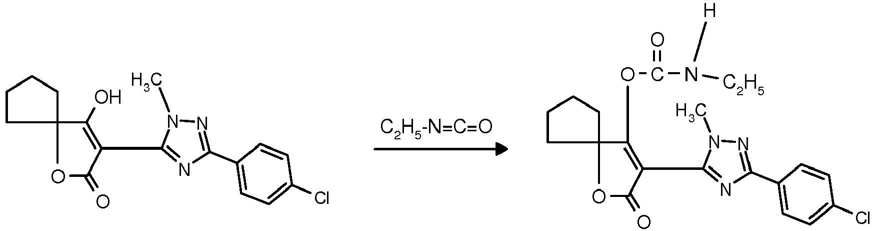

Verwendet man beispielsweise gemäß Verfahren (R) Variante ß 3-[3-(4-Chlorphenyl)-1-methyl-1H-1,2,4-triazol-5-yl]-4-hydroxy-5-methyl-1,5-dihydro-2H-pyrrol-2-on und Dimethylcarbamidsäurechlorid als Ausgangsprodukte, so kann der Reaktionsverlauf durch folgendes Schema wiedergegeben werden:

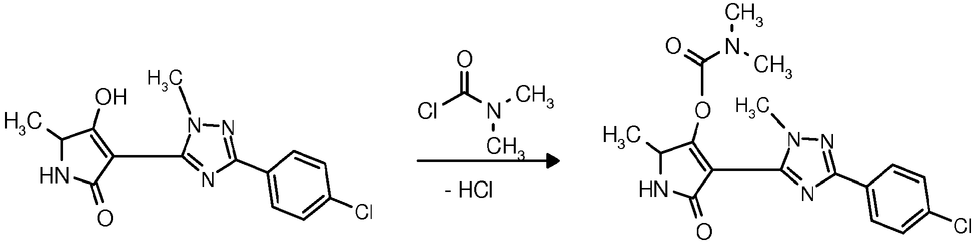

Die beim erfindungsgemäßen Verfahren (A) als Ausgangsstoffe benötigten Verbindungen der Formel (II) 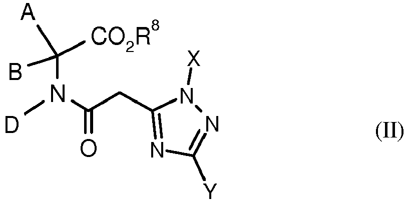 in welcher
A, B, D, X, Y und R⁸ die oben angegebenen Bedeutungen haben,
sind neu.
Man erhält die Acylaminosäureester der Formel (II) beispielsweise, wenn man Aminosäurederivate der Formel (XXVII) 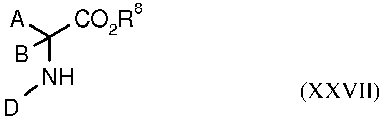 in welcher
A, B, R⁸ und D die oben angegebenen Bedeutungen haben,
mit substituierten 1,2,4-Triazolylessigsäurederivaten der Formel (XXVIII) 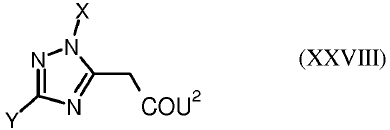 in welcher
- X und Y: die oben angegebenen Bedeutungen haben und
- U²: für eine durch Carbonsäureaktivierungsreagenzien wie Carbonyldiimidazol, Carbonyldiimide (wie z.B. Dicyclohexylcarbondiimid), Propane-phosphonicsäureanhydrid (T3P), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XXIX) 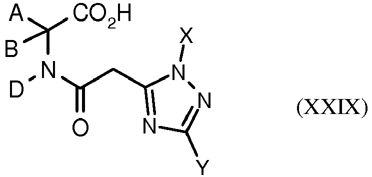 in welcher
A, B, D, X und Y die oben angegebenen Bedeutungen haben,
verestert (Chem. Ind. (London) 1568 (1968)).

Die Verbindungen der Formel (XXIX) 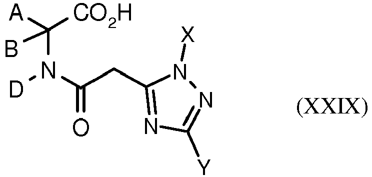 in welcher
A, B, D, X und Y die oben angegebenen Bedeutungen haben,
sind neu.

Man erhält die Verbindungen der Formel (XXIX), wenn man Aminosäuren der Formel (XXX) 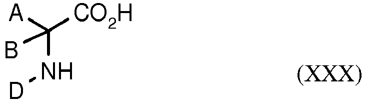 in welcher
- A, B und D: die oben angegebenen Bedeutungen haben,
mit substituierten Triazolylessigsäurederivaten der Formel (XXVIII) 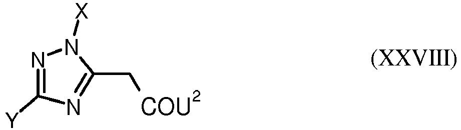 in welcher
- X und Y: die oben angegebenen Bedeutungen haben und
- U²: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXVIII) sind neu. Sie lassen sich nach im Prinzip bekannten Verfahren darstellen (s. z.B. H. Henecka, Houben-Weyl, Methoden der Organischen Chemie, Bd. 8, S. 467-469 (1952)) WO 97/02243, WO 99/43699 oder werden mit den oben angegebenen Reagenzien in situ erzeugt.

Man erhält die Verbindungen der Formel (XXVIII) beispielsweise, indem man substituierte Triazolylessigsäuren der Formel (XXXI) 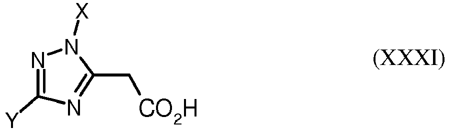 in welcher
- X und Y: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B.

POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXVII) und (XXX) sind teilweise aus der eingangs zitierten Patentliteratur bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXX), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als ß bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 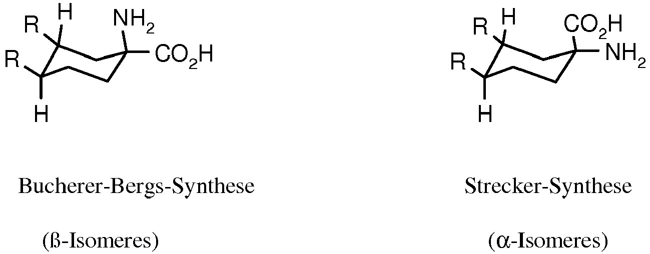 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 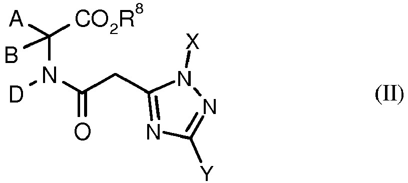 in welcher
A, B, D, X, Y und R⁸ die oben angegebenen Bedeutungen haben,
herstellen, wenn man Aminonitrile der Formel (XXXII) 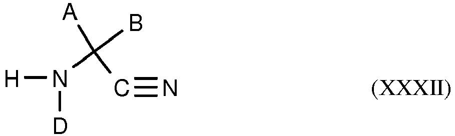 in welcher
A, B und D die oben angegebenen Bedeutungen haben,
mit substituierten Triazolylessigsäurederivaten der Formel (XXVIII) 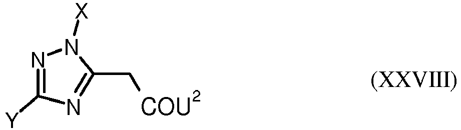 in welcher
X, Y und U² die oben angegebenen Bedeutungen haben,
zu Verbindungen der Formel (XXXIII) 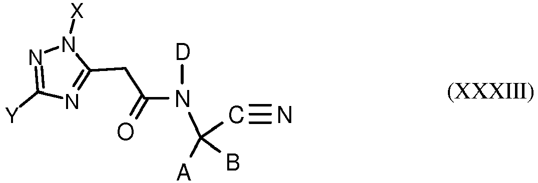 in welcher
A, B, D, X und Y die oben angegebenen Bedeutungen haben,
umsetzt,
und diese anschließend einer sauren Alkoholyse unterwirft.

Die Verbindungen der Formel (XXXIII) sind ebenfalls neu.

Die bei dem erfindungsgemäßen Verfahren (B) als Ausgangstoffe benötigten Verbindungen der Formel (III) 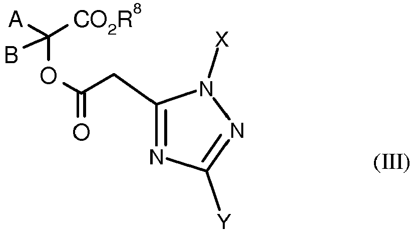 in welcher
A, B, X, Y und R⁸ die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXXIV-A) 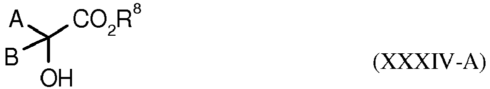 in welcher
A, B und R⁸ die oben angegebenen Bedeutungen haben,
mit substituierten Triazolylessigsäurederivaten der Formel (XXVIII) 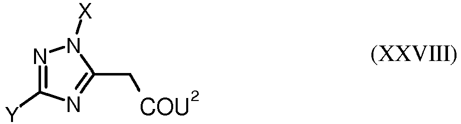 in welcher
X, Y und U² die oben angegebenen Bedeutungen haben,
acyliert (Chem. Reviews 52, 237-416 (1953)).

Weiterhin erhält man Verbindungen der Formel (III), wenn man
substituierte Triazolylessigsäuren der Formel (XXXI) 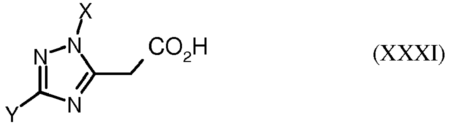 in welcher
X und Y die oben angegebenen Bedeutungen haben,
mit α-Halogencarbonsäureestern der Formel (XXXIV-B) 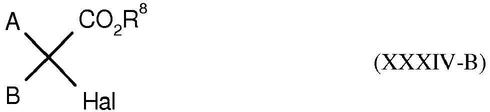 in welcher
A, B und R⁸ die oben angegebenen Bedeutungen haben und
Hal für Chlor oder Brom steht,
alkyliert.

Die Verbindungen der Formel (XXXIV-A) sind teilweise käuflich oder aus den eingangs erwähnten Offenbarungen bekannt.

Die Verbindungen der Formel (XXXIV-B) sind käuflich.

Die Verbindungen der Formel (XXXI) sind neu.

Beispielsweise erhält man die Verbindungen der Formel (XXXI), 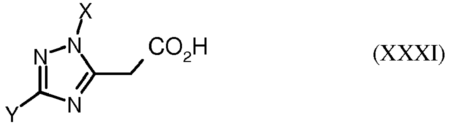 in welcher
X und Y die oben angegebenen Bedeutungen haben,
wenn man Triazolylessigsäureester der Formel (XXXV) 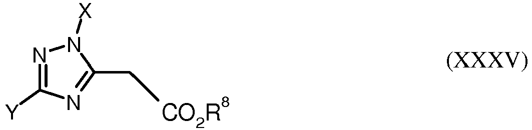 in welcher
X, Y und R⁸ die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXXV) sind mit Ausnahme der Verbindung XXXV-1 neu.

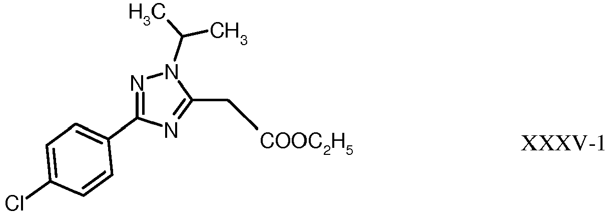

Die Verbindungen der Formel (XXXV), in welcher X Y und R⁸ die oben angegebene Bedeutung haben, erhält man, wenn man Triazolylessigsäureester der Formel (XXXV-A) 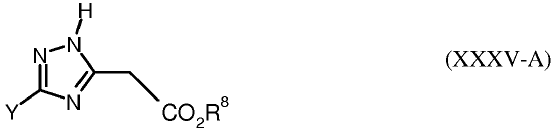 in welcher R⁸ und Y die oben angegebene Bedeutung haben
in Gegenwart von einem Alkylierungsmittel (z. B. Alkylhalogenid) in Gegenwart einer Base und gegebenenfalls in einem geeigneten Lösungsmittel, beispielsweise wie in Plant Physiology 144, 1303, Supplement Materials and Methods S 1 beschrieben, umsetzt.

Die Verbindungen der Formel (XXXV-A) sind mit Ausnahme der Verbindung XXXV-A1 (siehe Plant Physiology 144, 1303, Supplement Materials and Methods S1) neu.

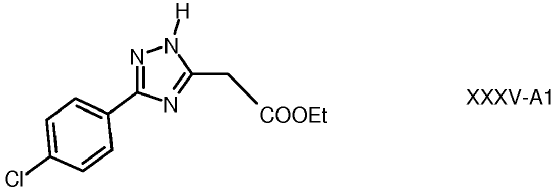

Die Herstellung der Triazolylessigsäureester der Formel (XXXV-A) sind prinzipiell, beispielsweise aus Synthesis 1999, 483 - 486 und Plant Physiology 144, 1303, Supplement Materials and Methods S1 bekannt und lassen sich nach den dort beschriebenen Verfahren herstellen.

Die bei den obigen Verfahren (H-α) als Ausgangsstoffe benötigten Halogencarbonylketene der Formel (VI) sind neu. Sie lassen sich nach im Prinzip bekannten Methoden herstellen (vgl. beispielsweise Org. Prep. Proced. Int., 7, (4), 155-158, 1975 und DE 1 945 703). So erhält man z.B. die Verbindungen der Formel (VI) 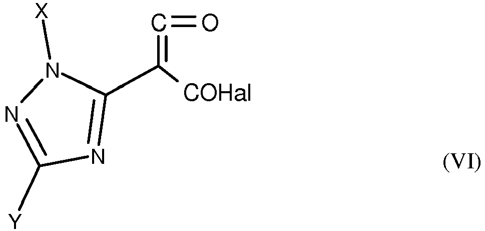 in welcher
X und Y die oben angegebenen Bedeutungen haben und
Hal für Chlor oder Brom steht,
wenn man
substituierte Triazolylmalonsäuren der Formel (XXXVII) 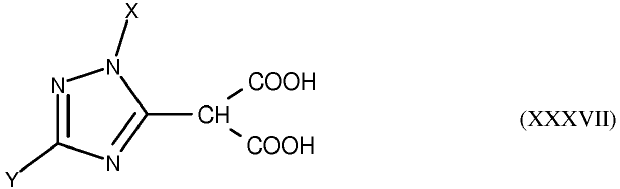 in welcher
X und Y die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Dimethylformamid, Methyl-Stearylformamid oder Triphenylphosphin und gegebenenfalls in Gegenwart von Basen wie z.B. Pyridin oder Triethylamin, umsetzt.

Die substituierten Phenylmalonsäuren der Formel (XXXVII) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Triazolylmalonsäuren der Formel (XXXVII) 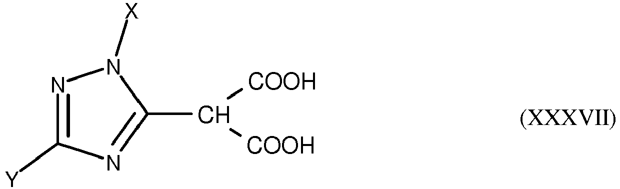 in welcher
X und Y die oben angegebenen Bedeutungen haben,
wenn man Triazolylmalonsäureester der Formel (XI) 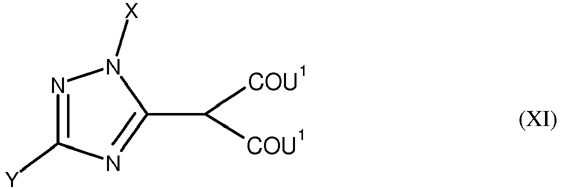 in welcher
X und Y die oben angegebene Bedeutung haben,
und U¹ für OR⁸ steht,
wobei R⁸ die oben angegebene Bedeutung hat,
zunächst in Gegenwart einer Base und einem Lösungsmittel verseift und anschließend vorsichtig ansäuert (s. beispielsweise EP-A-528 156, WO 96/35 664, WO 97/02 243).

Die Malonsäureester der Formel (XI) 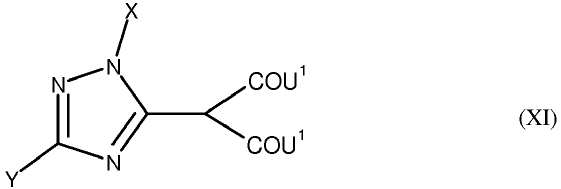 in welcher
X und Y die oben angegebene Bedeutung haben,
und U¹ für OR⁸ steht,
wobei R⁸ die oben angegebene Bedeutung hat
sind neu.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die bei dem obigen Verfahren (F) als Ausgangsstoffe benötigten Verbindungen der Formel (VIII) 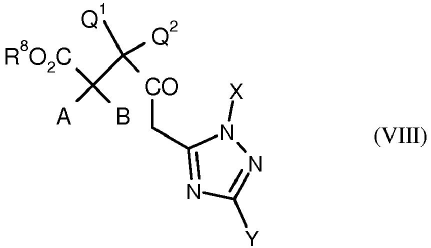 in welcher
A, B, Q¹, Q², X, Y und R⁸ die oben angegebene Bedeutung haben,
sind neu.

Sie lassen sich nach im Prinzip bekannten Methoden herstellen.

Man erhält die 5-Triazolyl-4-ketocarbonsäureester der Formel (VIII) beispielsweise, wenn man 5-Aryl-4-ketocarbonsäuren der Formel (XXXVIII) 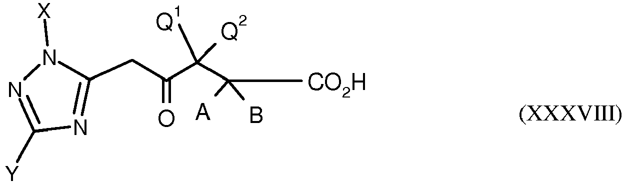 in welcher
X, Y, A, B, Q¹ und Q² die oben angegebene Bedeutung haben,
verestert (vgl. z.B. Organikum, 15. Auflage, Berlin, 1977, Seite 499) oder alkyliert (siehe Herstellungsbeispiel).

Die 5-Triazolyl-4-ketocarbonsäuren der Formel (XXXVIII) 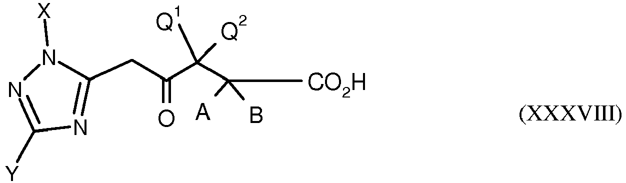 in welcher
A, B, Q¹, Q², X und Y die oben angegebene Bedeutung haben,
sind neu, lassen sich aber nach im Prinzip bekannten Methoden herstellen (WO 96/01 798, WO 97/14667, WO 98/39281).

Man erhält die 5-Triazolyl-4-ketocarbonsäuren der Formel (XXXVIII) beispielsweise, wenn man 2-Triazolyl-3-oxo-adipinsäureester der Formel (XXXIX) 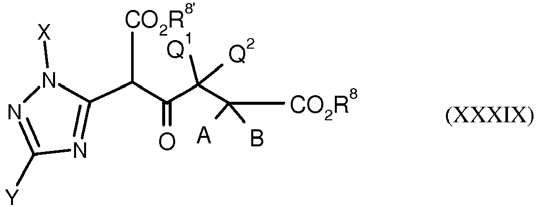 in welcher
A, B, Q¹, Q², X und Y die oben angegebene Bedeutung haben und
R⁸ und R^{8'} für Alkyl (insbesondere C₁-C₈-Alkyl) stehen und
bei Einsatz der Verbindung der Formel (XLI-a) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521, WO 96/01798, WO 97/14667, WO 98/39281).

Die Verbindungen der Formel (XXXIX) 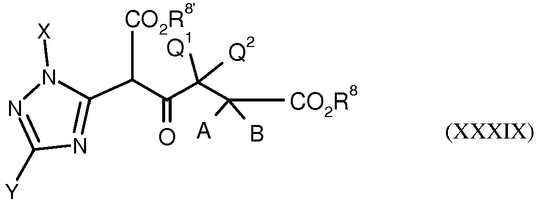 in welcher
A, B, Q¹, Q², X, Y, R⁸ und R^{8'} die oben angegebene Bedeutung haben und
bei Einsatz der Verbindung der Formel (XLI-a) R⁸ für Wasserstoff steht
sind neu.

Man erhält die Verbindungen der Formel (XXXIX) beispielsweise,
wenn man Dicarbonsäurehalbesterchloride der Formel (XL), 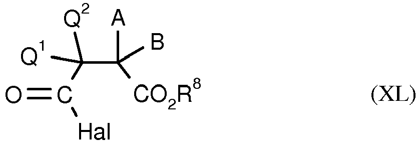 in welcher
A, B, Q¹, Q² und R⁸ die oben angegebene Bedeutung haben und
Hal für Chlor oder Brom steht,
oder Carbonsäureanhydride der Formel (XLI-a) 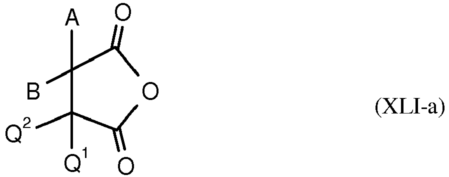 in welcher
A, B, Q¹ und Q² die oben angegebene Bedeutung haben,
mit einem Triazolylessigsäureester der Formel (XXXV) 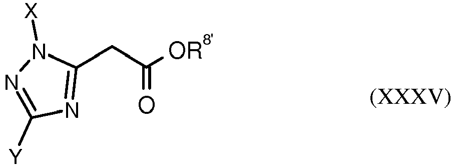 in welcher
X, Y und R^{8'} die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XL) und (XLI-a) sind teilweise bekannte Verbindungen der Organischen Chemie und/oder lassen sich nach im Prinzip bekannten Methoden in einfacher Weise herstellen.

Die für das erfindungsgemäße Verfahren (H-α) und (H-ß) als Ausgangsstoffe benötigten Hydrazine der Formel (X)

A-NH-NH-D (X)

in welcher
A und D die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-A-508 126, WO 92/16510, WO 99/47 525, WO 01/17 972).

Die für das erfindungsgemäße Verfahren (H-γ) benötigten Verbindungen der Formel (XII) 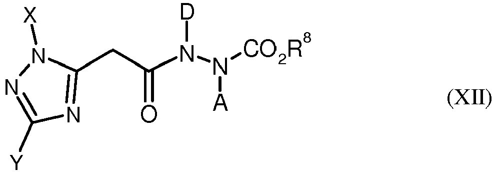 in welcher
A, D, X, Y und R⁸ die oben angegebene Bedeutung haben,
sind neu.

Man erhält die Acylcarbazate der Formel (XII) beispielsweise, wenn man Carbazate der Formel (XLV) 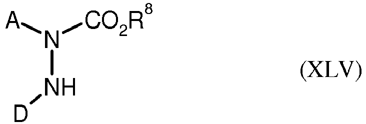 in welcher
A, R⁸ und D die oben angegebenen Bedeutungen haben,
mit substituierten Triazolylessigsäurederivaten der Formel (XXVIII) 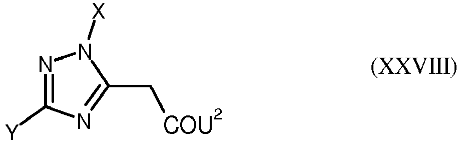 in welcher
X, Y und U² die oben angegebenen Bedeutungen haben
acyliert (Chem. Reviews 52, 237-416 (1953); Bhattacharya, Indian J. Chem. 6, 341-5, 1968).

Die Carbazate der Formel (XLV) sind teilweise käufliche und teilweise bekannte Verbindungen oder lassen sich nach im Prinzip bekannten Verfahren der organischen Chemie herstellen.

Die Verbindungen der Formel (XXVIII) wurden bereits bei den Vorstufen für das Verfahren (A) und (B) beschrieben.

Die zur Durchführung der erfindungsgemäßen Verfahren (L), (M) und (R) außerdem als Ausgangsstoffe benötigten Säurehalogenide der Formel (XVI), Carbonsäureanhydride der Formel (XVII), Chlorameisensäureester der Formel (XVIII), und Isocyanate der Formel (XXIV) und Carbamidsäurechloride der Formel (XXV) sind allgemein bekannte Verbindungen der Organischen bzw. Anorganischen Chemie.

Die Verbindungen der Formeln (X), (XXVII), (XXX), (XXXII), (XXXIV-A), (XXXIV-B), (XL), (XLI-a), (XLV), sind darüber hinaus aus den eingangs zitierten Patentanmeldungen bekannt Das Verfahren (A) ist dadurch gekennzeichnet, dass man Verbindungen der Formel (II), in welcher A, B, D, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, X, Y und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (F) ist dadurch gekennzeichnet, dass man Verbindungen der Formel (VIII), in welcher A, B, Q¹, Q², X, Y und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Collidin, Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (F) innerhalb eines größeren Bereiches variiert werden. Im Allgemeinen arbeitet man bei Temperaturen zwischen -75°C und 250°C, vorzugsweise zwischen -50°C und 150°C.

Das erfindungsgemäße Verfahren (F) wird im Allgemeinen unter Normaldruck durchgeführt.

Bei der Durchführung des erfindungsgemäßen Verfahrens (F) setzt man die Reaktionskomponenten der Formel (VIII) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das erfindungsgemäße Verfahren (H-α) ist dadurch gekennzeichnet, dass man Hydrazine der Formel (X) oder Salze dieser Verbindungen mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (H-α) alle inerten organischen Solventien eingesetzt werden. Vorzugsweise verwendbar sind gegebenenfalls chlorierte Kohlenwasserstoffe, wie beispielsweise Mesitylen, Chlorbenzol und Dichlorbenzol, Toluol, Xylol, ferner Ether, wie Dibutylether, Glykoldimethylether, Diglykoldimethylether und Diphenylethan, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (H-α) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethylanilin.

Die Reaktionstemperaturen können bei der Durchführung der erfindungsgemäßen Verfahrensvariante (H-α) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (H-α) wird zweckmäßigerweise unter Normaldruck durchgeführt.

Bei der Durchführung des erfindungsgemäßen Verfahrens (H-α) setzt man die Reaktionskomponenten der Formeln (VI) und (X), in welchen A, D, X und Y die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (H-ß) ist dadurch gekennzeichnet, dass man Hydrazine der Formel (X) oder Salze dieser Verbindung, in welcher A und D die oben angegebenen Bedeutungen haben, mit Malonestern oder Malonsäureamiden der Formel (XI), in welcher U¹, X, Y und R⁸ die oben angegebene Bedeutung haben, in Gegenwart einer Base einer Kondensation unterwirft.

Als Verdünnungsmittel können bei dem erfindungsgemäßen Verfahren (H-ß) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Tetrahydrofuran, Dioxan, Diphenylether, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-β) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)anunoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Verwendbar sind auch tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (H-ß) innerhalb eines größeren Bereiches variiert werden. Im Allgemeinen arbeitet man bei Temperaturen zwischen 0°C und 280°C, vorzugsweise zwischen 50°C und 180°C.

Das erfindungsgemäße Verfahren (H-ß) wird im Allgemeinen unter Normaldruck durchgeführt.

Bei der Durchführung des erfindungsgemäßen Verfahrens (H-ß) setzt man die Reaktionskomponenten der Formeln (XI) und (X) im Allgemeinen in etwa äquimolaren Mengen ein. Es ist jedoch auch möglich, die eine oder andere Komponente in einem größeren Überschuss (bis zu 3 mol) zu verwenden.

Das Verfahren (H-γ) ist dadurch gekennzeichnet, dass man Verbindungen der Formel (XII), in welcher A, D, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (H-γ) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-γ) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (H-γ) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (H-γ) wird im Allgemeinen unter Normaldruck durchgeführt.

Bei der Durchführung des erfindungsgemäßen Verfahrens (H-γ) setzt man die Reaktionskomponenten der Formel (XII) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (L-α) ist dadurch gekennzeichnet, dass man Verbindungen der Formeln (I-1-a) bis (1-11-a) jeweils mit Carbonsäurehalogeniden der Formel (XVI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (L-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (L-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (L-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (L-α) werden die Ausgangsstoffe der Formeln (I-1-a) bis (I-11-a) und das Carbonsäurehalogenid der Formel (XVI) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (L-ß) ist dadurch gekennzeichnet, dass man Verbindungen der Formeln (I-1-a) bis (1-11-a) mit Carbonsäureanhydriden der Formel (XVII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (L-ß) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (L-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (L-ß) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (L-ß) werden die Ausgangsstoffe der Formeln (I-1-a) bis (I-11-a) und das Carbonsäureanhydrid der Formel (XVII) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (M) ist dadurch gekennzeichnet, dass man Verbindungen der Formeln (I-1-a) bis (I-11-a) jeweils mit Chlorameisensäureestern oder Chlorameisensäurethiolestern der Formel (XVIII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (M) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (M) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (M) innerhalb eines größeren Bereiches variiert werden. Arbeitet man in Gegenwart eines Verdünnungsmittels und eines Säurebindemittels, so liegen die Reaktionstemperaturen im Allgemeinen zwischen - 20°C und +100°C, vorzugsweise zwischen 0°C und 50°C.

Das erfindungsgemäße Verfahren (M) wird im Allgemeinen unter Normaldruck durchgeführt.

Bei der Durchführung des erfindungsgemäßen Verfahrens (M) werden die Ausgangsstoffe der Formeln (I-1-a) bis (I-11-a) und der entsprechende Chlorameisensäureester bzw. Chlorameisensäurethiolester der Formel (XVIII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (R) ist dadurch gekennzeichnet, dass man Verbindungen der Formeln (I-1-a) bis (I-11-a) jeweils mit (R-α) Verbindungen der Formel (XXIV) gegebenenfalls in Gegenwart eines Verdünnungsmittels und gegebenenfalls in Gegenwart eines Katalysators oder (R-β) mit Verbindungen der Formel (XXV) gegebenenfalls in Gegenwart eines Verdünnungsmittels und gegebenenfalls in Gegenwart eines Säurebindemittels umsetzt.

Bei Herstellungsverfahren (R-α) setzt man pro Mol Ausgangsverbindung der Formeln (I-1-a) bis (1-11-a) ca. 1 Mol Isocyanat der Formel (XXIV) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (R-β) setzt man pro Mol Ausgangsverbindung der Formeln (I-1-a) bis (I-11-a) ca. 1 Mol Carbamidsäurechlorid der Formel (XXV) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-11-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., Epitrimerus pyri, Eutetranychus spp., Eriophyes spp., Glycyphagus domesticus, Halotydeus destructor, Hemitarsonemus spp., Hyalomma spp., Ixodes spp., Latrodectus spp., Loxosceles spp., Metatetranychus spp., Neutrombicula autumnalis, Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., Tetranychus spp., Trombicula alfreddugesi, Vaejovis spp., Vasates lycopersici.;
aus der Klasse der Chilopoda z.B. Geophilus spp., Scutigera spp.;
aus der Ordnung oder der Klasse der Collembola z.B. Onychiurus armatus.;
aus der Klasse der Diplopoda z.B. Blaniulus guttulatus;
aus der Klasse der Insecta, z.B. aus der Ordnung der Blattodea z.B. Blattella asahinai, Blattella germanica, Blatta orientalis, Leucophaea maderae, Panchlora spp., Parcoblatta spp., Periplaneta spp., Supella longipalpa;
aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptolestes ferrugineus, Cryptorhynchus lapathi, Cylindrocopturus spp., Dermestes spp., Diabrotica spp., Dichocrocis spp., Dicladispa armigera, Diloboderus spp., Epilachna spp., Epitrix spp., Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., Lachnosterna consanguinea, Lasioderma serricorne, Latheticus oryzae, Lathridius spp., Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Necrobia spp., Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllophaga helleri, Phyllotreta spp., Popillia japonica, Premnotrypes spp., Prostephanus truncatus, Psylliodes spp., Ptinus spp., Rhizobius ventralis, Rhizopertha dominica, Sitophilus spp., Sitophilus oryzae, Sphenophorus spp., Stegobium paniceum, Sternechus spp., Symphyletes spp., Tanymecus spp., Tenebrio molitor, Tenebrioides mauretanicus, Tribolium spp., Trogoderma spp., Tychius spp., Xylotrechus spp., Zabrus spp.;
aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Calliphora vicina, Ceratitis capitata, Chironomus spp., Chrysomyia spp., Chrysops spp., Chrysozona pluvialis, Cochliomyia spp., Contarinia spp., Cordylobia anthropophaga, Cricotopus sylvestris, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., Lucilia spp., Lutzomyia spp., Mansonia spp., Musca spp., Oestrus spp., Oscinella frit, Paratanytarsus spp., Paralauterborniella subcincta, Pegomyia spp., Phlebotomus spp., Phorbia spp., Phormia spp., Piophila casei, Prodiplosis spp., Psila rosae, Rhagoletis spp., Sarcophaga spp., Simulium spp., Stomoxys spp., Tabanus spp., Tetanops spp., Tipula spp.;
aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptocorisa varicornis, Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Homoptera z.B. Acizzia acaciaebaileyanae, Acizzia dodonaeae, Acizzia uncatoides, Acrida turrita, Acyrthosipon spp., Acrogonia spp., Aeneolamia spp., Agonoscena spp., Aleyrodes proletella, Aleurolobus barodensis, Aleurothrixus floccosus, Allocaridara malayensis, Amrasca spp., Anuraphis cardui, Aonidiella spp., Aphanostigma piri, Aphis spp., Arboridia apicalis, Arytainilla spp., Aspidiella spp., Aspidiotus spp., Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., Cryptomyzus ribis, Cryptoneossa spp., Ctenarytaina spp., Dalbulus spp., Dialeurodes citri, Diaphorina citri, Diaspis spp., Drosicha spp., Dysaphis spp., Dysmicoccus spp., Empoasca spp., Eriosoma spp., Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Macrosteles facifrons, Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nettigoniclla spectra, Nilaparvata lugens, Oncometopia spp., Orthezia praelonga, Oxya chinensis, Pachypsylla spp., Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Prosopidopsylla flava, Protopulvinaria pyriformis, Pseudaulacaspis pentagona, Pseudococcus spp., Psyllopsis spp., Psylla spp., Pteromalus spp., Pyrilla spp., Quadraspidiotus spp., Quesada gigas, Rastrococcus spp., Rhopalosiphum spp., Saissetia spp., Scaphoideus titanus, Schizaphis graminum, Selenaspidus articulatus, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., Trialeurodes vaporariorum, Trioza spp., Typhlocyba spp., Unaspis spp., Viteus vitifolii, Zygina spp.;
aus der Ordnung der Hymenoptera z.B. Acromyrmex spp., Athalia spp., Atta spp., Diprion spp., Hoplocampa spp., Lasius spp., Monomorium pharaonis, Sirex spp., Solenopsis invicta, Tapinoma spp., Urocerus spp., Vespa spp., Xeris spp.;
aus der Ordnung der Isopoda z.B. Armadillidium vulgare, Oniscus asellus, Porcellio scaber;
aus der Ordnung der Isoptera z.B. Coptotermes spp., Cornitermes cumulans, Cryptotermes spp., Incisitermes spp., Microtermes obesi, Odontotermes spp., Reticulitermes spp.;
aus der Ordnung der Lepidoptera z.B. Achroia grisella, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamstra brassicae, Melanitis leda, Mocis spp., Monopis obviella, Mythimna separata, Nemapogon cloacellus, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudaletia unipuncta, Pseudoplusia includens, Pyrausta nubilalis, Rachiplusia nu, Schoenobius spp., Scirpophaga spp., Scirpophaga innotata, Scotia segetum, Sesamia spp., Sesamia inferens, Sparganothis spp., Spodoptera spp., Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., Tryporyza incertulas, Tuta absoluta, Virachola spp.;
aus der Ordnung der Orthoptera oder Saltatoria z.B. Acheta domesticus, Dichroplus spp., Gryllotalpa spp., Hieroglyphus spp., Locusta spp., Melanoplus spp., Schistocerca gregaria;
aus der Ordnung der Phthiraptera z.B. Damalinia spp., Haematopinus spp., Linognathus spp., Pediculus spp., Phylloera vastatrix, Phtirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., Pulex irritans, Tunga penetrans, Xenopsylla cheopsis;
aus der Ordnung der Thysanoptera z.B. Anaphothrips obscurus, Baliothrips biformis, Drepanothrips reuteri, Enneothrips flavens, Frankliniella spp., Heliothrips spp., Hercinothrips femoralis, Rhipiphorothrips cruentatus, Scirtothrips spp., Taeniothrips cardamomi, Thrips spp.;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp.;
Schädlinge aus dem Stamm der Mollusca, insbesondere aus der Klasse der Bivalvia, z.B. Dreissena spp., sowie aus der Klasse der Gastropoda z.B. Arion spp., Biomphalaria spp., Bulinus spp., Deroceras spp., Galba spp., Lymnaea spp., Oncomelania spp., Pomacea spp., Succinea spp.;
Tierparasiten aus den Stämmen der Plathelminthes und Nematoda, z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;
Pflanzenschädlinge aus dem Stamm der Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus spp., Trichodorus spp., Tylenchulus spp., Xiphinema spp., Helicotylenchus spp., Tylenchorhynchus spp., Scutellonema spp., Paratrichodorus spp., Meloinema spp., Paraphelenchus spp., Aglenchus spp., Belonolaimus spp.,
Nacobbus spp., Rotylenchulus spp., Rotylenchus spp., Neotylenchus spp., Paraphelenchus spp., Dolichodorus spp., Hoplolaimus spp., Punctodera spp., Criconemella spp., Quinisulcius spp., Hemicycliophora spp., Anguina spp., Subanguina spp., Hemicriconemoides spp., Psilenchus spp., Pseudohalenchus spp., Criconemoides spp., Cacopaurus spp.

Weiterhin lässt sich aus dem Unterreich der Protozoa die Ordnung der Coccidia z.B. Eimeria spp. bekämpfen.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP-POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten, Nematoden oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen. Die Wirkstoffe können auch in transgenen Pflanzen, die sich durch höhere Erträge , z. B. durch eine verbesserte Photosyntheseleistung oder verbesserte Nährstoffaufnahme auszeichnen, eingesetzt werden.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant). Des weiteren wurden transgene Pflanzen beschrieben, die gegen synthetische Auxine (z. B 2,4 D) HRAC mode of action Class O und Aryloxy-phenoxy Propionate (fops, HRAC, Class A) resistent sind (DHT, Dow Agroscience Herbicide Tolerance Trait)
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- Gentechnisch veränderte Pflanzen, die neue Insektenresistenzen, z. B. basierend auf der Expression von Toxinen aus Photorhabdus, Xenorhabdus Symbionten aus entomopathogenen Nematoden und Toxinen aus Spinnen, Skorpionen, Ameisen, parasitischen Wespen aufweisen.
- gentechnisch veränderte Kulturpflanzen mit neuen Inhalts- oder Sekundärstoffen z. B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- Transgene Kulturpflanzen, die sich durch erhöhte Toleranzen gegen abiotische und biotische Stressoren auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetyl CoA Carboxylasen, Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der FOPs, Sulfonylharnstoffe, der Glyphosate, Glufosinate oder Benzoylisoxazole und analogen Wirkstoffe, oder gegen beliebige Kombinationen dieser Wirkstoffe, resistent sind.
- Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden. Des weiteren und besonders bevorzugt können die erfindungsgemäßen Verbindungen in transgenen Pflanzen, die gegen synthetische Auxine (z. B 2,4 D) mit "HRAC mode of action Class O" und Aryloxy-phenoxy Propionate (fops) mit "HRAC mode of action Class A" resistent sind (z. B. DHT, Dow Agroscience Herbicide Tolerance Trait) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere herbizidresistente Pflanzen sind Pflanzen, die gegenüber ACCase-Hemmern tolerant gemacht worden sind.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die Bezeichnung "Wirkstoffe" bzw. "Verbindungen" schließt immer auch die hier genannten Wirkstoffkombinationen mit ein.

### Herstellungsbeispiele:

### Beispiel I-1-a1-1= (I-1-a-1):

Verfahren A

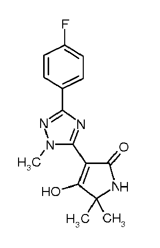

Zu einer Lösung von 2,1 mmol (236 mg) Kalium-tert.-butylat in 1 ml wasserfreiem DMF werden bei Raumtemperatur 0,5 mmol (167 mg) Methyl N-{[3-(4-fluorophenyl)-1-methyl-1H-1,2,4-triazol-5-yl]acetyl}-2-methylalaninat (Verbindung gemäß Beispiel II-1-a-1) in 0,5 ml wasserfreiem DMF zugetropft und 2 h bei Raumtemperatur gerührt. Der Ansatz wird mit 2,1 mmol (239 mg) Trifluoressigsäure in 0,5 ml Acetonitril versetzt. Das Rohprodukt wird über HPLC gereinigt. Ausbeute: 114mg (76 % der Theorie).
¹H-NMR (400 MHz, CDCl₃): δ = 1.4 (s, 6H), 4.5 (s, 3H), 7.2 (m, 2H), 7.9 (m, 2H).

### Beispiel I-1-a4-4 = (I-1-a-43):

Verfahren A

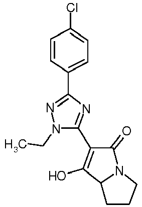

Zu einer Lösung von 1,6 mmol (180 mg) Kalium-tert.-butylat in 1 ml wasserfreiem DMF werden bei Raumtemperatur 0,5 mmol (188 mg) Methyl 1-{[3-(4-chlorophenyl)-1-ethyl-1H-1,2,4-triazol-5-yl]acetyl}prolinat in wasserfreiem 0,5 ml wasserfreiem DMF zugetropft und 2 h bei Raumtemperatur gerührt. Die Reaktionsmischung wird mit 182 mg (1,6 mmol) Trifluoressigsäure in 0,5 ml Acetonitril versetzt. Anschließend wird der Ansatz über HPLC gereinigt. Ausbeute: 139 mg (81 % der Theorie).
¹H-NMR (400 MHz, CDCl₃): δ = 1.5 (t, 3H), 2.2 (m, 3H), 3.2 (M, 1H), 3.7 (M, 1H), 4.2 (M, 1H), 5.0 (m, 2H), 7.5 (m, 2H), 7.8 (m, 2H).

In Analogie zu Beispiel I-1-a1-1 und Beispiel I-1-a4-4 sowie gemäß den allgemeinen Angaben zur Herstellung erhält man folgende Verbindungen der Formel (I-1-a):

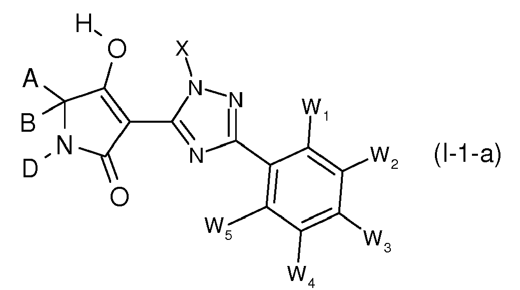

| Bsp-Nr. | X | W₁ | W₂ | W₃ | W₄ | W₅ | D | A | B | Analytik | Isomer |
|---|---|---|---|---|---|---|---|---|---|---|---|
| I-1-a1-2 (I-1-a-2) | C₂H₅ | H | H | F | H | H | H | CH₃ | CH₃ | 1H-NMR (400 MHz, CDCl₃: δ = 1.4 (s, 6H), 1.5 (t, 3H), 5.0 (q, 2H), 7.2 (m, 2H), 7.9 (m, 2H) | |
| I-1-a1-3 (I-1-a-3) | CH₃ | H | H | Cl | H | H | H | CH₃ | CH₃ | 1H-NMR (400 MHz, CDCl₃: δ = 1.4 (s, 6H), 4.5 (s, 3H), 7.5 (d, 2H), 7.8 (d, 2H) | |
| I-1-a1-4 (I-1-a-4) | C₂H₅ | H | H | Cl | H | H | H | CH₃ | CH₃ | 1H-NMR (400 MHz, CDCl₃: δ = 1,.4 (s, 6H), 1.5 (t, 3H), 5.0 (q, 2H), 7.5 (d, 2H), 7.8 (d, 2H) | |
| I-1-a1-5 (I-1-a-5) | CH₃ | H | H | H | H | H | H | CH₃ | CH₃ | 1H-NMR (400 MHz, CDCl₃: δ = 1.4 (s, 6H), 4.5 (s, 3H), 7.5 (m, 3H), 7.9 (d, 2H) | |
| I-1-a1-6 (I-1-a-6) | C₂H₅ | H | H | H | H | H | H | CH₃ | CH₃ | 1H-NMR (400 MHz, CDCl₃: δ = 1,.4 (s, 6H), 1.5 (t, 3H), 5.0 (q, 2H), 7.5 (t, 2H), 7.9 (d, 2H) | |
| I-1-a1-7 (I-1-a-7) | i-Pr | H | H | H | H | H | H | CH₃ | CH₃ | 1H-NMR (400 MHz, CDCl₃: δ = 1,.4 (s, 6H), 1.6 (d, 6H), 6.4 (m, 1H), 7.5 (m, 3H), 7.9 (d, 2H) | |
| I-1-a1-8 (I-1-a-8) | i-Pr | H | H | F | H | H | H | CH₃ | CH₃ | 1H-NMR (400 MHz, CDCl₃: δ = 1,.4 (s, 6H), 1.6 (d, 6H), 6.4 (m, 1H), 7.2 (t, 2H), 7.9 (m, 2H) | |
| I-1-a1-9 (I-1-a-9) | i-Pr | H | H | Cl | H | H | H | CH₃ | CH₃ | 1H-NMR (400 MHz, CDCl₃: δ = 1,.4 (s, 6H), 1.6 (d, 6H), 6.4 (m, 1H), 7.5 (d, 2H), 7.8 (d, 2H) | |
| I-1-a1-10 (I-1-a-10) | n-Pr | H | H | H | H | H | H | CH₃ | CH₃ | 1H-NMR (400 MHz, CDCl₃: δ = 1.0 (t, 3H), 1.4 (s, 6H), 2.0 (m, 2H), 5.0 (t, 2H), 7.5 (m, 3H), 7.8 (d, 2H) | |
| I-1-a1-12 (I-1-a-11) | n-Pr | H | H | Cl | H | H | H | CH₃ | CH₃ | 1H-NMR (400 MHz, CDCl₃: δ = 1.0 (t, 3H), 1.4 (s, 6H), 2.0 (m, 2H), 5.0 (t, 2H), 7.5 (d, 2H), 7.8 (d, 2H) | |
| I-1-a1-16 (I-1-a-12) | Me | Cl | H | Cl | H | H | H | CH₃ | CH₃ | 1H-NMR (400 MHz, CDCl₃: δ = 1.40 (s, 6H), 4.52 (s, 3H), 5.32 (s, br, 1H), 7.38-7.41 (m, 1H), 7.56 (s, 1H), 7.98-7.8.00 (m, 1H) | |
| I-1-a2-1(I-1-a-13) | CH₃ | H | H | F | H | H | H | -(CH₂)₂-CH(OCH₃)-(CH₂)₂- | | 1H-NMR (400 MHz, CDCl₃): δ = 1.4 (m,2H), 1.7 (m, 2H), 1.9 (m, 2H), 2.2 (m, 2H), 3.3 (m, 1H), 3.4 (s, 3H), 4.5 (s, 3H), 7.2 (t, 2H), 7.9 (m, 2H) | cis |
| I-1-a2-2 (I-1-a-14) | C₂H₅ | H | H | F | H | H | H | -(CH₂)₂-CH(OCH₃)-(CH₂)₂- | | 1H-NMR (400 MHz, DMSO-d₆): δ = 1.4 (t, 3H), 1.5 (m, 4H), 1.9 (m, 2H), 2.0 (m, 2H), 3.2 (m, 1H), 3.3 (s, 3H), 4.5 (q, 2H), 7.3 (t, 2H), 8.0 (m, 2H) | cis |
| I-1-a2-3 (I-1-a-15) | CH₃ | H | H | Cl | H | H | H | -(CH₂)₂-CH(OCH₃)-(CH₂)₂- | | 1H-NMR (400 MHz, CDCl₃): δ = 1.4 (m, 2H), 1.7 (m, 2H), 1.9 (m, 2H), 2.2 (m, 2H), 3.3 (m, 1H), 3.4 (s, 3H), 4.5 (s, 3H), 7.2 (t, 2H), 7.9 (m, 2H) | cis |
| I-1-a2-4 (I-1-a-16) | C₂H₅ | H | H | Cl | H | H | H | -(CH₂)₂-CH(OCH₃)-(CH₂)₂- | | 1H-NMR (400 MHz, CDCl₃): δ = 1.4 (m, 2H), 1.5 (t, 3H), 1.7 (m, 2H), 1.9 (m, 2H), 2.2 (m,2H), 3.3 (m, 1H), 3.4 (s, 3H), 5.0 (q, 2H), 7.5 (m, 3H), 7.8 (m, 2H) | cis |
| I-1-a2-5 (I-1-a-17) | CH₃ | H | H | H | H | H | H | -(CH₂)₂-CH(OCH₃)-(CH₂)₂- | | 1H-NMR (400 MHz, CDCl₃): δ = 1.4 (m, 2H), 1.7 (m, 2H), 1.9 (m, 2H), 2.2 (m, 2H), 3.3 (m, 1H), 3.4 (s, 3H), 4.5 (s, 3H), 7.5 (m, 3H), 7.9 (m, 2H) | cis |
| I-1-a2-6 (I-1-a-18) | C₂H₅ | H | H | H | H | H | H | -(CH₂)₂-CH(OCH₃)-(CH₂)₂- | | 1H-NMR (400 MHz, CDCl₃): δ = 1.4 (m, 2H), 1.5 (t, 3H), 1.7 (m, 2H), 1.9 (m, 2H), 2.2 (m, 2H), 3.3 (m, 1H), 3.4 (s, 3H), 5.0 (q, 2H), 7.5 (m, 3H), 7.8 (m, 2H) | cis |
| I-1-a2-7 (I-1-a-19) | i-Pr | H | H | H | H | H | H | -(CH₂)₂-CH(OCH₃)-(CH₂)₂- | | 1H-NMR (400 MHz, CDCl₃): δ = 1.4 (m, 2H), 1.5 (d, 6H), 1.7 (m, 2H), 1.9 (m, 2H), 2.2 (m, 2H), 3.3 (m, 1H), 3.4 (s, 3H), 6.5 (m, 1H), 7.5 (m, 3H), 7.8 (m, 2H) | cis |
| I-1-a2-8 (I-1-a-20) | i-Pr | H | H | F | H | H | H | -(CH₂)₂-CH(OCH₃)-(CH₂)₂- | | 1H-NMR (400 MHz, CDCl₃): δ = 1.4 (m, 2H), 1.5 (d, 6H), 1.7 (m, 2H), 1.9 (m, 2H), 2.2 (m, 2H), 3.3 (m, 1H), 3.4 (s, 3H), 6.4 (m, 1H), 7.2 (t, 2H), 7.9 (m, 2H) | cis |
| I-1-a2-9 (I-1-a-21) | i-Pr | H | H | Cl | H | H | H | -(CH₂)₂-CH(OCH₃)-(CH₂)₂- | | 1H-NMR (400 MHz, CDCl₃): δ = 1.4 (m, 2H), 1.5 (d, 6H), 1.7 (m, 2H), 1.9 (m, 2H), 2.2 (m, 2H), 3.3 (m, 1H), 3.4 (s, 3H), 6.4 (m, 1H), 7.5 (d, 2H), 7.9 (d, 2H) | cis |
| I-1-a2-10 (I-1-a-22) | n-Pr | H | H | H | H | H | H | -(CH₂)₂-CH(OCH₃)-(CH₂)₂- | | 1H-NMR (400 MHz, CDCl₃): δ = 1.0 (t, 3H), 1.4 (m, 2H), 1.7 (m, 2H), 1.9 (m, 4H), 2.2 (m, 2H), 3.3 (m, 1H), 3.4 (s, 3H), 5.0 (t, 2H), 7.5 (m, 3H), 7.9 (d, 2H) | CiS |
| I-1-a2-11 (I-1-a-23) | n-Pr | H | H | F | H | H | H | -(CH₂)₂-CH(OCH₃)-(CH₂)₂- | | 1H-NMR (400 MHz, CDCl₃): δ = 1.0 (t, 3H), 1.4 (m, 2H), 1.7 (m, 2H), 1.9 (m, 4H), 2.2 (m, 2H), 3.3 (m, 1H), 3.4 (s, 3H),5.0 (t, 2H), 7.2 (t, 2H), 7.9 (m, 2H) | cis |
| I-1-a2-12 (I-1-a-24) | n-Pr | H | H | Cl | H | H | H | -(CH₂)₂-CH(OCH₃)-(CH₂)₂- | | 1H-NMR (400 MHz, CDCl₃): δ = 1.0 (t, 3H), 1.4 (m, 2H), 1.7 (m, 2H), 1.9 (m, 4H), 2.2 (m, 2H), 3.3 (m, 1H), 3.4 (s, 3H), 5.0 (t, 2H), 7.5 (d, 2H), 7.8 (d, 2H) | cis |
| I-1-a2-13 (I-1-a-25) | CH₂CF₃ | H | H | F | H | H | H | -(CH₂)₂-CH(OCH₃)-(CH₂)₂- | | 1H-NMR (400 MHz, CDCl₃): δ = 1.5 (m, 2H), 1.7 (m, 2H), 1.9 (m, 2H), 2.2 (m, 2H), 2.2 (m, 2H), 3.3 (m, 1H), 3.4 (s, 3H), 5.9 (q, 2H), 7.2 (t, 2H), 8.0 (m, 2H) | cis |
| I-1-a2-14 (I-1-a-26) | n-Bu | H | H | F | H | H | H | -(CH₂)₂-CH(OCH₃)-(CH₂)₂- | | 1H-NMR (400 MHz, CDCl₃): δ = 1.0 (t, 3H), 1.4 (m, 4H), 1.7 (m, 2H), 1.9 (m, 4H), 2.2 (m, 2H), 3.3 (m, 1H), 3.4 (s, 3H), 5.0 (t, 2H), 7.2 (t, 2H), 7.9 (m, 2H) | cis |
| I-1-a2-15 (I-1-a-27) | CH₃ | H | H | Cl | H | H | H | -(CH₂)₂-CH(OCH₃)-(CH₂)₂- | | 1H-NMR (400 MHz, CDCl₃): δ = 1.4 (m, 2H), 1.7 (m, 2H), 1.9 (m, 2H), 2.2 (m, 2H), 3.3 (m, 1H), 3.3 (s, 3H), 3.4 (s, 3H), 3.9 (t, 2H), ), 5.2 (t, 2H), 7.5 (d, 2H), 7.8 (d, 2H) | cis |
| I-1-a2-16 (I-1-a-28) | CH₃ | H | H | Cl | H | H | H | -(CH₂)₂-CH(OCH₃)-(CH₂)₂- | | 1H-NMR (400 MHz, CDCl₃): δ = 1.46-1.49 (m, 2H), 1.84-1.87 (m, 2H), 1.95-1.98 (m, 2H), 2.20-2.24 (m, 2H), 3.18- 3.28 (m, 1H), 3.32 (s, 3H), 3.67 (s, 3H), 3.88 (s, 2H), 3.97 (s, 3H), 7.32-7.35 (m, 1H), 7.52 (s, 1H), 7.81-7.83 (m, 1H), 8.47 (s, br, 1H) | cis |
| I-1-a3-1 (I-1-a-29) | CH₃ | H | H | F | H | H | H | -(CH₂)₂-O-(CH₂)₂- | | 1H-NMR (400 MHz, CDCl₃): δ = 1.5 (d, 2H), 2.2 (m, 2H), 3.6 (m, 2H), 4.2 (m, 2H), 4.5 (s, 3H), 7.2 (t, 2H), 7.9 (m, 2H) | |
| I-1-a3-2 (I-1-a-30) | C₂H₅ | H | H | F | H | H | H | -(CH₂)₂-O-(CH₂)₂- | | 1H-NMR (400 MHz, d₆-DMSO): δ = 1.3 (d, 2H), 1.4 (t, 3H), 2.0 (m, 2H), 3.7 (m, 2H), 3.9 (m, 2H), 4.5(s, 3H), 7.2 (t, 2H), 7.9 (m, 2H) | |
| I-1-a3-3 (I-1-a-31) | CH₃ | H | H | Cl | H | H | H | -(CH₂)₂-O-(CH₂)₂- | | 1H-NMR (400 MHz, CDCl₃): δ = 1.5 (m, 2H), 2.2 (m, 2H), 3.6 (m, 2H), 4.2 (m, 2H), 4.5 (s, 3H), 7.5 (d, 2H), 7.8 (d, 2H) | |
| I-1-a3-4 (I-1-a-32) | C₂H₅ | H | H | Cl | H | H | H | -(CH₂)₂-O-(CH₂)₂- | | 1H-NMR (400 MHz, d₆-DMSO): δ = 1.3 (d, 2H), 1.4 (t, 3H), 2.0 (m, 2H), 3.7 (m, 2H), 3.9 (m, 2H), 4,4 (q, 2H), 7.6 (d, 2H), 8.0 (m, 2H) | |
| I-1-a3-5 (I-1-a-33) | CH₃ | H | H | H | H | H | H | -(CH₂)₂-O-(CH₂)₂- | | 1H-NMR (400 MHz, CDCl₃): δ = 1.5 (d, 2H), 2.2 (m, 2H), 3.6 (m, 2H), 4.2 (m, 2H), 4.5 (s, 3H), 7.5 (m, 3H), 7.9 (m, 2H) | |
| I-1-a3-6 (I-1-a-34) | C₂H₅ | H | H | H | H | H | H | -(CH₂)₂-O-(CH₂)₂- | | 1H-NMR (400 MHz, CDCl₃): δ = 1.5 (m, 5H), 2.2 (m, 2H), 3.6 (m, 2H), 4.2 (m, 2H), 5.1 (q, 2H), 7.5 (m, 3H), 7.9 (m, 2H) | |
| I-1-a3-7 (I-1-a-35) | i-Pr | H | H | H | H | H | H | -(CH₂)₂-O-(CH₂)₂- | | 1H-NMR (400 MHz, CDCl₃): δ = 1.5 (m, 5H), 2.2 (m, 2H), 3.6 (m, 2H), 4.1 (m, 2H), 6.5 (m, 1H), 7.5 (m, 3H), 7.9 (m, 2H) | |
| I-1-a3-8 (I-1-a-36) | i-Pr | H | H | F | H | H | H | -(CH₂)₂-O-(CH₂)₂- | | 1H-NMR (400 MHz, CDCl₃): δ = 1.5 (m, 5H), 2.2 (m, 2H), 3.6 (m, 2H), 4.1 (m, 2H), 6.5 (m, 1H), 7.2 (t, 2H), 7.9 (m, 2H) | |
| I-1-a3-9 (I-1-a-37) | i-Pr | H | H | Cl | H | H | H | -(CH₂)₂-O-(CH₂)₂- | | 1H-NMR (400 MHz, CDCl₃): δ = 1.5 (m, 5H), 2.2 (m, 2H), 3.6 (m, 2H), 4.1 (m, 2H), 6.4 (m, 1H), 7.5 (d, 2H), 7.9 (d, 2H) | |
| I-1-a3-10 (I-1-a-38) | n-Pr | H | H | H | H | H | H | -(CH₂)₂-O-(CH₂)₂- | | 1H-NMR (400 MHz, CDCl₃): δ = 1.0 (t, 3H), 1.5 (m, 2H), 2.0 (m, 2H), 2.2 (m, 2H), 3.6 (m, 2H), 4.1 (m, 2H), 5.0 (t, 2H), 7.5 (m, 3H), 7.9 (d, 2H) | |
| I-1-a3-11 (I-1-a-39) | n-Pr | H | H | F | H | H | H | -(CH₂)₂-O-(CH₂)₂- | | 1H-NMR (400 MHz, CDCl₃): δ = 1.0 (t, 3H), 1.5 (m, 2H), 2.0 (m, 2H), 2.2 (m, 2H), 3.6 (m, 2H), 4.1 (m, 2H), 5.0 (t, 2H), 7.2 (t, 2H), 7.9 (m, 2H) | |
| I-1-a4-1 (I-1-a-40) | CH₃ | H | H | F | H | H | -(CH₂)₃- | | H | 1H-NMR (400 MHz, CDCl₃): δ = 1.6 (m, 1H), 2.1 (m, 2H), 2.2 (m, 1H), 3.2 (m, 1H), 3.6 (m, 1H), 4.1 (m, 1H), 4.5 (s, 3H), 7.2 (t, 2H), 7.9 (m, 2H) | |
| I-1-a4-2 (I-1-a-41) | C₂H₅ | H | H | F | H | H | -(CH₂)₃- | | H | 1H-NMR (400 MHz, CDCl₃): δ = 1.5 (t, 3H), 1.6 (m, 1H), 2,.1 (m, 2H), 2.2 (m, 1H), 3.2 (m, 1H), 3.7 (m, 1H), 4.1 (m, 1H), 5.0 (m, 2H), 7.2 (t, 2H), 7.9 (m, 2H) | |
| I-1-a4-3 (I-1-a-42) | CH₃ | H | H | Cl | H | H | -(CH₂)₃- | | H | 1H-NMR (400 MHz, CDCl₃): δ = 1.6 (m, 1H), 2.1 (m, 2H), 2.2 (m, 1H), 3.3 (m, 1H), 3.6 (m, 1H), 4.2 (m, 1H), 4.4 (s, 3H), 7.5 (d, 2H), 7.8 (d, 2H) | |
| I-1-a4-4 (I-1-a-43) | C₂H₅ | H | H | Cl | H | H | -(CH₂)₃- | | H | 1H-NMR (400 MHz, CDCl₃): δ = 1.5 (t, 3H), 1.6 (m, 1H), 2.1 (m, 2H), 2.2 (m, 1H), 3.3 (m, 1H), 3.6 (m, 1H), 4.2 (m, 1H), 5.0 (m, 2H), 7.5 (d, 2H), 7.8 (d, 2H) | |
| I-1-a4-6 (I-1-a-44) | C₂H₅ | H | H | H | H | H | -(CH₂)₃- | | H | 1H-NMR (400 MHz, CDCl₃): δ = 1.5 (t, 3H), 2.1 (m, 2H), 2.2 (m, 1H), 3.3 (m, 1H), 3.6 (m, 1H), 4.1 (m, 1H), 5.0 (m, 2H), 7.5 (m, 3H), 7.8 (d, 2H) | |
| I-1-a4-7(I-1-a-45) | i-Pr | H | H | H | H | H | -(CH₂)₃- | | H | 1H-NMR (400 MHz, CDCl₃): δ = 1.5 (d,3H), 1.6 (d, 3H), 2.1 (m, 2H), 2.2 (m, 1H), 3.3 (m, 1H), 3.6 (m, 1H), 4.1 (m, 1H), 6.3 (m, 1H), 7.5 (m, 3H), 7.8 (d, 2H) | |
| I-1-a4-8 (I-1-a-46) | i-Pr | H | H | F | H | H | -(CH₂)₃- | | H | 1H-NMR (400 MHz, CDCl₃): δ = 1.5 (d, 3H), 1.6 (d, 3H), 2.1 (m, 2H), 2.2 (m, 1H), 3.3 (m, 1H), 3.6 (m, 1H), 4.1 (m, 1H), 6.3 (m, 1H), 7.2 (t, 2H), 7.9 (m, 2H) | |
| I-1-a4-9 (I-1-a-47) | i-Pr | H | H | Cl | H | H | -(CH₂)₃- | | H | 1H-NMR (400 MHz, CDCl₃): δ = 1.5 (d, 3H), 1.6 (d, 3H), 2.1 (m, 2H), 2.2 (m, 1H), 3.3 (m, 1H), 3.6 (m, 1H), 4.1 (m, 1H), 6.3 (m, 1H), 7.5 (d, 2H), 7.8 (d, 2H) | |
| I-1-a4-10 (I-1-a-48) | n-Pr | H | H | H | H | H | -(CH₂)₃- | | H | 1H-NMR (400 MHz, CDCl₃): δ = 1.0 (t, 3H), 1.6 (m, 1H), 2.0 (m, 2H), 2.1 (m, 2H), 2.2 (m, 1H), 3.3 (m, 1H), 3.6 (m, 1H), 4.1 (m, 1H), 4.9 (m, 1H), 5.0 (m, 1H), 7.5 (m, 3H), 7.8 (d, 2H) | |
| I-1-a4-11 (I-1-a-49) | n-Pr | H | H | F | H | H | -(CH₂)₃- | | H | 1H-NMR (400 MHz, CDCl₃): δ = 1.0 (t, 3H), 1.6 (m, 1H), 2.0 (m, 2H), 2.1 (m, 2H), 2.2 (m, 1H), 3.3 (m, 1H), 3.6 (m, 1H), 4.1 (m, 1H), 4.9 (m, 1H), 5.0 (m, 1H), 7.2 (t, 2H), 7.9 (m, 2H) | |
| I-1-a5-1 (I-1-α-50) | CH₃ | H | H | F | H | H | c-Pr | H | H | 1H-NMR (400 MHz, CDCl₃): δ = 0.8 (m, 4H), 2.7 (m, 1H), 3.8 (s, 2H), 4.5 (s, 3H), 7.2 (m, 2H), 7.9 (m, 2H) | |
| I-1-a5-2 (I-1-α-51) | C₂H₅ | H | H | F | H | H | c-Pr | H | H | 1H-NMR (400 MHz, CDCl₃): δ = 0.8 (m, 4H), 1.5 (t, 3H), 2.7 (m, 1H), 3.8 (s, 2H), 5.0 (q, 2H), 7.2 (m, 2H), 7.9 (m, 2H) | |
| I-1-a5-3 (I-1-a-52) | CH₃ | H | H | Cl | H | H | c-Pr | H | H | 1H-NMR (400 MHz, CDCl₃): δ = 0.8 (m, 4H), 2.7 (m, 1H), 3.8 (s, 2H), 4.5 (s, 3H), 7.2 (m, 2H), 7.9 (m, 2H) | |
| I-1-a5-4 (I-1-a-53) | C₂H₅ | H | H | Cl | H | H | c-Pr | H | H | 1H-NMR (400 MHz, CDCl₃): δ = 0.8 (m, 4H), 1.5 (t, 3H), 2.7 (m, 1H), 3.8 (s, 2H), 5.0 (q, 2H), 7.5 (d, 2H), 7.8 (d, 2H) | |
| I-1-a5-5 (I-1-a-54) | CH₃ | H | H | H | H | H | c-Pr | H | H | 1H-NMR (400 MHz, CDCl₃): δ = 0.8 (m, 4H), 2.7 (m, 1H), 3.8 (s, 2H), 4.5 (s, 3H), 7.5 (m, 3H), 7.9 (m, 2H) | |
| I-1-a5-6 (I-1-a-55) | C₂H₅ | H | H | H | H | H | c-Pr | H | H | 1H-NMR (400 MHz, CDCl₃): δ = 0.8 (m, 4H), 1.5 (t, 3H), 2.7 (m, 1H), 3.8 (s, 2H), 5.0 (q, 2H), 7.5 (m, 3H), 7.9 (m, 2H) | |
| I-1-a5-7 (I-1-a-56) | i-Pr | H | H | H | H | H | c-Pr | H | H | 1H-NMR (400 MHz, CDCl₃): δ = 0.8 (m, 4H), 1.5 (d, 6H), 2.7 (m, 1H), 3.7 (s, 2H), 6.5 (m, 1H), 7.5 (m, 3H), 7.9 (m, 2H) | |
| I-1-a5-8 (I-1-a-57) | i-Pr | H | H | F | H | H | c-Pr | H | H | 1H-NMR (400 MHz, CDCl₃): δ = 0.8 (m, 4H), 1.5 (d, 6H), 2.7 (m, 1H), 3.7 (s, 2H), 6.5 (m, 1H), 7.2 (t, 2H), 7.9 (d, 2H) | |
| I-1-a5-9 (I-1-a-58) | i-Pr | H | H | Cl | H | H | c-Pr | H | H | 1H-NMR (400 MHz, CDCl₃): δ = 0.8 (m, 4H), 1.5 (d, 6H), 2.7 (m, 1H), 3.7 (s, 2H), 6.4 (m, 1H), 7.5 (d, 2H), 7.9 (d, 2H) | |
| I-1-a5-10 (I-1-a-59) | n-Pr | H | H | H | H | H | c-Pr | H | H | 1H-NMR (400 MHz, CDCl₃): δ = 0.8 (m, 4H), 1.0 (t, 3H), 2.0 (m, 2H), 2.7 (m, 1H), 3.7 (s, 2H), 5.0 (t, 2H), 7.5 (d, 2H), 7.9 (d, 2H) | |
| I-1-a5-11 (I-1-a-60) | n-Pr | H | H | F | H | H | c-Pr | H | H | 1H-NMR (400 MHz, CDCl₃): δ = 0.8 (m, 4H), 1.0 (t, 3H), 2.0 (m, 2H), 2.7 (m, 1H), 3.7 (s, 2H), 5.0 (t, 2H), 7.2 (t, 2H), 7.9 (m, 2H) | |
| I-1-a6-3 (I-1-a-61) | CH₃ | H | H | Cl | H | H | H | -(CH₂)₂-CH(On-Pr)-(CH₂)₂- | | 1H-NMR (400 MHz, CDCl₃): δ = 0.9 (t, 3H), 1.4 (m, 2H), 1.7 (m, 4H), 1.9 (m, 2H), 2.2 (m, 2H), 3.3 (m, 1H), 3.5 (t, 2H), 4.5 (s, 3H), 7.5 (d, 2H), 7.8 (d, 2H) | cis |
| I-1-a7-2 (I-1-a-62) | C₂H₅ | H | H | F | H | H | H | -(CH₂)₂-CH(Me)-(CH₂)₂- | | 1H-NMR (400 MHz, CDCl₃): δ = 1.0 (d, 3H), 1.1 (m, 2H), 1.5 (t, 3H), 1.6 (m, 2H), 1.9 (m, 4H), 5.0 (q, 2H), 7.2 (t, 2H), 7.9 (m, 2H) | cis |
| I-1-a7-3 (I-1-a-63) | CH₃ | H | H | Cl | H | H | H | -(CH₂)₂-CH(Me)-(CH₂)₂- | | 1H-NMR (400 MHz, CDCl₃): δ = 1.0 (d, 3H), 1.1 (m, 2H), 1.6 (m, 3H), 1.9 (m, 4H), 4.5 (s, 3H), 7.5 (d, 2H), 7.8 (d, 2H) | cis |
| I-1-a7-7 (I-1-a-64) | i-Pr | H | H | H | H | H | H | -(CH₂)₂-CH(Me)-(CH₂)₂- | | 1H-NMR (400 MHz, CDCl₃): δ = 1.0 (d, 3H), 1.1 (m, 2H), 1.6 (m, 3H), 1.9 (m, 4H), 6.4 (m, 1H), 7.5 (m, 3H), 7.9 (m, 2H) | |
| I-1-a7-11 (I-1-a-65) | n-Pr | H | H | F | H | H | H | -(CH₂)₂-CH(Me)-(CH₂)₂- | | 1H-NMR (400 MHz, CDCl₃): δ = 1.0 (m, 6H), 1.5 (m, 1H), 1.6 (m, 2H), 1.9 (m, 6H), 5.0 (t, 2H), 7.2 (t, 2H), 7.9 (m, 2H) | |
| I-1-a8-3 (I-1-a-66) | CH₃ | H | H | Cl | H | H | H | -(CH₂)₂-CH(CF₃)-(CH₂)₂- | | 1H-NMR (400 MHz, CDCl₃): δ = 1.6 (m, 2H), 1.7 (m, 2H), 1.9 (m, 2H), 2.1 (m, 2H), 4.5 (s, 3H), 7.5 (d, 2H), 7.8 (d, 2H) | |
| I-1-a9-3 (I-1-a-67) | CH₃ | H | H | Cl | H | H | H | -(CH₂)₂-N(OEt)-(CH₂)₂- | | 1H-NMR (400 MHz, CDCl₃): δ = 1.2 (t, 3H), 1.6 (m, 2H), 2.2 (m, 2H), 2.5 (m, 2H), 3.4 (m, 2H), 3.8 (q, 2H), 4.5 (s, 3H), 7.5 (d, 2H), 7.8 (d, 2H) | |
| I-1-a10-3 (I-1-a-68) | CH₃ | H | H | Cl | H | H | H | -(CH₂)₂-N(OMe)-(CH₂)₂- | | 1H-NMR (400 MHz, CDCl₃): δ = 1.8 (m, 2H), 2.2 (m, 2H), 4.5 (s, 3H), 7.5 (d, 2H), 7.8 (d, 2H) | |
| I-1-a11-3 (I-1-a-69) | CH₃ | H | H | Cl | H | H | H | -(CH₂)₂-C(O-CH₂CHCH₂)-(CH₂)₂- | | 1H-NMR (400 MHz, CDCl₃): δ = 3.9 (m, 2H), 4.5 (s, 3H), 7.5 (d, 2H), 7.8 (d, 2H) | |
| I-1-a12-3 (I-1-a-70) | CH₃ | H | H | Cl | H | H | H | -(CH₂)₂-C(Et)(OMe)-(CH₂)₂- | | 1H-NMR (400 MHz, CDCl₃): δ = 0.9 (t, 3H), 1.5 (q, 2H), 2.0 (m, 2H), 2.2 (m, 2H), 3.2 (s, 3H), 4.5 (s, 3H), 7.5 (d, 2H), 7.8 (d, 2H) | |
| I-1-a13-3 (I-1-a-71) | CH₃ | H | H | Cl | H | H | H | -(CH₂)₂-C(OCH₂CHMeCH₂O)-(CH₂)₂- | | 1H-NMR (400 MHz, CDCl₃): δ = 0.8 (d, 3H), 1.6 (m, 4H), 1.7 (m, 2H), 2.2 (m, 4H), 3.5 (t, 2H), 3.6 (t, 2H), 3.8 (t, 2H), 4.5 (s, 3H), 7.5 (d, 2H), 7.8 (d, 2H) | |
| I-1-a14-3 (I-1-a-72) | CH₃ | H | H | Cl | H | H | H | c-Pr | c-Pr | 1H-NMR (400 MHz, CDCl₃): δ = 0.3 (m, 5H), 0.6 (m, 2H), 1.3 (m, 3H), 4.5 (s, 3H), 7.5 (d, 2H), 7.8 (d, 2H) | |
| I-1-a14-11 (I-1-a-73) | n-Pr | H | H | F | H | H | H | c-Pr | c-Pr | 1H-NMR (400 MHz, CDCl₃): δ = 0.3 (m, 5H), 0.6 (m, 2H), 1.0 (t, 3H), 1.3 (m, 2H), 2.0 (m, 2H), 4.9 (m, 2H), 7.2 (t, 2H), 7.9 (m, 2H) | |

| | | | | | | | | | | | |
|---|---|---|---|---|---|---|---|---|---|---|---|
| c-Pr = cyclo-Propyl; i-Pr = iso-Propyl; n-Pr = n-Propyl; n-Bu = n-Butyl, Pr = Propyl, Me = Methyl, Et = Ethyl | | | | | | | | | | | |

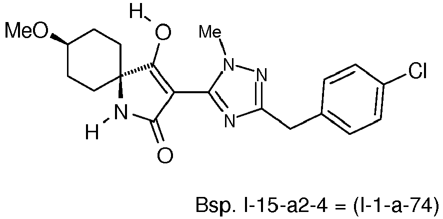

1H-NMR (CDCl₃): δ = 1.3 (m, 2H), 1.6 (m, 2H),), 1.8 (m, 2H), 2.2 (m, 2H), 3.2 (m, 1H), 3.4 (s, 3H), 4.0 (s, 2H), 4.4 (s, 3H), 7.2 (d, 2H), 7.4 (d, 2H)

### Beispiel I-1-b2-4 = (I-1-g-1)

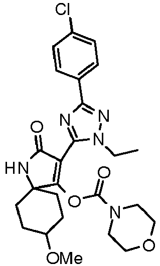

70 mg (0,17 mmol) I-8-a1 werden in 5 ml Dichlormethan gelöst. Zu dieser Lösung fügt man nacheinander 35,2 mg (0,347 mmol) Triethylamin und kühlt auf 0°C ab. Danach tropfz man eine Lösung von 52,0 mg (0,347 mmol) Morpholin-4-carbonylchlorid in 1 ml Dichlormetah hinzu. Die Mischung wird über Nacht bei Raumtemperatur gerührt. Danach wird mit Wasser gewaschen und über Magnesiumsulfat getrocknet. Die Lösung wird im Vakuum eingedampft und über HPLC gereinigt. Ausbeute: 78 mg (87%).
1H-NMR (CDCl₃): δ = 1.5 (t, 3H), 1.9 (m, 4H), ), 2.2 (m, 2H), 3.2 (m, 2H), 3.3 (m, 3H), 3.4 (s, 3H), 3.7 (m, 2H), 3.8 (m, 2H), ), 4.3 (s, 2H), 7.2 (d, 2H), 8.0 (m, 2H)

### Beispiel II-1-a3-4 = (II-1):

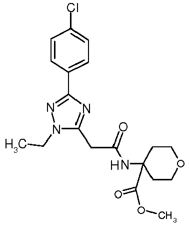

1,2 mmol (318 mg) [3-(4-Chlorphenyl)-1-ethyl-1H-1,2,4-triazol-5-yl]essigsäure und 0.1 mmol (7 mg) DMF werden in 5 ml wasserfreiem Dichlormethan gelöst. Man tropft 1,3 mmol Oxalylchlorid (163 mg) in 3 ml wasserfreiem Dichlormethan hinzu. Die Lösung wird 8 h bei Raumtemperatur gerührt und anschließend im Vakuum eingeengt. Das so erhaltene [3-(4-Chlorphenyl)-1-ethyl-1H-1,2,4-triazol-5-yl]acetyl chlorid wird roh weiterverarbeitet.

1,2 mmol (234 mg) 4-(methoxycarbonyl)tetrahydro-2H-pyran-4-aminium chlorid und 0,1 mmol (12 mg) N,N-Dimethylpyridin-4-amin (DMAP) werden in 5 ml Dichlormetan aufgenommen und auf 0 - 5°C abgekühlt. Dann tropft man 2,8 mmol (282 mg) Triethylamin in 2 ml Dichlormethan hinzu und lässt 0,25 h bei Raumtemperatur rühren. Die so erhaltene Lösung tropft man zu der oben beschriebenen Lösung aus [3-(4-Chlorphenyl)-1-ethyl-1H-1,2,4-triazol-5-yl]acetyl chlorid in 5 ml Dichlormethan und lässt 16 h bei Raumtemperatur rühren. Der Ansatz wird auf Eiswasser gegossen und mit Dichlormethan extrahiert. Die vereinigten organischen Phasen werden über MgSO₄ getrocknet und einrotiert. Das Rohprodukt wird über HPLC gereinigt. Ausbeute: 438 mg (90,0 % der Theorie).
¹H-NMR (400 MHz, CDCl₃): δ = 2.0 (m, 2H), 3.6 (s, 3H), 3.7 (m, 2H), 3.9 (m, 2H), 4.0 (s, 3H), 4.1 (s, 2H), 7.4 (d, 2H), 8.0 (d, 2H).

In Analogie zu Beispiel (II-1-a3-4) sowie gemäß den allgemeinen Angaben zur Herstellung erhält man folgende Verbindungen der Formel (II):

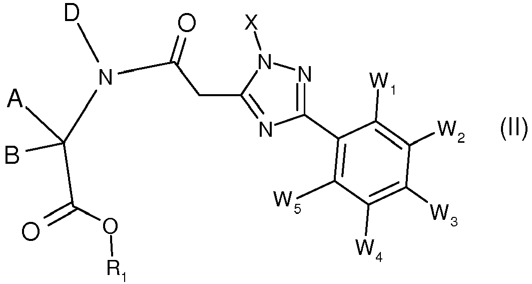

| Bsp-Nr. | X | W₁ | W₂ | W₃ | W₄ | W₅ | W₅ | R₁ | D | A | B | Analytik | Isomer |
|---|---|---|---|---|---|---|---|---|---|---|---|---|---|
| II-1-a1-5 (II-2) | CH₃ | H | H | H | H | H | H | CH₃ | H | CH₃ | CH₃ | 1H-NMR (400 MHz, CDCl₃: δ = 1.5 (s, 6H), 3.7 (s, 3H), 3.8 (s, 2H), 3.9 (s, 3H), 7.4 (m, 3H), 8.1 (d, 2H) | cis |
| II-1-a1-6 (II-3) | C₂H₅ | H | H | H | H | H | H | CH₃ | H | CH₃ | CH₃ | 1H-NMR (400 MHz, CDCl₃: δ = 1.5 (t, 3H), 1.5 (s, 6H), 3.7 (s, 3H), 3.8 (s, 2H), 4.2 (q, 2H), 7.4 (m, 3H), 8.1 (d, 2H) | cis |
| II-1-a1-7 (II-4) | i-Pr | H | H | H | H | H | H | CH₃ | H | CH₃ | CH₃ | 1H-NMR (400 MHz, CDCl₃: δ = 1.5 (d, 6H), 1.6 (s, 6H), 3.7 (s, 3H), 3.8 (s, 2H), 4.6 (m, 1H), 7.4 (m, 3H), 8.1 (d, 2H) | cis |
| II-1-a1-8 (II-5) | i-Pr | H | H | F | H | H | H | CH₃ | H | CH₃ | CH₃ | 1H-NMR (400 MHz, CDCl₃: δ = 1.5 (d, 6H), 1.6 (s, 6H), 3.7 (s, 3H), 3.8 (s, 2H), 4.6 (m, 1H), 7.1 (t, 2H), 8.1 (m, 2H) | cis |
| II-1-a1-9 (II-6) | i-Pr | H | H | Cl | H | H | H | CH₃ | H | CH₃ | CH₃ | 1H-NMR (400 MHz, CDCl₃: δ = 1.5 (d, 6H), 1.6 (s, 6H), 3.7 (s, 3H), 3.8 (s, 2H), 4.6 (m, 1H), 7.4 (d, 2H), 8.0 (d, 2H) | cis |
| II-1-a1-10 (II-7) | n-Pr | H | H | H | H | H | H | CH₃ | H | CH₃ | CH₃ | 1H-NMR (400 MHz, CDCl₃: δ = 1.0 (t, 3H), 1.5 (s, 6H), 1.9 (m, 2H), 3.7 (s, 3H), 3.8 (s, 2H), 4.1 (t, 2H), 7.4 (m, 3H), 8.1 (d, 2H) | cis |
| II-1-a1-11 (II-8) | n-Pr | H | H | F | H | H | H | CH₃ | H | CH₃ | CH₃ | 1H-NMR (400 MHz, CDCl₃: δ = 1.0 (t, 3H), 1.6 (s, 6H), 1.9 (m, 2H), 3.7 (s, 3H), 3.7 (s, 2H), 4.1 (t, 2H), 7.1 (t, 2H), 8.1 (m, 2H) | cis |
| II-1-a2-4 (II-9) | C₂H₅ | H | H | Cl | H | H | H | CH₃ | H | -(CH₂)₂-CH(OCH₃)-(CH₂)₂- | | 1H-NMR (400 MHz, CDCl₃): δ = 1.5 (t, 3H), 1.9 (m, 2H), 2.0 (m, 2H), 2.3 (m, 2H), 3.2 (m, 1H), 3.3 (s, 3H), 3.7 (s, 3H), 3.8 (s, 2H), 4.2 (q, 2H), 7.4 (d, 2H), 8.0 (d, 2H) | cis |
| II-1-a2-5 (11-10) | CH₃ | H | H | H | H | H | H | CH₃ | H | -(CH₂)₂-CH(OCH₃)-(CH₂)₂- | | 1H-NMR (400 MHz, CDCl₃): δ = 1.5 (m, 2H), 1.9 (m, 2H), 2.0 (m, 2H), 2.3 (m, 2H), 3.2 (m, 1H), 3.3 (s, 3H), 3.7 (s, 3H), 3.8 (s, 2H), 4.5 (m, 1H), 7.4 (m, 3H), 8.0 (d, 2H) | cis |
| II-1-a2-6 (11-11) | C₂H₅ | H | H | H | H | H | H | CH₃ | H | -(CH₂)₂-CH(OCH₃)-(CH₂)₂- | | 1H-NMR (400 MHz, CDCl₃): δ = 1.5 (t, 3H), 1.9 (m, 2H), 2.0 (m, 2H), 2.3 (m, 2H), 3.2 (m, 1H), 3.3 (s, 3H), 3.7 (s, 3H), 3.8 (s, 2H), 4.2 (q, 2H), 7.4 (m, 3H), 8.0 (d, 2H) | cis |
| II-1-a2-7 (11-12) | i-Pr | H | H | H | H | H | H | CH₃ | H | -(CH₂)₂-CH(OCH₃)-(CH₂)₂- | | 1H-NMR (400 MHz, CDCl₃): δ = 1.5 (d, 6H), 1.9 (m, 2H), 2.0 (m, 2H), 2.3 (m, 2H), 3.2 (m, 1H), 3.3 (s, 3H), 3.7 (s, 3H), 3.8 (s, 2H), 3.9 (s, 3H), 7.4 (m, 3H), 8.0 (d, 2H) | cis |
| II-1-a2-8 (11-13) | i-Pr | H | H | F | H | H | H | CH₃ | H | -(CH₂)₂-CH(OCH₃)-(CH₂)₂- | | 1H-NMR (400 MHz, CDCl₃): δ = 1.5 (d, 6H), 1.9 (m, 2H), 2.0 (m, 2H), 2.3 (m, 2H), 3.2 (m, 1H), 3.3 (s, 3H), 3.7 (s, 3H), 3.8 (s, 2H), 4.5 (m, 1H), 7.1 (t, 2H), 8.0 (m, 2H) | cis |
| II-1-a2-9 (11-14) | i-Pr | H | H | Cl | H | H | H | CH₃ | H | -(CH₂)₂-CH(OCH₃)-(CH₂)₂- | | 1H-NMR (400 MHz, CDCl₃): δ = 1.5 (d, 6H), 1.9 (m, 2H), 2.0 (m, 2H), 2.3 (m, 2H), 3.2 (m, 1H), 3.3 (s, 3H), 3.7 (s, 3H), 3.8 (s, 2H), 4.5 (m, 1H), 7.4 (d, 2H), 8.0 (d, 2H) | cis |
| II-1-a2-10 (11-15) | n-Pr | H | H | H | H | H | H | CH₃ | H | -(CH₂)₂-CH(OCH₃)-(CH₂)₂- | | 1H-NMR (400 MHz, CDCl₃): δ = 1.0 (t, 3H), 1,5 (m, 2H), 2.3 (m, 2H), 3.2 (m, 1H), 3.3 (s, 3H), 3.7 (s, 3H), 3.8 (s, 2H), 4.1 (t, 2H), 7.4 (d, 2H), 8.0 (d, 2H) | cis |
| II-1-a2-11 (11-16) | n-Pr | H | H | F | H | H | H | CH₃ | H | -(CH₂)₂-CH(OCH₃)-(CH₂)₂- | | 1H-NMR (400 MHz, CDCl₃): δ = 1.0 (t, 3H), 1,5 (m, 2H), 2.3 (m, 2H), 3.2 (m, 1H), 3.3 (s, 3H), 3.7 (s, 3H), 3.8 (s, 2H), 4.1 (t, 2H), 7.1 (t, 2H), 8.0 (m, 2H) | cis |
| II-1-a6-3 (II-17) | CH₃ | H | H | Cl | H | H | H | CH₃ | H | -(CH₂)₂-CH(On-Pr)-(CH₂)₂- | | 1H-NMR (400 MHz, CDCl₃): δ = 0.9 (t, 3H), 2.3 (m, 2H), 3.2 (m, 1H), 3.4 (t, 2H), 3.7 (s, 3H), 3.9 (s, 3H), 7.4 (d, 2H), 8.0 (d, 2H) | |
| II-1-a7-2 (11-18) | C₂H₅ | H | H | F | H | H | H | CH₃ | H | -(CH₂)₂-CH(Me)-(CH₂)₂- | | 1H-NMR (400 MHz, CDCl₃): δ = 0.9 (d, 3H), 1,2 (m, 2H), 1.4 (m, 1H), 1.5 (t, 3H), 1.6 (m, 2H), 1.8 (m, 2H), 2.2 (m, 2H), 3.6 (s, 3H), 4.1 (s, 2H), 4.3 (t, 2H), 7.2 (t, 2H), 8.1 (m, 2H) | |
| II-1-a7-3 (11-19) | CH₃ | H | H | Cl | H | H | H | CH₃ | H | -(CH₂)₂-CH(Me)-(CH₂)₂- | | 1H-NMR (400 MHz, CDCl₃): δ = 0.9 (d, 3H), 1,2 (m, 2H), 1.4 (m, 1H), 1.6 (m, 2H), 1.8 (m, 2H),), 2.2 (m, 2H), 3.6 (s, 3H), 4.0 (s, 2H), 4.3 (q, 2H), 7.4 (d, 2H), 8.0 (m, 2H) | |
| II-1-a7-7 (11-20) | i-Pr | H | H | H | H | H | H | CH₃ | H | -(CH₂)₂-CH(Me)-(CH₂)₂- | | 1H-NMR (400 MHz, CDCl₃): δ = 0.9 (d, 3H), 1,2 (m, 2H), 1.4 (m, 1H), 1.6 (m, 2H), 1.8 (m, 2H), 2.0 (m, 2H), 2.2 (m, 2H), 3.6 (s, 3H), 4.1 (s, 2H), 4.3 (t, 2H), 7.2 (t, 2H), 8.1 (m, 2H) | |
| II-1-Ia7-11 (11-21) | n-Pr | H | H | F | H | H | H | CH₃ | H | -(CH₂)₂-CH(Me)-(CH₂)₂- | | 1H-NMR (400 MHz, CDCl₃): δ = 0.9 (d, 3H), 1.0 (t, 3H), 1,2 (m, 2H), 1.4 (m, 1H), 1.6 (d, 6H), 1.8 (m, 2H), 2.0 (m, 2H), 2.2 (m, 2H), 4.8 (m, 1H), 7.5 (m, 3H), 8.1 (m, 2H) | |
| II-1-a9-3 (11-22) | CH₃ | H | H | Cl | H | H | H | CH₃ | H | -(CH₂)₂-N(OEt)-(CH₂)₂- | | 1H-NMR (400 MHz, CDCl₃): δ =1,2 (t, 3H), 2.2 (m, 2H), 3.7 (s 3H), 3.8 (s, 2H), 3.9 (s, 3H), 7.4 (d, 2H), 8.0 (d, 2H) | |
| II-1-a10-3 (11-23) | CH₃ | H | H | Cl | H | H | H | CH₃ | H | -(CH₂)₂-N(OMe)-(CH₂)₂- | | 1H-NMR (400 MHz, CDCl₃): δ = 2.2 (m, 2H), 3.5 (s, 3H), 3.7 (s 3H), 3.8 (s, 2H), 3.9 (s, 3H), 7.4 (d, 2H), 8.0 (d, 2H) | |
| II-1-a3-11 (11-24) | n-Pr | H | H | F | H | H | H | CH₃ | H | -(CH₂)₂-O-(CH₂)₂- | | 1H-NMR (400 MHz, CDCl₃): δ = 1.0 (t, 3H), 1.9 (m, 2H), 2.0 (m, 2H), 2.2 (m, 2H), 3.7 (m, 2H), 3.7 (s, 3H), 3.8 (s, 2H), 3.9 (m, 2H), 4.1 (t, 2H), 7.1 (t, 2H), 8.0 (m, 2H) | |

### Beispiel (XXXI-4)

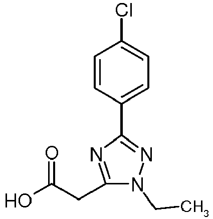

14,3 mmol (4,2 g) Ethyl [1-ethyl-3-(4-chlorphenyl)-1H-1,2,4-triazol-5-yl]acetat werden in 30 ml Ethanol gelöst. Man fügt tropfenweise 15 ml einer 15%-igen Natronlauge hinzu und rührt drei Stunden bei 45°C. Der Ansatz wird eingeengt und der Rückstand in 50 ml Eiswasser aufgenommen. Es wird tropfenweise 2N 10%-ige Salzsäure hinzugetropft bis pH = 2 erreicht ist. Der angefallene Niederschlag wird abgesaugt, mit Wasser gewaschen und getrocknet. Man erhält 3,6 g (95,2 % der Theorie).
¹H-NMR (400 MHz) in (DMSO-d₆): δ = 1,4 (t, 3H), 4,0 (s, 2H), 4,2 (q, 2H), 7,5 (d, 2H), 8,0 (d, 2H)

### Beispiel (XXXI-10)

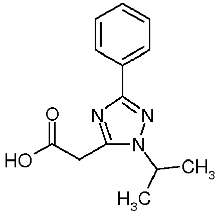

19,4 mmol (5,3 g) Ethyl (1-isopropyl-3-phenyl-1H-1,2,4-triazol-5-yl)acetat werden in 40 ml Ethanol gelöst. Man fügt tropfenweise 20 ml einer 15%-igen Natronlauge hinzu und rührt drei Stunden bei 45°C. Der Ansatz wird eingeengt und der Rückstand in 50 ml Eiswasser aufgenommen. Es wird tropfenweise 2N 10%-ige Salzsäure hinzugetropft bis pH = 2 erreicht ist. Der angefallene Niederschlag wird abgesaugt, mit Wasser gewaschen und getrocknet. Man erhält 4,5 g (94,7 % der Theorie).
¹H-NMR (400 MHz) in (DMSO-d₆): δ = 1,4 (d, 6H), 4,0 (s, 2H), 4,6 (m, 1H), 7,4 (m, 3H), 8,0 (d, 2H)

In Analogie zu Beispiel (XXXI-4) und (XXXI-10) sowie gemäß den allgemeinen Angaben zur Herstellung erhält man folgende Verbindungen der Formel (XXXI):

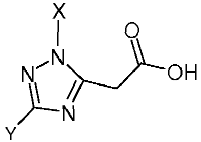

| Nr. | Y | X |
|---|---|---|
| XXXI-1 | 4-F-Ph | CH₃ |
| XXXI-2 | 4-F-Ph | C₂H₅ |
| XXXI-3 | 4-Cl-Ph | CH₃ |
| XXXI-5 | Ph | CH₃ |
| XXXI-6 | Ph | C₂H₅ |
| XXXI-7 | 4-F-Ph | i-Pr |
| XXXI-8 | 4-F-Ph | n-Pr |
| XXXI-9 | Ph | n-Pr |
| XXXI-11 | 4-F-Ph | CH₂CF₃ |
| XXXI-12 | 4-F-Ph | n-Bu |
| XXXI-13 | 4-Cl-Ph | CH₂CH₂OCH₃ |

| | | |
|---|---|---|
| Ph = Phenyl, Pr = Propyl, Bu = Butyl | | |

¹H-NMR (400 MHz) von XXXI-1 in (DMSO-d₆): δ = 3.8 (s, 3H), 4.0 (s, 2H), 7.3 (m, 2H), 8.0 (m, 2H)
¹H-NMR (400 MHz) von XXXI-2 in (DMSO-d₆): δ = 1.4 (t, 3H), 4.0 (s, 2H), 4.2 (q, 2H), 7.3 (m, 2H), 8.0 (m, 2H)
¹H-NMR (400 MHz) von XXXI-3 in (DMSO-d₆): δ = 3.8 (s, 3H), 4.0 (s, 2H), 7.5 (d, 2H), 7.9 (d, 2H)
¹H-NMR (400 MHz) von XXXI-5 in (DMSO-d₆): δ = 3.8 (s, 3H), 4.0 (s, 2H), 7.4 (m, 3H), 7.9 (d, 2H)
¹H-NMR (400 MHz) von XXXI-6 in (DMSO-d₆): δ = 1.4 (t, 3H), 4.0 (s, 2H), 4.2 (q, 2H), 7.4 (m, 3H), 8.0 (d, 2H)
¹H-NMR (400 MHz) von XXXI-7 in (DMSO-d₆): δ = 1.4 (d, 6H), 4.0 (s, 2H), 4.6 (septett, 1H), 7.3 (m, 3H), 8.0 (m, 2H)
¹H-NMR (400 MHz) von XXXI-8 in (DMSO-d₆): δ = 0.9 (t, 3H), 1.3 (m, 2H), 4.0 (s, 2H), 4.1 (t, 2H), 7.3 (m, 2H), 8.0 (m, 2H)
¹H-NMR (400 MHz) von XXXI-9 in (DMSO-d₆): δ = 0.9 (t, 3H), 1.3 (m, 2H), 4.0 (s, 2H), 4.1 (t, 2H), 7.4 (m, 3H), 8.0 (d, 2H)
¹H-NMR (400 MHz) von XXXI-11 in (CDCl₃): δ = 4.0 (s, 2H), 4.8 (m, 2H), 7.1 (m, 2H), 8.1 (m, 2H)
¹H-NMR (400 MHz) von XXXI-12 in (CDCl₃): δ = 1.0 (t, 3H), 1.4 (m, 2H), 1.9 (s, 2H), 4.1 (m, 4H), 7.1 (m, 2H), 8.1 (m, 2H)
¹H-NMR (400 MHz) von XXXI-13 in (DMSO-d₆): δ = 3.3 (s, 3H), 3.7 (t, 2H), 4.0 (s, 2H), 4.4 (t, 2H), 7.5 (d, 2H), 8.0 (d, 2H)

### Beispiel (XXXV-1)

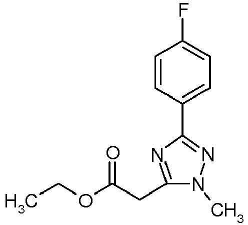

0,0915 mol (22,8 g) Ethyl [5-(4-fluorphenyl)-1H-1,2,4-triazol-3-yl]acetat werden in 225 ml Acetonitril gelöst. Unter Argon fügt man 0,183 mol (25,2 g) Kaliumcarbonat innerhalb von 15 Minuten hinzu. Anschließend werden 0,091 mol (13,0 g) Jodmethan, gelöst in 40 ml Acetonitril, innerhalb von 15 min. zugetropft. Der Ansatz wird 16 h geschüttelt. Die Reaktionslösung wird über eine Glasfritte filtriert und das Filtrat eingeengt. Das Rohprodukt wird über Säulenchromatographie an Silicagel gereinigt. Als Laufmittel wird ein Gemisch aus Hexan : Ethylacetat = 3 : 1 genutzt. Man erhält 13,7 g (56 % der Theorie).
¹H-NMR (400 MHz) von XXXV-1 in (CDCl₃): δ = 1.3 (t, 3H), 3.9 (s, 3H), 3.9 (s, 2H), 4.2 (q, 2H), 7.1 (m, 2H), 8.0 (m, 2H)

### Beispiel (XXXV-6)

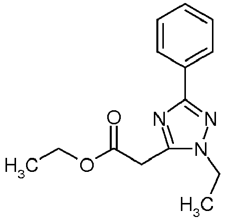

0,030 mol (7,0 g) Ethyl (5-phenyl-1H-1,2,4-triazol-3-yl)acetat werden in 60 ml DMSO gelöst. Unter Argon fügt man 0,091 mol (12,5 g) Kaliumcarbonat innerhalb von 15 Minuten hinzu. Anschließend werden 0,033 mol (5,2 g) Iodethan, gelöst in 40 ml DMSO, innerhalb von 15 min. zugetropft. Der Ansatz wird 1,5 h bei 60°C gerührt. Die Reaktionslösung wird über eine Glasfritte filtriert und das Filtrat im Hochvakuum eingeengt. Das Rohprodukt wird über HPLC gereinigt. Man erhält 5,3 g (68,8 % der Theorie).
¹H-NMR (400 MHz) von XXXV-6 in (CDCl₃): δ = 1.3 (t, 3H), 1.5 (t, 3H), 3.9 (s, 2H), 4.2 (m, 2H), 7.3 (m, 3H), 8.1 (d, 2H)

In Analogie zu Beispiel (XXXV-1) und (XXXV-6) sowie gemäß den allgemeinen Angaben zur Herstellung erhält man folgende Verbindungen der Formel (XXXI):

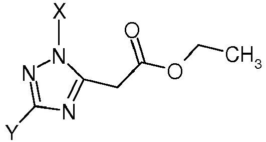

| Nr. | Y | X |
|---|---|---|
| XXXV-2 | 4-F-Ph | C₂H₅ |
| XXXV-3 | 4-Cl-Ph | CH₃ |
| XXXV-4 | 4-Cl-Ph | C₂H₅ |
| XXXV-5 | Ph | CH₃ |
| XXXV-7 | 4-F-Ph | i-Pr |
| XXXV-8 | 4-F-Ph | n-Pr |
| XXXV-9 | Ph | n-Pr |
| XXXV-10 | Ph | i-Pr |
| XXXV-11 | 4-F-Ph | CH₂CF₃ |
| XXXV-12 | 4-F-Ph | n-Bu |
| XXXV-13 | 4-Cl-Ph | CH₂CH₂OCH₃ |

¹H-NMR (400 MHz) von XXXV-2 in (CDCl₃): δ = 1.3 (t, 3H), 1.5 (t, 3H), 3.9 (s, 2H), 4.2 (m, 4H), 7.1 (m, 2H), 8.0 (m, 2H)
¹H-NMR (400 MHz) von XXXV-3 in (CDCl₃): δ = 1.3 (t, 3H), 3.9 (s, 3H), 3.9 (s, 2H), 4.2 (q, 2H), 7.4 (m, 2H), 8.0 (d, 2H)
¹H-NMR (400 MHz) von XXXV-4 in (CDCl₃): δ = 1.3 (t, 3H), 1.5 (t, 3H), 3.8 (s, 2H), 4.2 (m, 4H), 7.5 (d, 2H), 7.6 (d, 2H)
¹H-NMR (400 MHz) von XXXV-5 in (CDCl₃): δ = 1.3 (t, 3H), 3.9 (s, 3H), 3.9 (s, 2H), 4.2 (q, 2H), 7.4 (m, 3H), 8.1 (d, 2H)
¹H-NMR (400 MHz) von XXXV-7 in (CDCl₃): δ = 1.3 (t, 3H), 1.5 (d, 6H), 3.8 (s, 2H), 4.2 (q, 2H), 4.6 (septett, 1H), 7.2 (m, 2H), 7.6 (m, 2H)
¹H-NMR (400 MHz) von XXXV-8 in (CDCl₃): δ = 0.9 (t, 3H), 1.3 (t, 3H), 1.9 (m, 1H), 3.8 (s, 2H), 4.1 (t, 2H), 4.2 (q, 2H), 7.2 (m, 2H), 7.6 (m, 2H)
¹H-NMR (400 MHz) von XXXV-9 in (CDCl₃): δ = 1.0 (t, 3H), 1.3 (t, 3H), 2.0 (m, 1H), 3.8 (s, 2H), 4.1 (t, 2H), 4.2 (q, 2H), 7.4 (m, 3H), 8.1 (d, 2H)
¹H-NMR (400 MHz) von XXXV-10 in (CDCl₃): δ = 1.3 (t, 3H), 1.6 (d, 6H), 3.9 (s, 2H), 4.2 (q, 2H), 4.5 (septett, 1H), 7.4 (m, 3H), 8.1 (m, 2H)
¹H-NMR (400 MHz) von XXXV-11 in (CDCl₃): δ = 1.3 (t, 3H), 4.2 (q, 2H), 4.9 (q, 2H), 7.1 (m, 2H), 8.1 (m, 2H)
¹H-NMR (400 MHz) von XXXV-12 in (CDCl₃): δ = 1.0 (t, 3H), 1.3 (t, 3H), 1.4 (m, 2H), 2.0 (m, 2H), 4.2 (t, 2H), 4.3 (q, 2H), 4.3 (s, 2H), 7.1 (m, 2H), 8.2 (m, 2H)
¹H-NMR (400 MHz) von XXXI-13 in (CDCl₃): δ = 1.3 (t, 3H), 3.3 (s, 3H), 3.7 (t, 2H), 4.0 (s, 2H), 4.2 (q, 2H), 4.4 (t, 2H), 7.4 (d, 2H), 8.0 (d, 2H)

### Beispiel (XXXV-A-1)

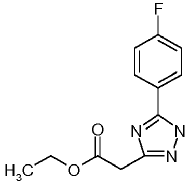

Man fügt 0,46 mol (89,7 g) 1,3-diethoxy-3-oxopropan-1-iminium chlorid zu einer gut gerührten Mischung aus 500 ml Chloroform und 300 ml gesättigter Natriumbicarbonatlösung. Nach 15 Minuten trennt man die organische Phase ab, wäscht mit Kochsalzlösung, trocknet über Magnesiumsulfat und engt das Filtrat ein.

Der Rückstand wird in Toluol aufgenommen, unter Argon gestellt und bei 0°Grad zunächst mit 0,74 mol (74,5 g) Triethylamin und anschließend tropfenweise mit einer Lösung von 0,80 mol (126,4 g) 4-Fluorbenzoylchlorid in Toluol versetzt. Man rührt über Nacht bei RT weiter. Der Niederschlag wird abfiltriert und das Filtrat eingeengt. Der Rückstand wird in 500 ml Dichlormethan gelöst und dann tropfenweise mit 500 ml einer wasserfreien 1N Hydrazinlösung in THF versetzt. Man rührt 16 h bei Raumtemperatur. Die Reaktionslösung wird zweimal mit Eiswasser gewaschen. Die vereinigten organischen Phasen werden über Magnesiumsulfat getrocknet und eingeengt. Das Rohprodukt wird über Säulenchromatographie an Silicagel gereinigt. Als Laufmittel wird ein Gemisch aus Hexan : Ethylacetat = 1 : 1 genutzt. Ausbeute: 48,2 g (42,1 % der Theorie)
¹H-NMR (400 MHz) in (DMSO-d₆): δ = 1.2 (t, 3H), 3.9 (s, 2H), 4.3 (m, 2H), 7.4 (m, 2H), 8.0 (m, 2H)

### Beispiel (XXXV-A-2)

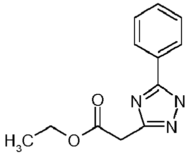

Man fügt 0,46 mol (89,7 g) 1,3-diethoxy-3-oxopropan-1-iminium chlorid zu einer gut gerührten Mischung aus 500 ml Chloroform und 300 ml gesättigter Natriumbicarbonatlösung. Nach 15 Minuten trennt man die organische Phase ab, wäscht mit Kochsalzlösung, trocknet über Magnesiumsulfat und engt das Filtrat ein.

Der Rückstand wird in Toluol aufgenommen, unter Argon gestellt und bei 0°Grad zunächst mit 0,048 mol (74,5 g) Triethylamin und anschließend tropfenweise mit einer Lösung von 0,80 mol (140,0 g) 4-Chlorbenzoylchlorid in Toluol versetzt. Man rührt über Nacht bei RT weiter. Der Niederschlag wird abfiltriert, das Filtrat eingeengt. Der Rückstand wird in 500 ml Dichlormethan gelöst und dann tropfenweise mit 500 ml einer wasserfreien 1N Hydrazinlösung in THF versetzt. Man rührt 16 h bei Raumtemperatur.

Die Reaktionslösung wird 2 x mit Eiswasser gewaschen. Die vereinigten organischen Phasen werden über Magnesiumsulfat getrocknet und eingeengt. Das Rohprodukt wird über Säulenchromatographie an Silicagel gereinigt. Als Laufmittel wird ein Gemisch aus Hexan : Ethylacetat = 1: 1 genutzt. Ausbeute: 47,3 g (44,5 % der Theorie)
¹H-NMR (400 MHz) in (CDCl₃): δ = 1.3 (t, 3H), 4.0 (s, 2H), 4.3 (q, 2H), 7.4 (m, 3H), 8.0 (m, 2H)

### Beispiel I-2a3-2 = (I-2-a-1)

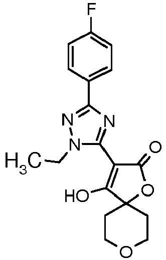

208 mg (1,85 mmol) Kalium-t-butylat werden in 3 ml DMF vorgelegt und auf 0°C gekühlt. Eine Lösung von 300 mg (0,74 mmol) Ethyl 4-Ethoxy-2-{2-[1-ethyl-3-(4-fluorphenyl)-1H-1,2,4-triazol-5-yl]acetoxy}butanoat in 3 ml DMF bei RT zugetropft und über Nacht bei Raumtemperatur gerührt.

Zur Aufarbeitung wird das DMF abrotiert, der Rückstand in Wasser verrührt, die alk. Phase mit Methyl-t-butylether extrahiert, die wäßrige Phase mit Salzsäure, mit Dichlormethan extrahiert, getrocknet, abfiltriert und eingeengt. Das Rohprodukt wird mittels Chromatographie an Kieselgel (Eluent Essigester/Cyclohexan) gereinigt. Ausbeute: 195 mg (73% d.Th.)
1H-NMR (CDCl₃): δ = 1.5 (t, 3H), 2.2 (m, 2H), 3.9 (m, 2H), 4.0 (m, 2H), 5.0 (q, 2H), 7.3 (m, 2H), 7.9 (m, 2H)

In Analogie zu dem Beispiel (I-2-a3-2) und gemäß den allgemeinen Angaben zur Herstellung erhält man folgende Verbindungen der Formel (I-2-a):

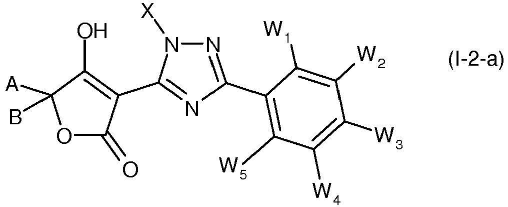

| Bsp. Nr. | X | W₁ | W₂ | W₃ | W₄ | W₅ | A | B | Analytik | Isomer |
|---|---|---|---|---|---|---|---|---|---|---|
| I-2-a1-2 (I-2-a-2) | C₂H₅ | H | H | F | H | H | CH₃ | CH₃ | a) | |
| I-2-a2-3a (I-2-a-3) | CH₃ | H | H | Cl | H | H | -(CH₂)₂-CHOCH₃-(CH₂)₂- | | b) | cis |
| I-2-a2-3b (I-2-a-4) | CH₃ | H | H | Cl | H | H | -(CH₂)₂-CHOCH₃-(CH₂)₂- | | c) | trans |
| I-2-a6-3 (I-2-a-5) | CH₃ | H | H | Cl | H | H | -(CH₂)₂-CHCH₂CH₃- (CH₂)₂- | | d) | |
| I-2-a2- 12a (I-2-a-6) | n-Pr | H | H | F | H | H | -(CH₂)₂-CHOCH₃-(CH₂)₂- | | e) | cis |
| I-2-a2- 12b (I-2-a-7) | n-Pr | H | H | F | H | H | -(CH₂)₂-CHOCH₃-(CH₂)₂- | | f) | trans |

| | | | | | | | | | | |
|---|---|---|---|---|---|---|---|---|---|---|
| a) 1H-NMR (CDCl₃): δ = 1.5 (s, 6H), 1.5 (t, 3H), 2.0 (m, 4H), 5.0 (q, 2H), 7.3 (m, 2H), 7.9 (m, 2H) b) 1H-NMR (CDCl₃): δ = 1.7 (m, 2H), 1.8 (m, 2H), 1.9 (m, 2H), 2.1 (m, 2H), 3.3 (m, 1H), 3.4 (s, 3H), 4.5 (s, 3H), 7.5 (d, 2H), 7.8 (d, 2H) c) 1H-NMR (CDCl₃): δ = 1.5 (m, 2H), 1.9 (m, 2H), 2.0 (m, 2H), 2.2 (m, 2H), 3.3 (s, 3H), 3.6 (m, 1H), 4.5 (s, 3H), 7.5 (d, 2H), 7.8 (d, 2H) d) 1H-NMR (CDCl₃): δ = 0.9 (t, 3H),1.4 (m, 2H), 1.7 (m, 4H), 1.9 (m, 2H), 2.0 (m, 2H), 4.5 (s, 3H), 7.5 (d, 2H), 7.8 (d, 2H) e) 1H-NMR (CDCl₃): δ = 1.0 (t, 3H), 1.7 (m, 2H), 1.9 (m, 2H), 3.3 (m, 1H), 3.4 (s, 3H), 4.5 (t, 2H), 7.1 (t, 2H), 8.1 (m, 2H) f) 1H-NMR (CDCl₃): δ =1.0 (t, 3H), 1.5 (m, 2H), 1.9 (m, 2H), 2.2 (m, 2H), 3.3 (s, 3H), 3.6 (m, 1H), 4.8 (t, 3H), 7.2 (t, 2H), 8.0 (m, 2H) | | | | | | | | | | |

### Beispiel III-1-a2-3 = (III-1)

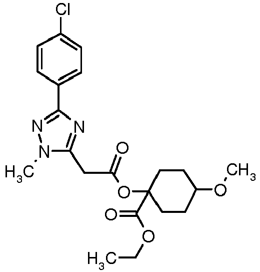

500 mg (1,99 mmol) Triazolylessigsäure XXXI-3, 442 mg (2,2 mmol) Ethyl 1-Hydroxy-4-methoxycyclohexanecarboxylat und 513 mg (4.0 mmol) N-Ethyl-diisopropylamin werden in 5 ml Dichlormethan gelöst und auf 0°C abgekühlt. Unter Rühren tropft man von 1,52 g 2,4,6-Tripropyl-1,3,5,2,4,6-trioxatriphosphinane 2,4,6-trioxide (T3P) in 3 ml THF langsam zu und schüttelt über Nacht. Der Ansatz wird eingeengt und mittels HPLC gereinigt: Ausbeute: 435 mg (67%)
1H-NMR (CDCl₃): δ = 1.2 (t, 3H), 1.5 (m, 2H), 1.8 (m, 2H), 1.9 (m, 2H), 2.3 (m, 2H), 3.2 (m, 1H), 3.3 (s, 3H), 3.9 (s, 3H), 4.0 (s, 2H), 4.2 (q, 2H), 7.4 (d, 2H), 8.0 (d, 2H)

### Beispiel (I-8-a1)

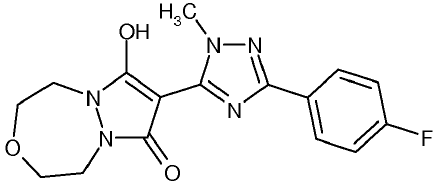

308 mg (2,74 mmol) Kalium-t-butylat werden in 3 ml DMF vorgelegt und auf 0°C gekühlt. Eine Lösung von 300 mg (0,74 mmol) Methyl 5-{[3-(4-fluorophenyl)-1-methyl-1H-1,2,4-triazol-5-yl]acetyl}-1,4,5-oxadiazepan-4-carboxylat in 3 ml DMF bei RT zugetropft und über Nacht bei Raumtemperatur gerührt. Zur Aufarbeitung wird das DMF abrotiert, der Rückstand in Wasser verrührt, die alk. Phase mit Methyl-t-butylether extrahiert, die wäßrige Phase mit Salzsäure, mit Dichlormethan extrahiert, getrocknet, abfiltriert und eingeengt. Das Rohprodukt wird mittels Chromatographie an Kieselgel (Eluent Essigester/Cyclohexan) gereinigt. Ausbeute: 188 mg (69% d.Th.)
1H-NMR (CDCl₃): δ = 3.9 (m, 4H), 4.0 (m, 4H), 4.5 (s, 3H), 7.2 (t, 2H), 7.9 (m, 2H)

In Analogie zu dem Beispiel (I-8-a1) und gemäß den allgemeinen Angaben zur Herstellung erhält man folgende Verbindungen der Formel (I-8-a):

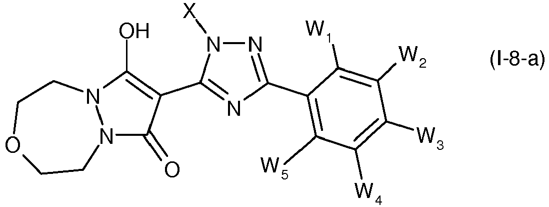

| Bsp. Nr. | X | W₁ | W₂ | W₃ | W₄ | W₅ | Analytik |
|---|---|---|---|---|---|---|---|
| I-8-a-2 | i-Pr | H | H | H | H | H | a) |
| I-8-a-3 | CH₃ | H | H | Cl | H | H | b) |
| I-8-a-4 | n-Pr | H | H | F | H | H | c) |
| I-8-a-5 | C₂H₅ | H | H | F | H | H | d) |

| | | | | | | | |
|---|---|---|---|---|---|---|---|
| a) 1H-NMR (CDCl₃): δ = 1.5 (d, 6H), 3.9 (m, 4H), 4.0 (m, 4H), 6.5 (m, 1H), 7.2 (m, 2H), 7.9 (m, 2H) b) 1H-NMR (DMSO-d₆): δ = 3.6 (m, 4H), 3.8 (m, 4H), 4.0 (s, 3H), 7.5 (d, 2H), 8.0 (d, 2H) c) 1H-NMR (CDCl₃): δ = 1.0 (t, 3H), 2.0 (m, 2H), 3.9 (m, 4H), 4.1 (m, 4H), 4.8 (t, 2H), 7.2 (m, 2H), 8.0 (m, 2H) d) 1H-NMR (CDCl₃): δ = 1.5 (t, 3H), 3.9 (m, 4H), 5.0 (q, 2H), 7.2 (m, 2H), 7.9 (m, 2H) | | | | | | | |

### Beispiel I-8-b1 = (I-8-g-1)

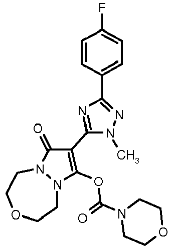

45 mg (0,13 mmol) I-8-a1 werden in 5 ml Dichlormethan gelöst. Zu dieser Lösung fügt man nacheinander 19,8 mg (0,195 mmol) Triethylamin und kühlt auf 0°C ab. Danach tropfz man eine Lösung von 21 mg (0,143 mmol) Morpholin-4-carbonylchlorid in 1 ml Dichlormetah hinzu. Die Mischung wird über Nacht bei Raumtemperatur gerührt. Danach wird mit Wasser gewaschen und über Magnesiumsulfat getrocknet. Die Lösung wird im Vakuum eingedampft und über HPLC gereinigt. Ausbeute: 54 mg (92%).
1H-NMR (CDCl₃): δ = 3.4 (m, 2H), 3.6 (m, 2H), 3.7 (m, 4H), 4.0 (m, 4H), 4.1 (s, 3H), 4.2 (m, 2H), 4.3 (m, 2H), 7.2 (t, 2H), 7.9 (m, 2H)

### Beispiel XII-8-a1 = (XII-1)

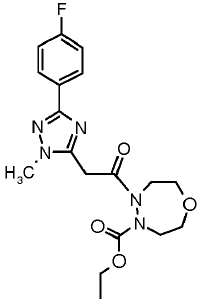

500 mg (2,13 mmol) Triazolylessigsäure XXXI-1, 403 mg (2,32 mmol) Ethyl 1,4,5-oxadiazepane-4-carboxylat und 824 mg (6.4 mmol) N-Ethyl-diisopropylamin werden in 5 ml Dichlormethan gelöst und auf 0°C abgekühlt. Unter Rühren tropft man von 1,52 g 2,4,6-Tripropyl-1,3,5,2,4,6-trioxatriphosphinane 2,4,6-trioxide (T3P) in 3 ml THF langsam zu und schüttelt über Nacht. Der Ansatz wird eingeengt und mittels HPLC gereinigt: Ausbeute: 673 mg (81%)
1H-NMR (CDCl₃): δ = 1.3 (m, 3H), 3.9 (s, 3H), 7.1 (t, 2H), 8.0 (m, 2H)

In Analogie zu dem Beispiel (XII-8-a-1) und gemäß den allgemeinen Angaben zur Herstellung erhält man folgende Verbindungen der Formel (XII):

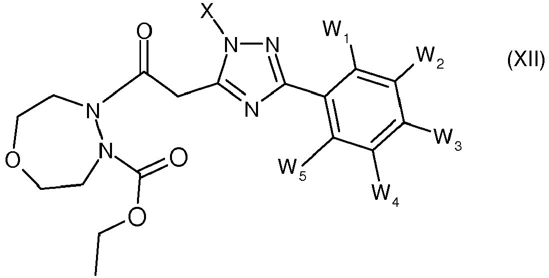

| Bsp. Nr. | X | W₁ | W₂ | W₃ | W₄ | W₅ | Analytik |
|---|---|---|---|---|---|---|---|
| XII-8-a2 (XII-2) | i-Pr | H | H | H | H | H | a) |
| XII-8-a3 (XII-3) | CH₃ | H | H | Cl | H | H | b) |
| XII-8-a4 (XII-4) | n-Pr | H | H | F | H | H | c) |
| XII-8-a5 (XII-5) | Et | H | H | F | H | H | d) |

| | | | | | | | |
|---|---|---|---|---|---|---|---|
| a) 1H-NMR (CDCl₃): δ = 1.3 (m, 3H), 1.5 (d, 3H),), 1.6 (d, 3H), 4.6 (septett, 1H), 7.4 (m, 3H), 8.1 (d, 2H) b) 1H-NMR (CDCl₃): δ = 1.3 (m, 3H), 3.9 (s, 3H), 7.4 (d, 2H), 8.0 (d, 2H) c) 1H-NMR (CDCl₃): δ = 1.0 (t, 3H), 1.3 (m, 3H),), 2.0 (m, 2H), 4.6 (septett, 1H), 7.1 (m, 2H), 8.0 (m, 2H) d) 1H-NMR (CDCl₃): δ = 1.3 (m, 3H), 1.5 (t, 3H),), 4.2 (q, 2H), 7.1 (m, 2H), 8.0 (m, 2H) | | | | | | | |

Bei Herstellungsbeispielen, die zwei Beispielnummern besitzen, bezieht sich die zweite Nummerierung auf die Erklärungen in der Beschreibung.

### Beispiel 1

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von ca. 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Alopecurus myosuroides, Avena fatua, Echinocloa crus-galli, Lolium multiflorum und Setaria viridis eine Wirkung von ≥ 80 %: I-1-a1-1, I-1-a1-2, I-1-a1-4, I-1-a1-9, I-1-a1-10, 1-1-a2-1, I-1-a2-2, I-1-a2-3, I-1-a2-4, I-1-a2-6, I-1-a2-7, I-1-a2-9, I-1-a2-10, I-1-a3-2, I-1-a3-3, I-1-a3-4, I-1-a3-6, I-1-a4-3, I-1-a4-2, I-1-a4-4, I-1-a4-11, I-1-a5-8, I-1-a6-3, I-1-a7-3, I-1-a8-3, I-1-a9-3, 1-1-a10-3, I-1-a11-3, I-1-a12-3, I-1-a13-3, I-2-a2-12a.

### Beispiel 2

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 320 g/ha gegen Alopecurus myosuroides, Avena fatua, Echinocloa crus-galli, Lolium multiflorum und Setaria viridis eine Wirkung von ≥ 80 %: I-1-a1-1, I-1-a1-2, I-1-a1-4, I-1-a2-1, I-1-a2-2, I-1-a2-3, I-1-a2-4, I-1-a2-6, I-1-a2-9, I-1-a2-10, I-1-a3-1, I-1-a3-2, I-1-a3-3, I-1-a3-4, I-1-a4-1, I-1-a4-2, I-1-a4-3, I-1-a4-4, I-1-a5-4, I-1-a7-3, I-1-a8-3, I-1-a9-3, I-1-a10-3,1-2a3-2.

### Beispiel 3: Lucilia cuprina (48h)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 48 Stunden wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 90 % bei einer Aufwandmenge von 100 ppm : I-1-a1-3.

### Beispiel 4: Tetranychus - Sprühtest: OP-resistent (TETRUR)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele eine Wirkung von 100 % bei einer Aufwandmenge von 500 g/ha: I-1-a1-3, I-1-a2-1, I-1-a2-3, I-1-a3-1, I-1-a4-4, I-1-a8-3, I-2-a2-3a, I-2-a2-3b.

Bei diesem Test zeigen z.B. die folgenden Verbindungen der Herstellungsbeispiele eine Wirkung von 90 % bei einer Aufwandmenge von 500 g/ha: I-1-a6-3, I-1-a7-3, I-2-a2-12a.

Bei diesem Test zeigen z.B. die folgenden Verbindungen der Herstellungsbeispiele eine Wirkung von 80 % bei einer Aufwandmenge von 500 g/ha: I-1-a1-4.

Bei diesem Test zeigen z.B. die folgenden Verbindungen der Herstellungsbeispiele eine Wirkung von 90 % bei einer Aufwandmenge von 100 g/ha: I-1-a1-9, I-1-a9-3, I-1-a10-3, I-1-a11-3, I-8-a3, I-8-a5.

### Beispiel 5: Myzus persicae - Sprühtest (MYZUPE)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100 % bei einer Aufwandmenge von 500 g/ha: I-1-a2-1, I-1-a2-3, I-1-a2-5, I-1-a3-5, I-2-a2-3a, I-2-a2-3b.

Bei diesem Test zeigen z. B. die folgenden Verbindungen der Herstellungsbeispiele Wirkung von 90 % bei einer Aufwandmenge von 500 g/ha: I-1-a6-3, I-1-a7-3, I-1-a9-3, I-1-a10-3, I-1-a11-3, I-1-a13-3, I-2-a2-12a, I-8-a3.

Bei diesem Test zeigen z. B. die folgenden Verbindungen der Herstellungsbeispiele Wirkung von 80 % bei einer Aufwandmenge von 500 g/ha: : I-1-a1-4.

### Beispiel 6: Phaedon cochleariae - Sprühtest (PHAECO)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 500 g/ha: I-1-a6-3.

## Patentansprüche

1. Verbindungen der Formel (I) 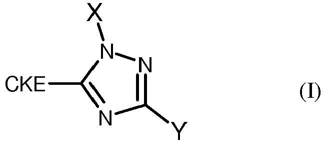 in welcher
X für Methyl, Ethyl, n-Propyl, i-Propyl, n-Butyl, -CH₂CF₃ oder Cyclopropyl steht,
Y für Phenyl, 4-Cl-Benzyl, 4-F-Phenyl, 4-Cl-Phenyl oder 2,4-Cl₂-Phenyl steht,
CKE für eine der Gruppen 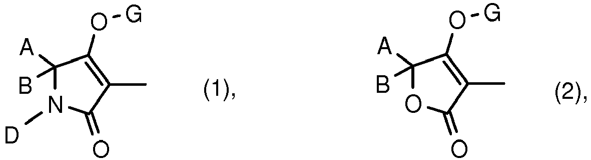 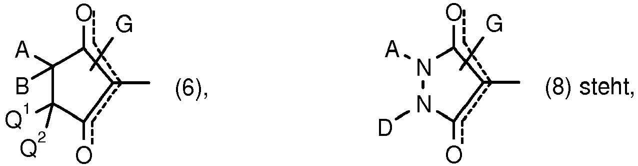
A für Wasserstoff, Methyl, Ethyl oder Cyclopropyl steht,
B für Wasserstoff, Methyl oder Cyclopropyl steht,
A, B und das Kohlenstoffatom an das sie gebunden sind, für gesättigtes C₅-C₆-Cycloalkyl, in welchem gegebenenfalls ein Ringglied durch Sauerstoff oder Stickstoff ersetzt ist und welches gegebenenfalls einfach oder zweifach durch Methyl, Ethyl, Methoxymethyl, Methoxy, Ethoxy, Propoxy, Butoxy, Trifluorethoxy, Trifluormetyhl oder -O-CH₂CHCH₂ substituiert ist, wobei Methoxy oder Ethoxy auch als N-Substituenten in Frage kommen, oder
A, B und das Kohlenstoffatom, an das sie gebunden sind, für C₆-Cycloalkyl stehen, welches gegebenenfalls durch eine mit zwei nicht direkt benachbarten Sauerstoffatomen enthaltende Alkylendioxyl-Gruppe substituiert ist, wobei ein weiterer 5- oder 6-Ring gebildet wird, der einfach oder zweifach durch Methyl substituiert sein kann,
D für Wasserstoff oder cyclo-Propyl steht, oder
A und D gemeinsam für C₃-C₅-Alkandiyl stehen, worin gegebenenfalls ein Kohlenstoffatom durch Sauerstoff ersetzt ist oder
A und D gemeinsam für C₃-C₅-Alkandiyl stehen, welches gegebenenfalls durch eine mit zwei nicht direkt benachbarten Sauerstoffatomen enthaltende gegebenenfalls einfach bis zweifach durch Methyl substituierte Alkylendioxyl-Gruppe substituiert ist, wobei ein weiterer 5- Ring gebildet wird, oder
A und Q¹ gemeinsam für C₃-C₄-Alkandiyl stehen,
Q² für Wasserstoff steht,
G für Wasserstoff (a) oder für eine der Gruppen 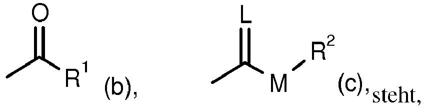
in welchen
L für Sauerstoff steht,
M für Sauerstoff steht,
R¹ für C₁-C₆-Alhyl steht,
R² für C₁-C₆-Alkyl steht,
G für die Gruppe (g) 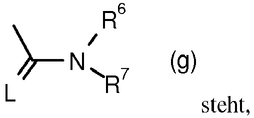 wobei L für Sauerstoff steht und
R⁶ und R⁷ zusammen für einen C₅-Alkylenrest stehen, in welchem ein Kohlenstoffatom durch Sauerstoff ersetzt ist.

2. Verbindungen der Formel (I) gemäß Anspruch 1, in welcher
X für Methyl oder Ethyl steht,
Y für 4-F-Phenyl oder 4-Cl-Phenyl steht,
CKE für eine der Gruppen 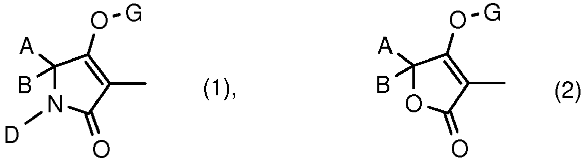 steht,
A für Wasserstoff oder Methyl steht,
B für Wasserstoff oder Methyl steht,
A, B und das Kohlenstoffatom an das sie gebunden sind, für gesättigtes C₆-Cycloalkyl stehen, in welchem gegebenenfalls ein Ringglied durch Sauerstoff ersetzt ist und welches gegebenenfalls einfach durch Methyl oder Methoxy substituiert ist,
D für Wasserstoff oder cyclo-Propyl steht, oder
A und D gemeinsam hervorgehoben für C₃-Alkandiyl stehen,
G für Wasserstoff (a) steht.

3. Verbindungen der Formel (I) gemäß Anspruch 1, in welcher
X für Methyl, Ethyl, i-Propyl, n-Propyl, n-Butyl oder -CH₂-CF₃ steht,
Y für Phenyl, 4-Cl-Benzyl, 4-F-Phenyl, 4-Cl-Phenyl oder 2,4-Cl₂-Phenyl steht,
CKE für eine der Gruppen 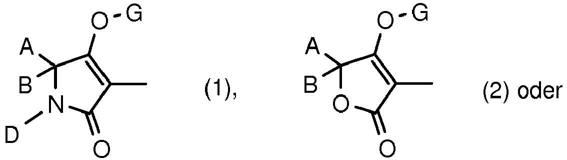 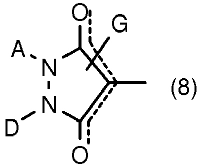 steht,
A für Wasserstoff, Methyl oder cyclo-Propyl steht,
B für Wasserstoff, Methyl oder cyclo-Propyl steht,
A, B und das Kohlenstoffatom an das sie gebunden sind, für gesättigtes C₆-Cycloalkyl, in welchem gegebenenfalls ein Ringglied durch Sauerstoff oder Stickstoff ersetzt ist und welches gegebenenfalls einfach oder zweifach durch Methyl, Ethyl, Methoxy, Ethoxy, n-Propoxy, Trifluormethyl oder -O-CH₂CHCH₂ substituiert ist, wobei Methoxy oder Ethoxy auch als N-Substituenten in Frage kommen,
A, B und das Kohlenstoffatom, an das sie gebunden sind, für C₆-Cycloalkyl stehen, welches gegebenenfalls durch eine mit zwei nicht direkt benachbarten Sauerstoffatomen enthaltende Alkylendioxyl-Gruppe substituiert ist, wobei ein weiterer 6-Ring gebildet wird, der einfach durch Methyl substituiert sein kann,
D für Wasserstoff oder cyclo-Propyl steht, oder
A und D gemeinsam für C₃-Alkandiyl stehen,
A und D im Fall von CKE = Gruppe (8) gemeinsam für -(CH₂)₂-O-(CH₂)₂- stehen,
G für Wasserstoff (a) oder für die Gruppe (g) 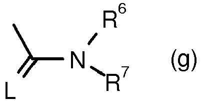
steht, wobei L für Sauerstoff steht und
R⁶ und R⁷ zusammen für einen C₅-Alkylenrest stehen, in welchem ein Kohlenstoffatom durch Sauerstoff ersetzt ist.

4. Verfahren zur Herstellung von Verbindungen der Formel (I) gemäß Anspruch 1, **dadurch gekennzeichnet, dass** man zum Erhalt von
(A) Verbindungen der Formel (I-1-a) 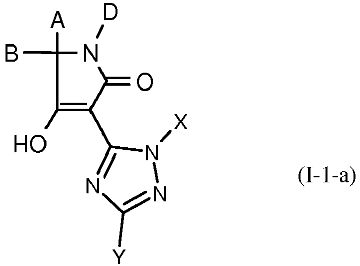 in welcher
A, B, D, X und Y die in Anspruch 1 angegebenen Bedeutungen haben,
Verbindungen der Formel (II) 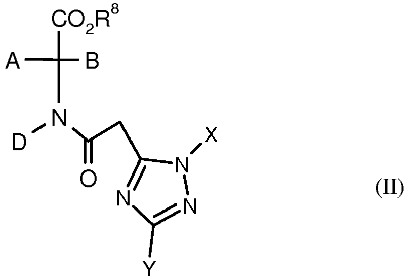 in welcher
A, B, D, X und Y die in Anspruch 1
angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 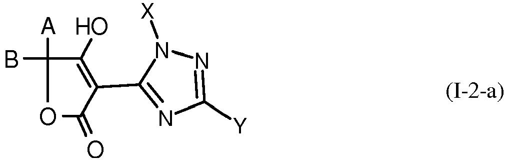 in welcher
A, B, X und Y die in Anspruch 1 angegebenen Bedeutungen haben,
Verbindungen der Formel (III) 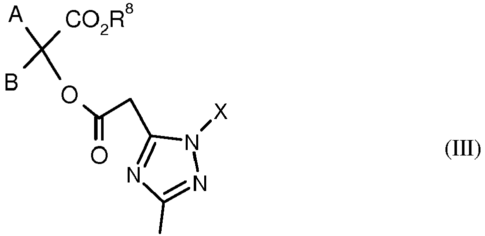 in welcher
A, B, X, Y, und R⁸ die in Anspruch 1 angegebenen Bedeutungen haben,
in Gegenwart eines Verdünnungsmittels und in Gegenwart einer Base intramolekular kondensiert,
(F) Verbindungen der Formel (I-6-a) 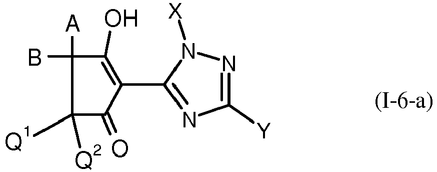 in welcher
A, B, Q¹, Q², X und Y die in Anspruch 1 angegebene Bedeutung haben, Verbindungen der Formel (VIII) 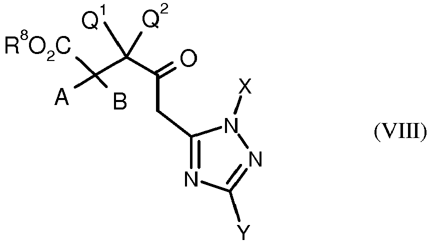 in welchen
A, B, Q¹, Q², X und Y die in Anspruch 1 angegebene Bedeutung haben, und
R⁸ für Alkyl steht,
gegebenenfalls in Gegenwart eines Verdünnungsmittels und in Gegenwart einer Base intramolekular cyclisiert,
(H) Verbindungen der Formel (I-8-a) 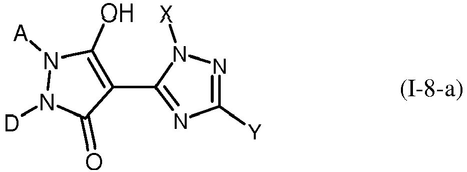 in welcher
A, D, X und Y die in Anspruch 1 angegebenen Bedeutungen haben, Verbindungen der Formel (X) 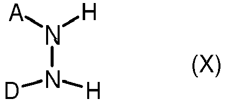 in welcher
A und D die oben angegebene Bedeutung haben,
α) mit Verbindungen der Formel (VI) 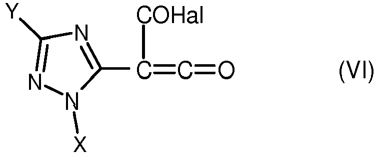 in welcher
Hal, X und Y die in Anspruch 4 angegebenen Bedeutungen haben, gegebenenfalls in Gegenwart eines Verdünnungsmittels und gegebenenfalls in Gegenwart eines Säureakzeptors umsetzt, oder
ß) mit Verbindungen der Formel (XI) 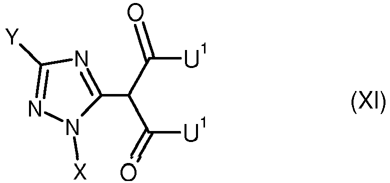 in welcher
X und Y die in Anspruch 1 angegebene Bedeutung haben,
und U¹ für NH₂ oder O-R⁸ gegebenenfalls in Gegenwart eines Verdünnungsmittels und gegebenenfalls in Gegenwart einer Base umsetzt, oder
γ) Verbindungen der Formel (XII) 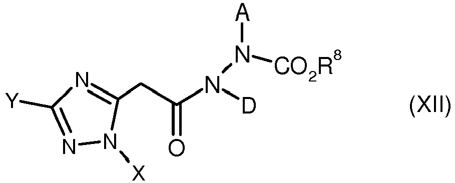 in welcher
A, D, X, Y und R⁸ die in Anspruch 1 angegebene Bedeutung haben, gegebenenfalls in Gegenwart eines Verdünnungsmittels und gegebenenfalls in Gegenwart einer Base umsetzt,
(L) Verbindungen der oben gezeigten Formeln (I-1-b), (I-2-b), (I-6-b), (I-8-b), in welchen A, B, D, Q¹, Q², R¹, X und Y die in Anspruch 1 angegebenen Bedeutungen haben,
Verbindungen der oben gezeigten Formeln (I-1-a), (I-2-a), (I-6-a), (I-8-), in welchen A, B, D, Q¹, Q², X und Y die in Anspruch 1 angegebenen Bedeutungen haben, jeweils
(α) mit Säurehalogeniden der Formel (XVI) 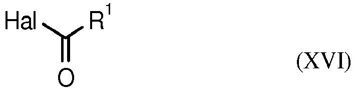 in welcher
R¹ die in Anspruch 1 angegebene Bedeutung hat und
Hal für Halogen steht
oder
(ß) mit Carbonsäureanhydriden der Formel (XVII)
R¹-CO-O-CO-R¹ (XVII)
in welcher
R¹ die in Anspruch 1 angegebene Bedeutung hat,
gegebenenfalls in Gegenwart eines Verdünnungsmittels und gegebenenfalls in Gegenwart eines Säurebindemittels umsetzt;
(M) Verbindungen der oben gezeigten Formeln (I-1-c), (I-2-c), (I-6-c), (I-8-c), in welchen A, B, D, Q¹, Q², R², M, X, und Y die oben angegebenen Bedeutungen haben und L für Sauerstoff steht, Verbindungen der oben gezeigten Formeln (I-1-a), (I-2-a), (I-6-a), (I-8-a), in welchen A, B, D, Q¹, Q², X und Y die oben angegebenen Bedeutungen haben, jeweils
mit Chlorameisensäureestern der Formel (XVIII)
R²-M-CO-Cl (XVIII)
in welcher
R² und M die in Anspruch 1 angegebenen Bedeutungen haben, gegebenenfalls in Gegenwart eines Verdünnungsmittels und gegebenenfalls in Gegenwart eines Säurebindemittels umsetzt;
(R) Verbindungen der oben gezeigten Formeln (I-1-g), (I-2-g), (I-6-g), (I-8-g), in welchen
A, B, D, L, Q¹, Q², R⁶, R⁷, X und Y die in Anspruch 1 angegebenen Bedeutungen haben, Verbindungen der oben gezeigten Formeln (I-1-a), (I-2-a), (I-6-a), (I-8-a), in welchen
A, B, D, Q¹, Q², X und Y die in Anspruch 1 angegebenen Bedeutungen haben, jeweils
(α) mit Isocyanaten der Formel (XXIV)
R⁶-N=C=L (XXIV)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt oder
(ß) mit Carbamidsäurechloriden der Formel (XXV) 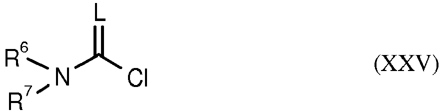 in welcher
L, R⁶ und R⁷ die in Anspruch 1 angegebenen Bedeutungen haben, (VI)
gegebenenfalls in Gegenwart eines Verdünnungsmittels und gegebenenfalls in Gegenwart eines Säurebindemittels, umsetzt.

5. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

6. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

7. Verwendung von Verbindungen der Formel (I) gemäß Anspruch 1 zur Bekämpfung von tierischen Schädlingen und/oder unerwünschtem Pflanzenbewuchs.

8. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

9. Verwendung von Verbindungen der Formel (I) gemäß Anspruch 1 zur Herstellung von Mitteln zur Bekämpfung von Schädlingen und/oder unerwünschtem Pflanzenwuchs.

10. Mittel enthaltend einen wirksamen Gehalt an einer Wirkstoffkombination umfassend als Komponenten
(a') mindestens eine Verbindung der Formel (I), in welcher X, Y und CKE die in Anspruch 1 angegebene Bedeutung haben und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14, S15.

11. Mittel gemäß Anspruch 10, bei denen die Kulturpflanzen-Verträglichkeit verbessernde Verbindung Mefenpyr-diethyl ist.

12. Verfahren zum Bekämpfen von unerwünschtem Pflanzenwuchs, **dadurch gekennzeichnet, dass** man ein Mittel gemäß Anspruch 10 auf die Pflanzen oder ihre Umgebung einwirken lässt.

13. Verwendung eines Mittels gemäß Anspruch 10 zum Bekämpfen von unerwünschten Pflanzenwuchs.

14. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 10 in zeitlich naher Abfolge getrennt auf die Pflanzen oder ihre Umgebung einwirken lässt.

15. Verbindungen der Formel (II) 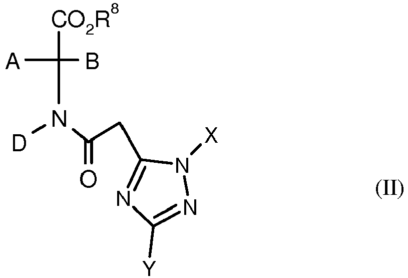 in welcher
A, B, D, X und Y die in Anspruch 1 angegebenen Bedeutungen haben, und
R⁸ für Alkyl steht.

16. Verbindungen der Formel (III) 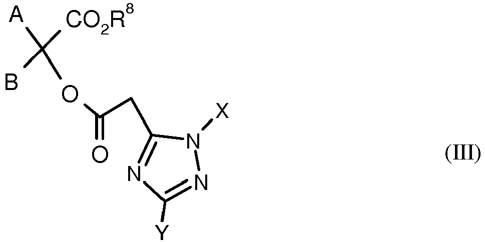 in welcher (III)
A, B, X, Y, und R⁸ die in Anspruch 1 angegebenen Bedeutungen haben.

17. Verbindungen der Formel (VI) 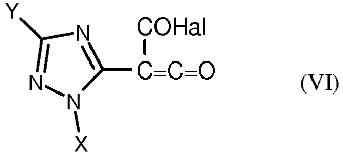 in welcher
X und Y die in Anspruch 1 angegebenen Bedeutungen haben und
Hal für Halogen steht.

18. Verbindungen der Formel (VIII) 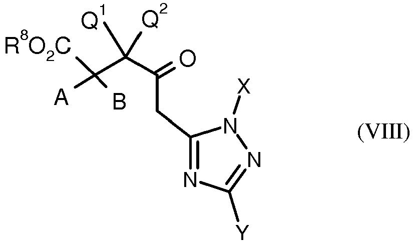 in welchen
A, B, Q¹, Q², R⁸, X und Y die in Anspruch 1 angegebene Bedeutung haben.

19. Verbindungen der Formel (XII) 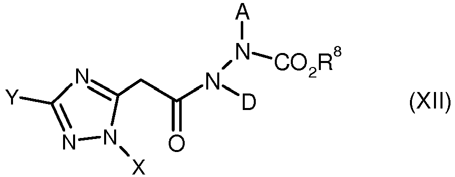 in welcher
A, D, X, Y und R⁸ die in Anspruch 1 angegebene Bedeutung haben

20. Verbindungen der Formel (XI) 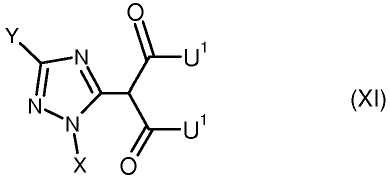 in welcher
X und Y die in Anspruch 1 angegebene Bedeutung haben,
und U¹ für NH₂ oder O-R⁸ steht, wobei R⁸ die in Anspruch 15 genannte Bedeutung hat.

21. Verbindungen der Formel (XXVIII) 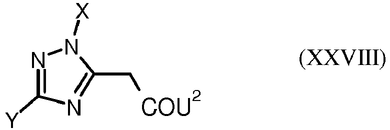 in welcher
X und Y die in Anspruch 1 angegebenen Bedeutungen haben und
U² für eine durch Carbonsäureaktivierungsreagenzien, Propanephosphonicsäureanhydrid, Phosphorylierungsreagenzen, Halogenierungsmittel, Phosgen oder Chlorameisensäsureester eingeführte Abgangsgruppe steht.

22. Verbindungen der Formel (XXIX) 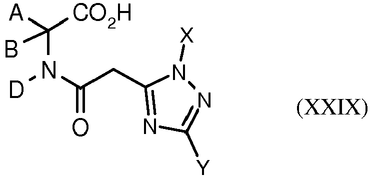 in welcher
A, B, D, X und Y die in Anspruch 1 angegebenen Bedeutungen haben.

23. Verbindunen der Formel (XXXI) 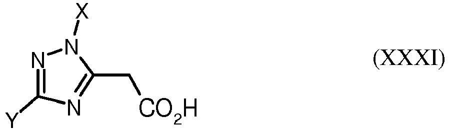 in welcher
X und Y die in Anspruch 1 angegebene Bedeutung haben.

24. Verbindungen der Formel (XXXIII) 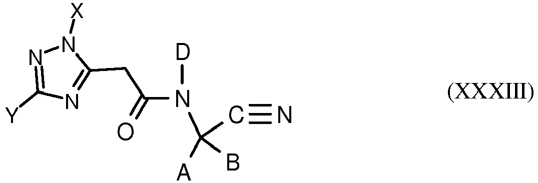 in welcher
A, B, D, X und Y die in Anspruch 1 angegebenen Bedeutungen haben.

25. Verbindungen der Formel (XXXV) 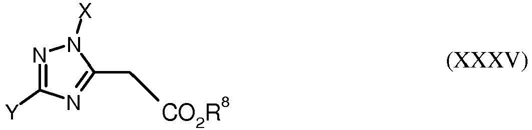 in welcher
X, Y und R⁸ die in Anspruch 1 bzw. 15 angegebene Bedeutung haben, ausgenommen die Verbindung

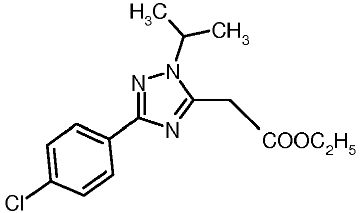

26. Verbindungen der Formel (XXXV-A) 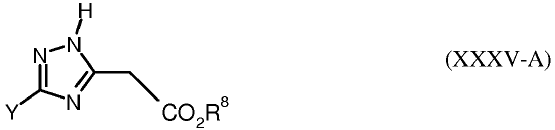 in welcher R⁸ und Y die in Anspruch 1 bzw. 15 angegebene Bedeutung haben, ausgenommen die Verbindung

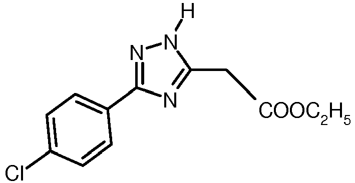

27. Verbindungen der Formel (XXXVII) 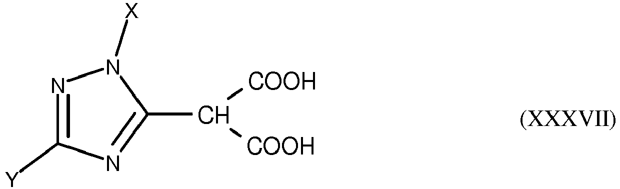 in welcher
X und Y die in Anspruch 1 angegebenen Bedeutungen haben.

28. Verbindungen der Formel (XXXVIII) 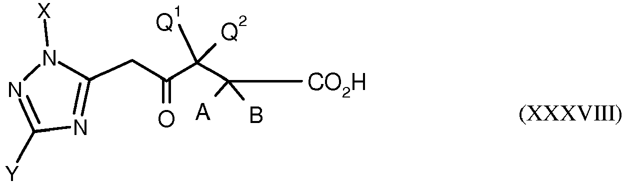 in welcher
X, Y, A, B, Q¹ und Q² die in Anspruch 1 angegebene Bedeutung haben.

29. Verbindungen der Formel (XXXIX) 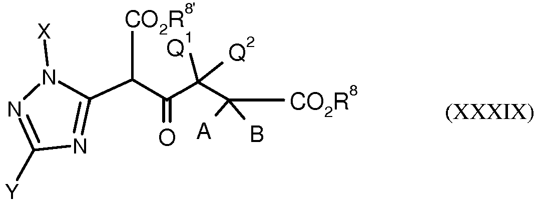 in welcher
A, B, Q¹, Q², X und Y die in Anspruch 1 angegebene Bedeutung haben und
R^{8'} für Alkyl stehen und
R⁸ für Wasserstoff oder Alkyl steht.

30. Verbindungen der Formeln 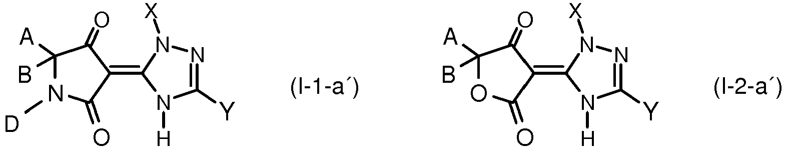 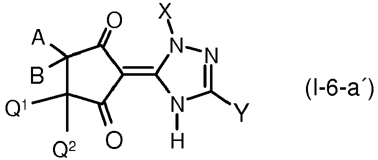 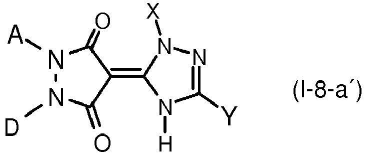 wobei X, Y, A, B, D, Q¹ und Q² die in Anspruch 1 angegebenen Bedeutungen haben.

## Claims

1. Compounds of the formula (I) 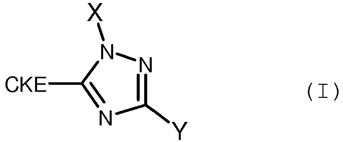 in which
X represents methyl, ethyl, n-propyl, isopropyl, n-butyl, -CH₂CF₃ or cyclopropyl,
Y represents phenyl, 4-Cl-benzyl, 4-F-phenyl, 4-Cl-phenyl or 2,4-Cl₂-phenyl,
CKE represents one of the groups 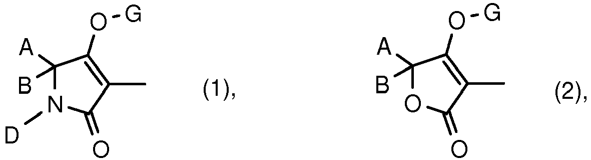 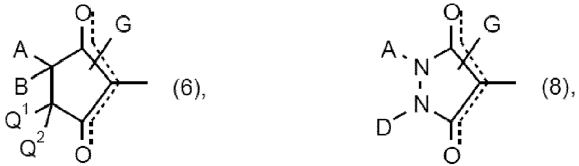
A represents hydrogen, methyl, ethyl or cyclopropyl,
B represents hydrogen, methyl or cyclopropyl,
A, B and the carbon atom to which they are attached represent saturated C₅-C₆-cycloalkyl in which optionally one ring member is replaced by oxygen or nitrogen and which is optionally mono- or disubstituted by methyl, ethyl, methoxymethyl, methoxy, ethoxy, propoxy, butoxy, trifluorethoxy, trifluoromethyl or -O-CH₂CHCH₂, where methoxy or ethoxy are also suitable as N-substituents, or
A, B and the carbon atom to which they are attached represent C₆-cycloalkyl which is optionally substituted by an alkylenedioxy group which contains two not directly adjacent oxygen atoms, thus forming a further 5- or 6-membered ring which may be mono- or disubstituted by methyl,
D represents hydrogen or cyclopropyl, or
A and D together with emphasis represent C₃-C₅-alkanediyl in which optionally one carbon atom is replaced by oxygen, or
A and D together represent C₃-C₅-alkanediyl which is optionally substituted by an alkylenedioxy group which optionally contains two not directly adjacent oxygen atoms and is optionally mono- to disubstituted by methyl, thus forming a further 5-membered ring, or
A and Q¹ together represent C₃-C₄-alkanediyl,
Q² represents hydrogen,
G represents hydrogen (a) or represents one of the groups 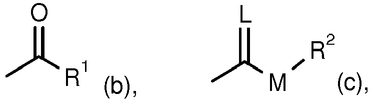
in which
L represents oxygen,
M represents oxygen,
R¹ represents C₁-C₆-alkyl,
R² represents C₁-C₆-alkyl,
G represents group (g) 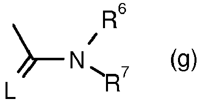
where L represents oxygen and
R⁶ and R⁷ together represent a C₅-alkylene radical in which one carbon atom is replaced by oxygen.

2. Compounds of the formula (I) according to Claim 1 in which
X represents methyl or ethyl,
Y represents 4-F-phenyl or 4-Cl-phenyl,
CKE represents one of the groups 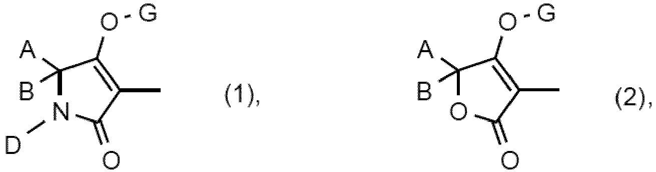
A represents hydrogen or methyl,
B represents hydrogen or methyl,
A, B and the carbon atom to which they are attached represent saturated C₆-cycloalkyl in which optionally one ring member is replaced by oxygen and which is optionally monosubstituted by methyl or methoxy,
D represents hydrogen or cyclopropyl, or
A and D together with emphasis represent C₃-alkanediyl,
G represents hydrogen (a).

3. Compounds of the formula (I) according to Claim 1 in which
X represents methyl, ethyl, isopropyl, n-propyl, n-butyl or -CH₂-CF₃,
Y represents phenyl, 4-Cl-benzyl, 4-F-phenyl, 4-Cl-phenyl or 2,4-Cl₂-phenyl,
CKE represents one of the groups 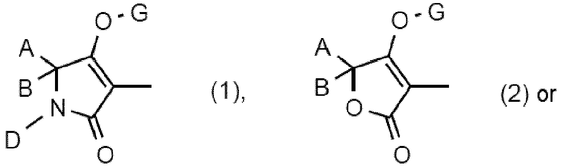 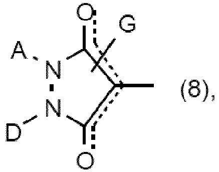
A represents hydrogen, methyl or cyclopropyl,
B represents hydrogen, methyl or cyclopropyl,
A, B and the carbon atom to which they are attached represent saturated C₆-cycloalkyl in which optionally one ring member is replaced by oxygen or nitrogen and which is optionally mono- or disubstituted by methyl, ethyl, methoxy, ethoxy, n-propoxy, trifluoromethyl or -O-CH₂CHCH₂, where methoxy or ethoxy are also suitable as N-substituents,
A, B and the carbon atom to which they are attached represent C₆-cycloalkyl which is optionally substituted by an alkylenedioxy group which contains two not directly adjacent oxygen atoms, thus forming a further 6-membered ring which may be monosubstituted by methyl,
D represents hydrogen or cyclopropyl, or
A and D together represent C₃-alkanediyl,
A and D in the case of CKE = group (8) together represent - (CH₂)₂-O-(CH₂)₂-,
G represents hydrogen (a) or represents group (g) 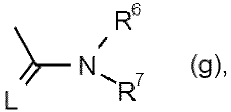
where L represents oxygen and
R⁶ and R⁷ together represent a C₅-alkylene radical in which one carbon atom is replaced by oxygen.

4. Process for preparing compounds of the formula (I) according to Claim 1, **characterized in that**, to obtain
(A) compounds of the formula (I-1-a) 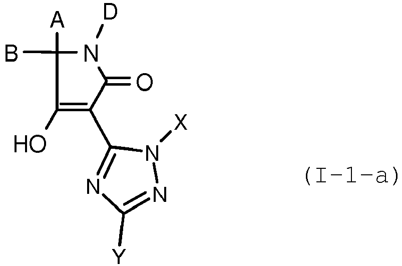 in which
A, B, D, X and Y have the meanings given in Claim 1, compounds of the formula (II) 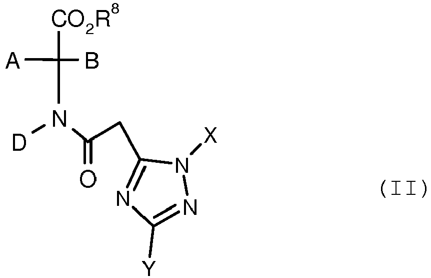 in which
A, B, D, X and Y have the meanings given in Claim 1, and
R⁸ represents alkyl,
are condensed intramolecularly in the presence of a diluent and in the presence of a base,
(B) compounds of the formula (I-2-a) 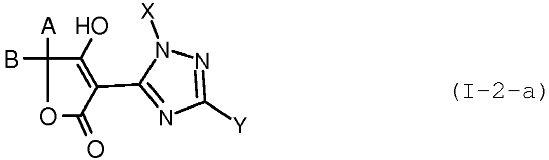 in which
A, B, X and Y have the meanings given in Claim 1, compounds of the formula (III) 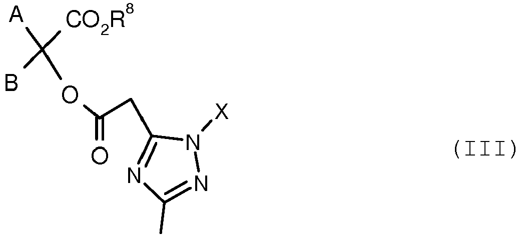 in which
A, B, X, Y and R⁸ have the meanings given in Claim 1,
are condensed intramolecularly in the presence of a diluent and in the presence of a base,
(F) compounds of the formula (I-6-a) 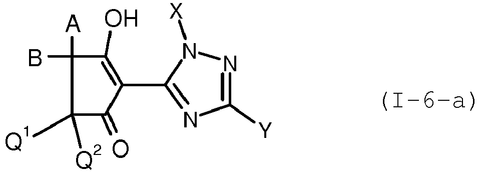 in which
A, B, Q¹, Q², X and Y have the meaning given in Claim 1
compounds of the formula (VIII) 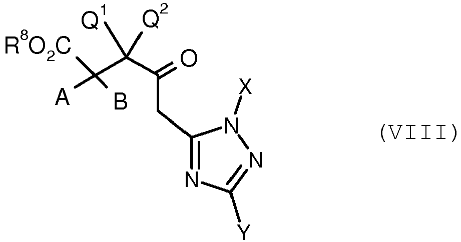 in which
A, B, Q¹, Q², X and Y have the meaning given in Claim 1 and
R⁸ represents alkyl,
are cyclized intramolecularly, if appropriate in the presence of a diluent and in the presence of a base,
(H) compounds of the formula (I-8-a) 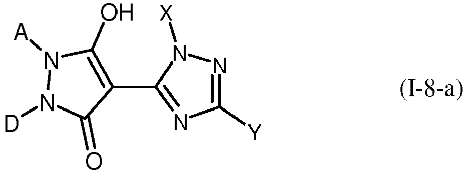 in which
A, D, X and Y have the meanings given in Claim 1, compounds of the formula (X) 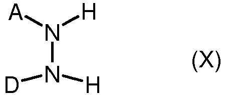 in which
A and D have the meaning given above
α) are reacted with compounds of the formula (VI) 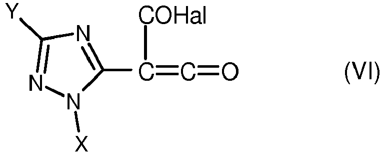 in which
Hal, X and Y have the meanings given in Claim 4,
if appropriate in the presence of a diluent and if appropriate in the presence of an acid acceptor, or
ß) are reacted with compounds of the formula (XI) 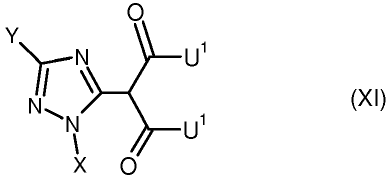 in which
X and Y have the meaning given in Claim 1
and U¹ represents NH₂ or O-R⁸, where R⁸ has the meaning mentioned above,
if appropriate in the presence of a diluent and if appropriate in the presence of a base, or
γ) compounds of the formula (XII) 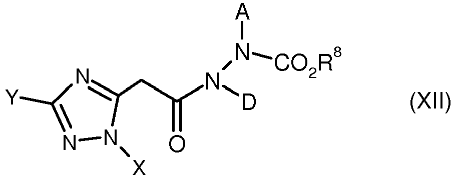 in which
A, D, X, Y and R⁸ have the meaning given in Claim 1,
are reacted, if appropriate in the presence of a diluent and if appropriate in the presence of a base,
(L) compounds of the formulae (I-1-b), (I-2-b), (I-6-b) and (I-8-b) shown above in which A, B, D, Q¹, Q², R¹, X and Y have the meanings given in Claim 1, compounds of the formulae (I-1-a), (I-2-a), (I-6-a), (I-8-) shown above in which A, B, D, Q¹, Q², X and Y have the meanings given in Claim 1 are in each case reacted
(α) with acid halides of the formula (XVI) 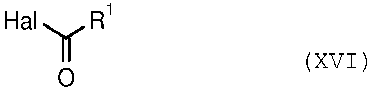 in which
R¹ has the meaning given in Claim 1 and
Hal represents halogen,
or
(ß) with carboxylic anhydrides of the formula (XVII)
R¹-CO-O-CO-R¹ (XVII)
in which
R¹ has the meaning given in Claim 1,
if appropriate in the presence of a diluent and if appropriate in the presence of an acid binder;
(M) compounds of the formulae (I-1-c), (I-2-c), (I-6-c), (I-8-c) shown above in which A, B, D, Q¹, Q², R², M, X and Y have the meanings given above and L represents oxygen, compounds of the formulae (I-1-a), (I-2-a), (I-6-a), (I-8-a) shown above in which A, B, D, Q¹, Q², X and Y have the meanings given above are in each case
reacted with chloroformic esters of the formula (XVIII)
R²-M-CO-Cl (XVIII)
in which
R² and M have the meanings given in Claim 1,
if appropriate in the presence of a diluent and if appropriate in the presence of an acid binder,
(R) compounds of the formulae (I-1-g), (I-2-g), (I-6-g), (I-8-g) shown above in which A, B, D, L, Q¹, Q², R⁶, R⁷, X and Y have the meanings given in Claim 1, compounds of the formulae (I-1-a), (I-2-a), (I-6-a), (I-8-a) shown above in which A, B, D, Q¹, Q², X and Y have the meanings given in Claim 1 are in each case
(α) reacted with isocyanates of the formula (XXIV)
R⁶-N=C=L (XXIV)
in which
R⁶ and L have the meanings given in Claim 1,
if appropriate in the presence of a diluent and if appropriate in the presence of a catalyst, or
(ß) reacted with carbamoyl chlorides of the formula (XXV) 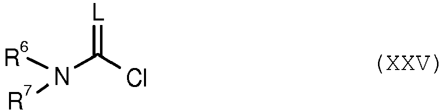 in which
L, R⁶ and R⁷ have the meanings given in Claim 1,
(VI)
if appropriate in the presence of a diluent and if appropriate in the presence of an acid binder.

5. Composition for controlling pests and/or unwanted vegetation, **characterized in that** it comprises at least one compound of the formula (I) according to Claim 1.

6. Method of controlling animal pests and/or unwanted vegetation, **characterized in that** compounds of the formula (I) according to Claim 1 are allowed to act on pests, unwanted vegetation and/or their habitat.

7. Use of compounds of the formula (I) according to Claim 1 for controlling animal pests and/or unwanted vegetation.

8. Process for preparing compositions for controlling pests and/or unwanted vegetation, **characterized in that** compounds of the formula (I) according to Claim 1 are mixed with extenders and/or surfactants.

9. Use of the compounds of the formula (I) according to Claim 1 for preparing compositions for controlling pests and/or unwanted vegetation.

10. Composition comprising an effective amount of an active compound combination comprising, as components,
(a') at least one compound of the formula (I) in which X, Y and CKE have the meaning given in Claim 1
and
(b') at least one crop plant compatibility-improving compound from the following group of compounds: S1, S2, S3, S4, S5, S6, S7, S8, S9, S10, S11, S12, S13, S14, S15.

11. Composition according to Claim 10 where the crop plant compatibility-improving compound is mefenpyr-diethyl.

12. Method for controlling unwanted vegetation, **characterized in that** a composition according to Claim 10 is allowed to act on the plants or their surroundings.

13. Use of a composition according to Claim 10 for controlling unwanted vegetation.

14. Method for controlling unwanted vegetation, **characterized in that** a compound of the formula (I) according to Claim 1 and the crop plant compatibility-improving compound as set forth in Claim 10 are allowed to act separately, in close temporal succession, on the plants or their surroundings.

15. Compounds of the formula (II) 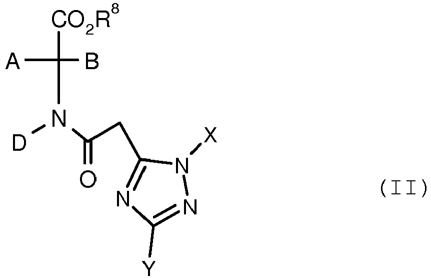 in which
A, B, D, X and Y have the meanings given in Claim 1, and
R⁸ represents alkyl.

16. Compounds of the formula (III) 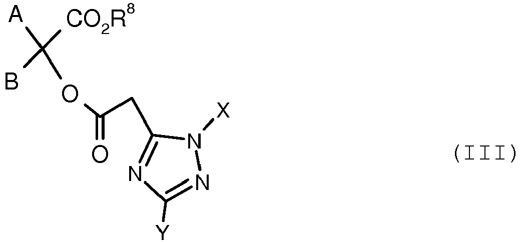 in which (III)
A, B, X, Y, and R⁸ have the meanings given in Claim 1.

17. Compounds of the formula (VI) 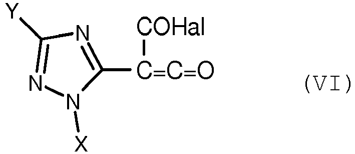 in which
X and Y have the meanings given in Claim 1 and
Hal represents halogen.

18. Compounds of the formula (VIII) 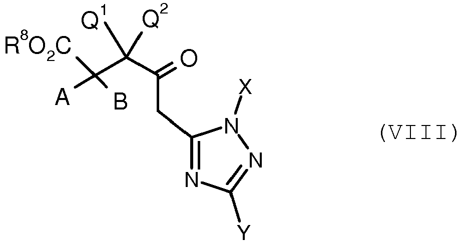 in which
A, B, Q¹, Q², R⁸, X and Y have the meaning given in Claim 1.

19. Compounds of the formula (XII) 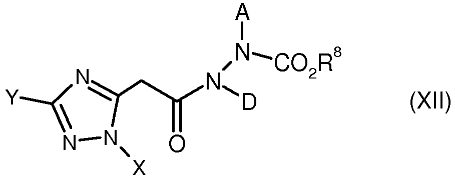 in which
A, D, X, Y and R⁸ have the meaning given in Claim 1.

20. Compounds of the formula (XI) 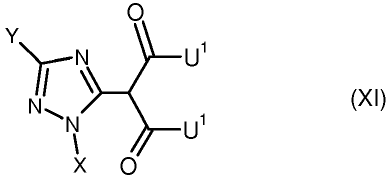 in which
X and Y have the meaning given in Claim 1
and U¹ represents NH₂ or O-R⁸, where R⁸ has the meaning mentioned in Claim 15.

21. Compounds of the formula (XXVIII) 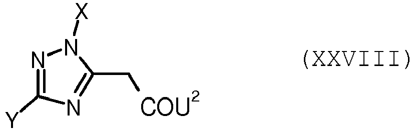 in which
X and Y have the meanings given in Claim 1 and
U² represents a leaving group introduced by reagents for activating carboxylic acids, propanephosphonic anhydride, phosphorylating agents, halogenating agents, phosgene or chloroformic esters.

22. Compounds of the formula (XXIX) 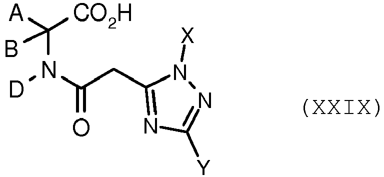 in which
A, B, D, X and Y have the meanings given in Claim 1.

23. Compounds of the formula (XXXI) 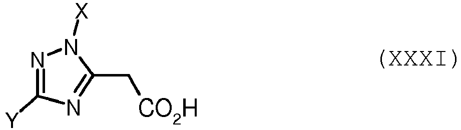 in which
X and Y have the meaning given in Claim 1.

24. Compounds of the formula (XXXIII) 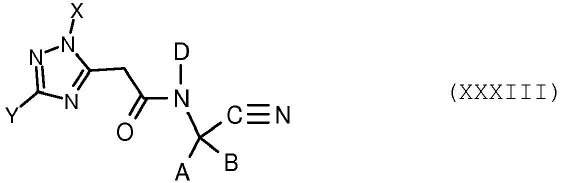 in which
A, B, D, X and Y have the meanings given in Claim 1.

25. Compounds of the formula (XXXV) 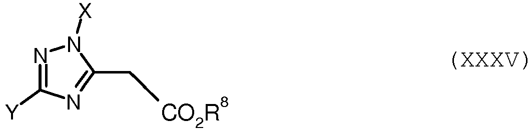 in which
X, Y and R⁸ have the meaning given in Claim 1 or 15, except for the compound

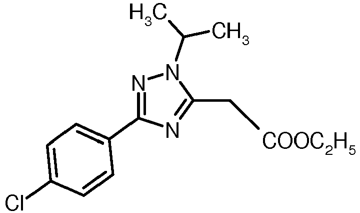

26. Compounds of the formula (XXXV-A) 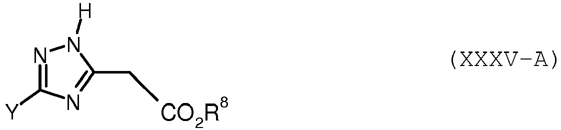 in which R⁸ and Y have the meaning given in Claim 1 or 15, except for the compound

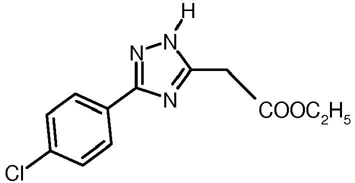

27. Compounds of the formula (XXXVII) 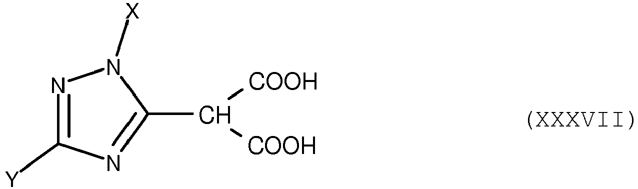 in which
X and Y have the meanings given in Claim 1.

28. Compounds of the formula (XXXVIII) 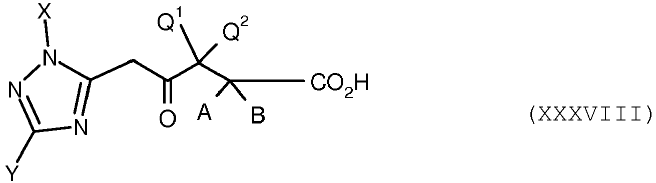 in which
X, Y, A, B, Q¹ and Q² have the meaning given in Claim 1.

29. Compounds of the formula (XXXIX) 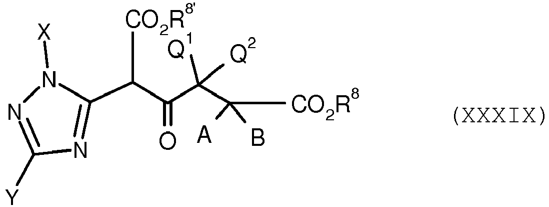 in which
A, B, Q¹ Q², X and Y have the meaning given in Claim 1 and
R^{8'} represents alkyl and
R⁸ represents hydrogen or alkyl.

30. Compounds of the formula 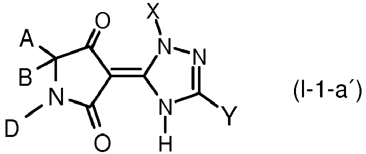 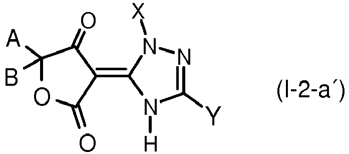 where X, Y, A, B, D, Q¹ and Q² have the meanings given in Claim 1.

## Revendications

1. Composés de formule (I) 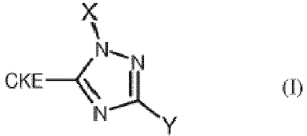 dans laquelle
X représente le groupe méthyle, éthyle, n-propyle, isopropyle, n-butyle, -CH₂CF₃ ou cyclopropyle,
Y représente le groupe phényle, 4-Cl-benzyle, 4-F-phényle, 4-Cl-phényle ou 2,4-Cl₂-phényle,
CKE représente l'un des groupes 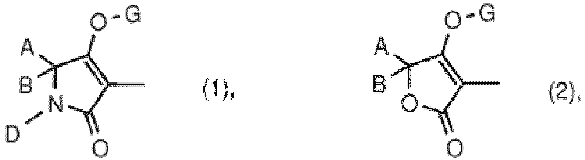 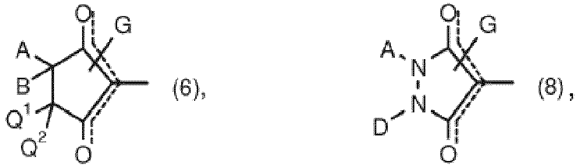
A représente un atome d'hydrogène, le groupe méthyle, éthyle ou cyclopropyle,
B représente un atome d'hydrogène, le groupe méthyle ou cyclopropyle,
A, B et l'atome de carbone, auquel ils sont liés, représentent un groupe cycloalkyle en C₅-C₆ saturé, dans lequel un chaînon formant le cycle est éventuellement remplacé par un atome d'oxygène ou d'azote, et qui est éventuellement une ou deux fois substitué par méthyle, éthyle, méthoxyméthyle, méthoxy, éthoxy, propoxy, butoxy, trifluoroéthoxy, trifluorométhyle ou -O-CH₂CHCH₂, le groupe méthoxy ou éthoxy étant également pris en considération comme substituants sur N, ou
A, B et l'atome de carbone, auquel ils sont liés, représentent un groupe cycloalkyle en C₆, qui est éventuellement substitué par un groupe alkylènedioxy contenant deux atomes d'oxygène non contigus, avec formation d'un cycle pentagonal ou hexagonal supplémentaire, qui peut être une ou deux fois substitué par méthyle,
D représente un atome d'hydrogène ou le groupe cyclopropyle, ou
A et D représentent ensemble un groupe alcane(C3-C5), dans lequel un atome de carbone est éventuellement remplacé par un atome d'oxygène ou
A et D représentent ensemble un groupe alcane(C3-C5)-diyle, qui est éventuellement substitué par un groupe alkylènedioxy contenant deux atomes d'oxygène non contigus, éventuellement une ou deux fois substitué par méthyle, de sorte qu'un cycle pentagonal supplémentaire est formé, ou
A et Q¹ représentent ensemble un groupe alcane(C₃-C₄)-diyle,
Q² représente un atome d'hydrogène,
G représente un atome d'hydrogène (a) ou représente l'un des groupes 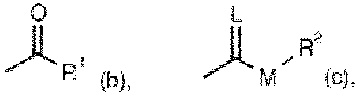
dans lesquels
L représente un atome d'oxygène,
M représente un atome d'oxygène,
R¹ représente un groupe alkyle en C₁-C₆,
R² représente un groupe alkyle en C₁-C₆,
G représente le groupe (g) 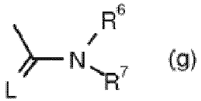
où L représente un atome d'oxygène et
R⁶ et R⁷ représentent ensemble un radical alkylène en C₅, dans lequel un atome de carbone est remplacé par un atome d'oxygène.

2. Composés de formule (I) selon la revendication 1, dans lesquels
X représente le groupe méthyle ou éthyle,
Y représente le groupe 4-F-phényle ou 4-Cl-phényle,
CKE représente l'un des groupes 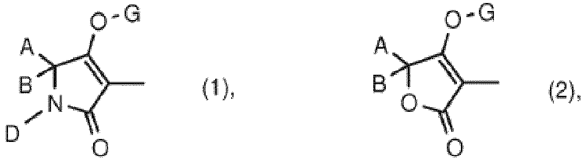
A représente un atome d'hydrogène ou le groupe méthyle,
B représente un atome d'hydrogène ou le groupe méthyle,
A, B et l'atome de carbone, auquel ils sont liés, représentent un groupe cycloalkyle en C₆ saturé, dans lequel un chaînon formant le cycle est éventuellement remplacé par un atome d'oxygène, et qui est éventuellement une fois substitué par méthyle ou méthoxy,
D représente un atome d'hydrogène ou le groupe cyclopropyle, ou
A et D pris ensemble représentent un groupe alcane(C₃)-diyle,
G représente un atome d'hydrogène (a) .

3. Composés de formule (I) selon la revendication 1, dans lesquels
X représente le groupe méthyle, éthyle, isopropyle, n-propyle, n-butyle ou -CH₂-CF₃,
Y représente le groupe phényle, 4-Cl-benzyle, 4-F-phényle, 4-Cl-phényle ou 2, 4-Cl₂-phényle,
CKE représente l'un des groupes 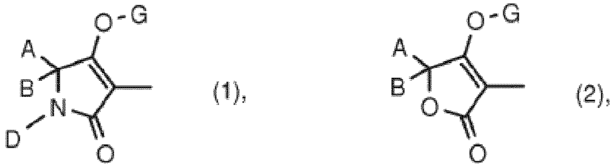 ou 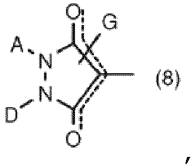
A représente un atome d'hydrogène, le groupe méthyle ou cyclopropyle,
B représente un atome d'hydrogène, le groupe méthyle ou cyclopropyle,
A, B et l'atome de carbone, auquel ils sont liés, représentent un groupe cycloalkyle en C₆ saturé, dans lequel un chaînon formant le cycle est éventuellement remplacé par un atome d'oxygène ou d'azote, et qui est éventuellement une ou deux fois substitué par méthyle, éthyle, méthoxy, éthoxy, n-propoxy, trifluorométhyle ou -O-CH₂CHCH₂, le groupe méthoxy ou éthoxy étant également pris en considération comme substituants sur N,
A, B et l'atome de carbone, auquel ils sont liés, représentent un groupe cycloalkyle en C₆, qui est éventuellement substitué par un groupe alkylènedioxy contenant deux atomes d'oxygène non contigus, avec formation d'un cycle hexagonal supplémentaire, qui peut être une fois substitué par méthyle,
D représente un atome d'hydrogène ou le groupe cyclopropyle, ou
A et D représentent ensemble un groupe alcane(C₃)diyle,
A et D, dans le cas où CKE = groupe (8), représentent ensemble le groupe -(CH₂)₂-O-(CH₂)₂-,
G représente un atome d'hydrogène (a) ou le groupe (g) 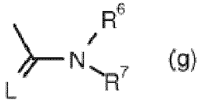
où L représente un atome d'oxygène et,
R⁶ et R⁷ représentent ensemble un radical alkylène en C₅, dans lequel un atome de carbone est remplacé par un atome d'oxygène.

4. Procédé pour la préparation de composés de formule (I) selon la revendication 1, **caractérisé en ce que**, pour l'obtention
(A) de composés de formule (I-1-a) 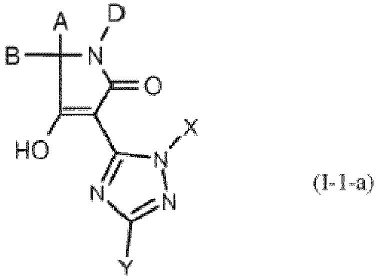 dans laquelle
A, B, D, X et Y ont les significations indiquées dans la revendication 1,
on condense par condensation intramoléculaire des composés de formule (II) 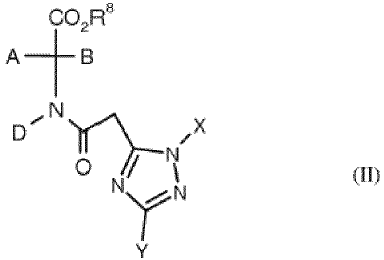 dans laquelle
A, B, D, X et Y ont les significations indiquées dans la revendication 1,
et
R⁸ représente un groupe alkyle,
en présence d'un diluant et en présence d'une base,
(B) de composés de formule (I-2-a) 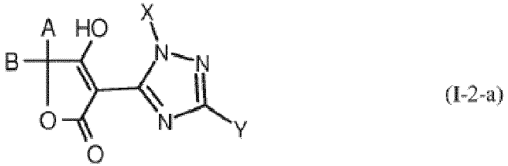 dans laquelle
A, B, X et Y ont les significations indiquées dans la revendication 1,
on condense par condensation intramoléculaire des composés de formule (III) 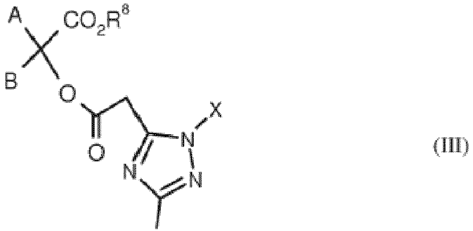 dans laquelle
A, B, X, Y et R⁸ ont les significations indiquées dans la revendication 1,
en présence d'un diluant et en présence d'une base,
(F) de composés de formule (I-6-a) 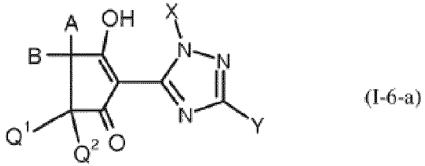 dans laquelle
A, B, Q¹, Q², X et Y ont les significations indiquées dans la revendication 1,
on cyclise par cyclisation intramoléculaire des composés de formule (VIII) 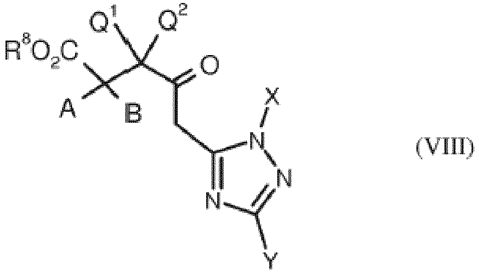 dans laquelle
A, B, Q¹, Q², X et Y ont les significations indiquées dans la revendication 1, et
R⁸ représente un groupe alkyle,
éventuellement en présence d'un diluant et en présence d'une base,
(H) de composés de formule (I-8-a) 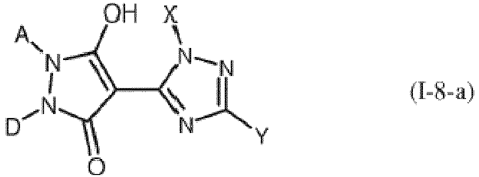 dans laquelle
A, D, X et Y ont les significations indiquées dans la revendication 1,
on fait réagir des composés de formule (X) 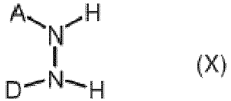 dans laquelle
A et D ont les significations indiquées ci-dessus,
α) avec des composés de formule (VI) 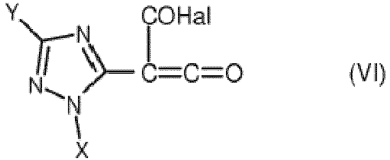 dans laquelle
Hal, X et Y ont les significations indiquées dans la revendication 4,
éventuellement en présence d'un diluant et éventuellement en présence d'un capteur d'acide, ou
β) avec des composés de formule (XI) 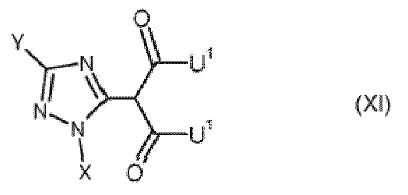 dans laquelle
X et Y ont la signification indiquée dans la revendication 1,
et U¹ représente NH₂ ou O-R⁸, R⁸ ayant la signification indiquée plus haut,
éventuellement en présence d'un diluant et éventuellement en présence d'une base, ou
γ) des composés de formule (XII) 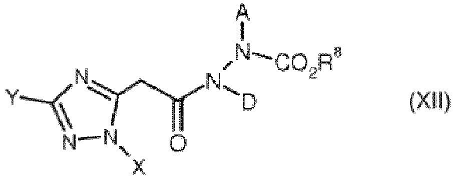 dans laquelle
A, D, X, Y et R⁸ ont la signification indiquée dans la revendication 1,
éventuellement en présence d'un diluant et éventuellement en présence d'une base,
(L) de composés de formules générales (I-1-b), (I-2-b), (I-6-b), (I-8-b), indiquées plus haut, dans lesquelles A, B, D, Q¹, Q², R¹, X et Y ont les significations indiquées dans la revendication 1,
on fait réagir des composés de formules générales (I-1-a), (I-2-a), (I-6-a), (I-8-), indiquées plus haut, dans (lesquelles A, B, D, Q¹, Q², X et Y ont haut, dans lesquelles A, B, D, Q¹, Q², X et Y ont les significations indiquées dans la revendication 1, respectivement
(α) avec des halogénures d'acides de formule (XVI) 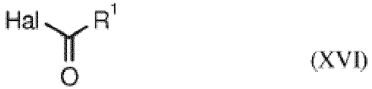 dans laquelle
R¹ a la signification indiquée dans la revendication 1 et
Hal représente un atome d'halogène
ou
(β) avec des anhydrides d'acides carboxyliques de formule (XVII)
R¹-CO-O-CO-R¹ (XVII)
dans laquelle
R¹ a la signification indiquée dans la revendication 1,
éventuellement en présence d'un diluant et éventuellement en présence d'un capteur d'acide ;
(M) de composés de formules (I-1-c), (I-2-c), (I-6-c), (I-8-c), indiquées plus haut, dans lesquelles A, B, D, Q¹, Q², R², M, X et Y ont les significations indiquées plus haut et L représente un atome d'oxygène, on fait réagir des composés de formules (I-1-a), (I-2-a), (I-6-a), (I-8-a), indiquées plus haut, dans (lesquelles A, B, D, Q¹, Q², X et Y ont haut, dans lesquelles A, B, D, Q¹, Q², X et Y ont les significations indiquées plus haut, respectivement
avec des esters d'acide chloroformique de formule (XVIII)
R²-M-CO-Cl (XVIII)
dans laquelle
R² et M ont les significations indiquées dans la revendication 1,
éventuellement en présence d'un diluant et éventuellement en présence d'un capteur d'acide ;
(R) de composés de formules (I-1-g), (I-2-g), (I-6-g), (I-8-g), indiquées plus haut, dans lesquelles A, B, D, L, Q¹, Q², R⁶, R⁷, X et Y ont les significations indiquées dans la revendication 1, on fait réagir des composés de formules (I-1-a), (I-2-a), (I-6-a), (I-8-a), indiquées plus haut, dans lesquelles A, B, D, Q¹, Q², X et Y ont les significations indiquées dans la revendication 1, respectivement
(α) avec des isocyanates de formule (XXIV)
R⁶-N=C=L (XXIV)
dans laquelle
R⁶ et L ont les significations indiquées dans la revendication 1,
(VI)
éventuellement en présence d'un diluant et éventuellement en présence d'un catalyseur ou
(β) avec des chlorures d'acide carbamique de formule (XXV) 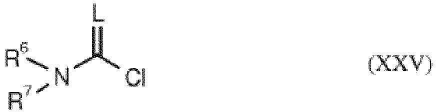 dans laquelle
L, R⁶ et R⁷ ont les significations indiquées dans la revendication 1,
(VI)
éventuellement en présence d'un diluant et éventuellement en présence d'un capteur d'acide.

5. Composition destinée à la lutte contre des ravageurs animaux et/ou une végétation indésirable, **caractérisée par** une teneur en au moins un composé de formule (I) selon la revendication 1.

6. Procédé pour la lutte contre des ravageurs animaux et/ou une végétation indésirable, **caractérisé en ce qu'**on fait agir sur les ravageurs, la végétation indésirable et/ou leur habitat des composés de formule (I) selon la revendication 1.

7. Utilisation de composés de formule (I) selon la revendication 1, pour la lutte contre des ravageurs animaux et/ou une végétation indésirable.

8. Procédé pour la fabrication de compositions destinées à la lutte contre des ravageurs et/ou une végétation indésirable, **caractérisé en ce qu'**on mélange des composés de formule (I) selon la revendication 1 avec des excipients et/ou des substances tensioactives.

9. Utilisation de composés de formule (I) selon la revendication 1, pour la fabrication de compositions destinées à la lutte contre des ravageurs et/ou une végétation indésirable.

10. Composition ayant une teneur efficace en une association de substances actives comprenant comme composants
(a') au moins un composé de formule (I), dans laquelle X, Y et CKE ont la signification indiquée dans la revendication 1
et
(b') au moins un composé améliorant la tolérance par les plantes cultivées, choisi dans le groupe suivant de composés : S1, S2, S3, S4, S5, S6, S7, S8, S9, S10, 511, S12, S13, S14, S15.

11. Composition selon la revendication 10, dans laquelle le composé améliorant la tolérance par les plantes cultivées est le méfenpyr-diéthyle.

12. Procédé pour la lutte contre une végétation indésirable, **caractérisé en ce qu'**on fait agir sur les plantes ou leur environnement une composition selon la revendication 10.

13. Utilisation d'une composition selon la revendication 10, pour la lutte contre une végétation indésirable.

14. Procédé pour la lutte contre une végétation indésirable, **caractérisé en ce qu'**on fait agir sur les plantes ou leur environnement, séparément en un court intervalle de temps, un composé de formule (I) selon la revendication 1 et le composé améliorant la tolérance par les plantes cultivées, selon la revendication 10.

15. Composés de formule (II) 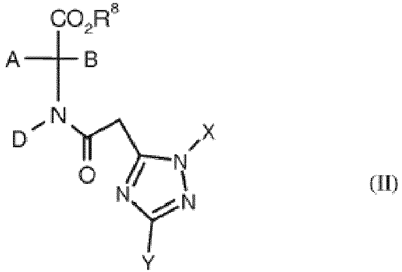 dans laquelle
A, B, D, X et Y ont les significations indiquées dans la revendication 1,
et
R⁸ représente un groupe alkyle.

16. Composés de formule (III) 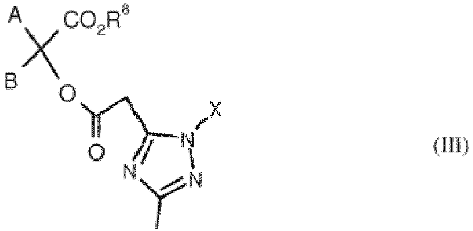 dans laquelle
(III)
A, B, X, Y et R⁸ ont les significations indiquées dans la revendication 1.

17. Composés de formule (VI) 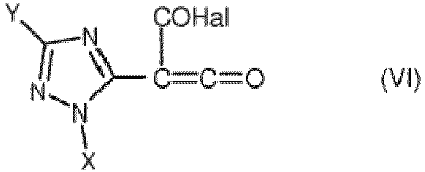 dans laquelle
X et Y ont les significations indiquées dans la revendication 1 et
Hal représente un atome d'halogène.

18. Composés de formule (VIII) 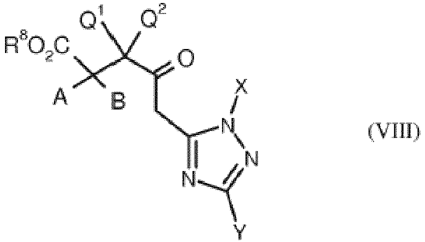 dans laquelle
A, B, Q¹, Q², R⁸, X et Y ont les significations indiquées dans la revendication 1.

19. Composés de formule (XII) 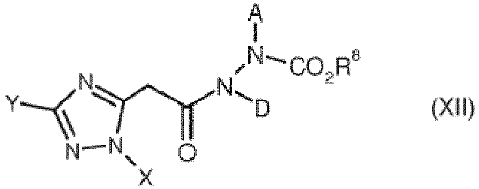 dans laquelle
A, D, X, Y et R⁸ ont la signification indiquée dans la revendication 1.

20. Composés de formule (XI) 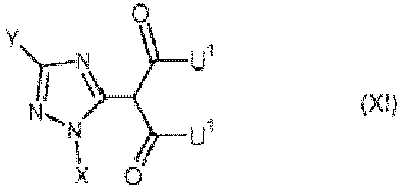 dans laquelle
X et Y ont la signification indiquée dans la revendication 1,
et U¹ représente NH₂ ou O-R⁸, R⁸ ayant la signification indiquée dans la revendication 15.

21. Composés de formule (XXVIII) 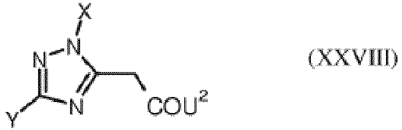 dans laquelle
X et Y ont les significations indiquées dans la revendication 1 et
U² représente un groupe partant introduit au moyen de réactifs d'activation d'acides carboxyliques, d'anhydride d'acide propanephosphonique, de réactifs de phosphorylation, d'agents d'halogénation, de phosgène ou d'esters d'acide chloroformique.

22. Composés de formule (XXIX) 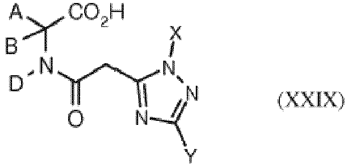 dans laquelle
A, B, D, X et Y ont les significations indiquées dans la revendication 1.

23. Composés de formule (XXXI) 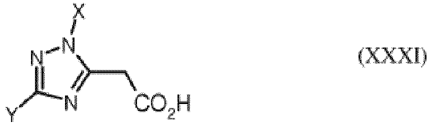 dans laquelle
X et Y ont les significations indiquées dans la revendication 1.

24. Composés de formule (XXXIII) 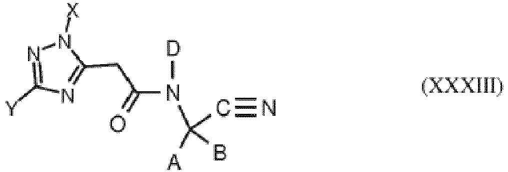 dans laquelle A, B, D, X et Y ont les significations indiquées dans la revendication 1.

25. Composés de formule (XXXV) 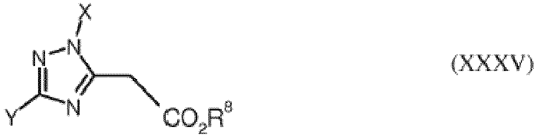 dans laquelle
X, Y et R⁸ ont la signification indiquée dans la revendication 1 ou 15, à l'exclusion du composé

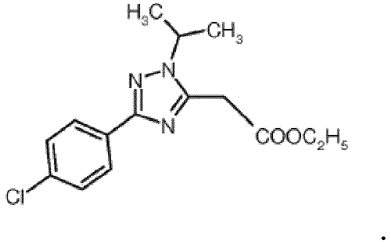

26. Composés de formule (XXXV-A) 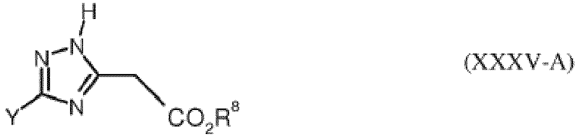 dans laquelle R⁸ et Y ont la signification indiquée dans la revendication 1 ou 15, à l'exclusion du composé

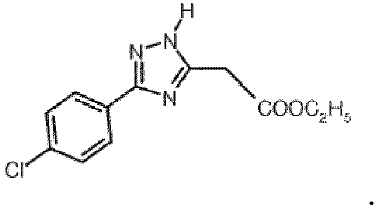

27. Composés de formule (XXXVII) 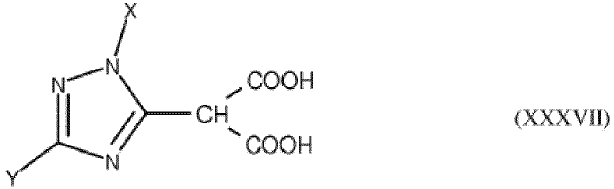 dans laquelle
X et Y ont les significations indiquées dans la revendication 1.

28. Composés de formule (XXXVIII) 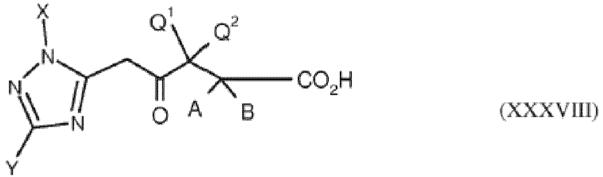 dans laquelle
X, Y, A, B, Q¹ et Q² ont la signification indiquée dans la revendication 1.

29. Composés de formule (XXXIX) 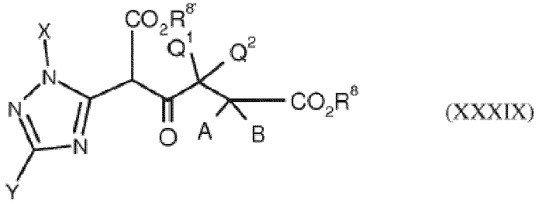 dans laquelle
A, B, Q¹, Q², X et Y ont la signification indiquée dans la revendication 1 et
R^{8'} représente un groupe alkyle et
R⁸ représente un atome d'hydrogène ou un groupe alkyle.

30. Composés de formules 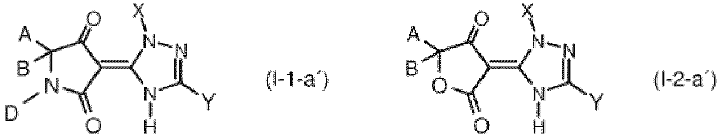 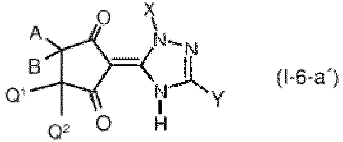 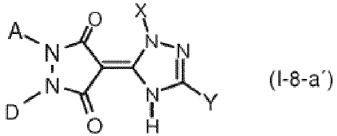 où X, Y, A, B, D, Q¹ et Q² ont les significations indiquées dans la revendication 1.
